(19) Europäisches Patentamt
European Patent Office
Office européen des brevets

(11) **EP 1 576 180 B1**

(12) **EUROPEAN PATENT SPECIFICATION**

(45) Date of publication and mention
of the grant of the patent:
**18.02.2009 Bulletin 2009/08**

(51) Int Cl.:
***C12Q 1/48*** (2006.01)

(21) Application number: **03812515.9**

(22) Date of filing: **04.12.2003**

(86) International application number:
**PCT/US2003/038628**

(87) International publication number:
**WO 2004/050898 (17.06.2004 Gazette 2004/25)**

(54) **AMPK PATHWAY COMPONENTS**

KOMPONENTEN DES AMPK-WEGS

COMPOSANTS DE LA VOIE AMPK

(84) Designated Contracting States:
**AT BE BG CH CY CZ DE DK EE ES FI FR GB GR HU IE IT LI LU MC NL PT RO SE SI SK TR**

(30) Priority: **04.12.2002 US 430804 P**
**18.07.2003 US 488261 P**

(43) Date of publication of application:
**21.09.2005 Bulletin 2005/38**

(73) Proprietor: **Elixir Pharmaceuticals, Inc.**
**Cambridge, MA 02139 (US)**

(72) Inventors:
• **APFELD, Javier**
**Cambridge, MA 02139 (US)**
• **O'CONNOR, Greg**
**Natick, MA 01760 (US)**

(74) Representative: **Wichmann, Hendrik**
**Isenbruck Bösl Hörschler Wichmann Huhn LLP**
**Patentanwälte**
**Prinzregentenstrasse 68**
**81675 München (DE)**

(56) References cited:
• LIN S-J ET AL: "Requirement of NAD and SIR2 for life-span extension by calorie restriction in saccharomyces cerevisiae" SCIENCE, AMERICAN ASSOCIATION FOR THE ADVANCEMENT OF SCIENCE,, US, vol. 289, 22 September 2000 (2000-09-22), pages 2126-2128, XP002966705 ISSN: 0036-8075

• BEST J D ET AL: "NOVEL AGENTS FOR MANAGING DYSLIPIDAEMIA" EXPERT OPINION ON INVESTIGATIONAL DRUGS, ASHLEY PUBLICATIONS LTD., LONDON, GB, vol. 10, no. 11, November 2001 (2001-11), pages 1901-1911, XP008010536 ISSN: 1354-3784
• VANFLETEREN J R ET AL: "Modulation of kinase activities in dauers and in long-lived mutants of Caenorhabditis elegans." THE JOURNALS OF GERONTOLOGY. SERIES A, BIOLOGICAL SCIENCES AND MEDICAL SCIENCES. JUL 1997, vol. 52, no. 4, July 1997 (1997-07), pages B212-B216, XP008064507 ISSN: 1079-5006
• GUARENTE LEONARD ET AL: "Genetic pathways that regulate ageing in model organisms" NATURE (LONDON), vol. 408, no. 6809, 9 November 2000 (2000-11-09), pages 255-262, XP002382391 ISSN: 0028-0836
• APFELD J ET AL: "The AMP-activated protein kinase AAK-2 links energy levels and insulin-like signals to lifespan in C. elegans" GENES AND DEVELOPMENT 15 DEC 2004 UNITED STATES, vol. 18, no. 24, 15 December 2004 (2004-12-15), pages 3004-3009, XP002382392 ISSN: 0890-9369
• APFELD J ET AL: "Erratum: The AMP-activated protein kinase AAK-2 links energy levels and insulin-like signals to lifespan in C. elegans (Genes and Development (2005))" GENES AND DEVELOPMENT 01 JAN 2005 UNITED STATES, vol. 19, no. 1, 1 January 2005 (2005-01-01), page 188, XP002382393 ISSN: 0890-9369
• LIN SU-JU ET AL: "Calorie restriction extends Saccharomyces cerevisiae lifespan by increasing respiration." NATURE (LONDON), vol. 418, no. 6895, 18 July 2002 (2002-07-18), pages 344-348, XP002382492 ISSN: 0028-0836

- **BRAECKMAN B P ET AL: "Insulin-like signaling, metabolism, stress resistance and aging in Caenorhabditis elegans" MECHANISMS OF AGEING AND DEVELOPMENT, ELSEVIER SEQUOIA, LAUSANNE,, CH, vol. 122, no. 7, 31 May 2001 (2001-05-31), pages 673-693, XP002280657 ISSN: 0047-6374**
- **LONGO VALTER D ET AL: "Evolutionary medicine: From dwarf model systems to healthy centenarians?" SCIENCE (WASHINGTON D C), vol. 299, no. 5611, 28 February 2003 (2003-02-28), pages 1342-1346, XP002382493 ISSN: 0036-8075**
- **BEST ET AL.: 'Novel agents for managing dyslipidaemia.' EXPERT OPIN INVESTIG DRUGS. vol. 10, no. 11, 2001, pages 1901 - 1911, XP008010536**

**Description**

*BACKGROUND*

The AMP-activated protein kinase (AMPK) is a regulator of metabolism in mammals, including humans. The net effects of AMPK activation include stimulation of hepatic fatty acid oxidation and ketogenesis, inhibition of cholesterol synthesis, lipogenesis, and triglyceride synthesis, inhibition of adipocyte lipolysis and lipogenesis, stimulation of skeletal muscle fatty acid oxidation and muscle glucose uptake, and modulation of insulin secretion by pancreatic beta-cells. Mammalian AMPK can be activated by cellular stresses associated with ATP depletion. AMPK is a heterotrimer comprising a catalytic alpha subunit with associated beta and gamma subunits. Different isoforms exist.

AMPK can be activated by various types of stress associated with ATP depletion, such as hypoxia, heat shock, metabolic poisoning and, in muscle, exercise. AMPK phosphorylates multiple targets which switch off anabolic pathways and switch on alternative catabolic pathways. At a molecular level, AMPK is allosterically regulated by 5'-AMP (AMP) which can activate the kinase. Creatine phosphate can allosterically inhibit the kinase. ATP also inhibits AMPK.

One mechanism of activation of AMPK is mediated by AMPKK which phosphorylated AMPK. AMP binding to AMPK improves its affinity for AMPKK. In addition AMP also activates AMPKK directly. AMPKK phosphorylates AMPK on threonine 172, thereby activating AMPK. Conversely, AMPK is inactivated by protein phosphatase 2C which removes the phosphate from threonine 172. AMP binding to AMPK also reduces its affinity for protein phosphatase 2C.

*SUMMARY*

The invention is based, in part, on the discovery that AMPK pathway components control elements of lifespan regulation.

The invention features a method of evaluating a compound, the method comprising:

- contacting a polypeptide *in vitro* with a test compound, wherein the polypeptide comprises a sequence at least 85 % identical to (i) an alpha AMPK subunit, (ii) a beta AMPK subunit, (iii) a gamma AMPK subunit, or (iv) a functional domain thereof,
- evaluating an interaction between the test compound and the polypeptide;
- contacting a cell or non-human organism that produces the polypeptide with the test compound; and
- evaluating a rate of aging of the cell or non-human organism

wherein the compound increases activity of AMPK, thereby enhancing lifespan of the cell or non-human organism.

The invention further features a method of evaluating a protein, the method comprising

(a) identifying a candidate protein, wherein the candidate protein comprises a sequence of at least 100 amino acids that is at least 50 % identical to an AMPK subunit or is at least 30 % identical to an AMPK kinase domain;
(b) identifying one or more polymorphisms in the gene that encodes the candidate protein; and
(c) evaluating relationships between the presence of one or more of the polymorphisms and the longevity of the non-human organism that contains the polymorphism to identify a correlation between the one or more polymorphisms and longevity of the organism, thereby evaluating the protein.

In one embodiment, the evaluating the rate of aging includes one or more of: (a) assessing the life span of the cell or the organism; (b) assessing the presence or abundance of a gene transcript or gene product in the cell or organism that has a biological age-dependent expression pattern; (c) evaluating resistance of the cell or organism to stress; (d) evaluating one or more metabolic parameters of the cell or organism; (e) evaluating the proliferative capacity of the cell or a set of cells present in the organism; and (f) evaluating physical appearance or behavior of the cell or organism.

Exemplary forms of stress include oxidative stress (e.g., hypoxia), genotoxic stress, thermal stress. Exemplary agents that mediate stress include: a stress, e.g., UV light, oxygen radicals, toxins, a particular diet, and so forth.

Evaluating the rate of aging includes measuring the life span of one or more organisms contacted with the test compound. For example, a statistical value descriptive of life span for a group of genetically matched organisms contacted with the test compound is measured and that statistical value is compared to a corresponding statistical value descriptive of life span for a control group of genetically matched organisms that have not been contacted with the test compound. Exemplary statistical values includes an average, a standard deviation, and a median.

The protein can include a polypeptide having a mammalian (e.g., murine, bovine, feline, canine, equine, simian, human) amino acid sequence of at least 50, 100, 200, or 300 amino acids. For example, the polypeptide includes a sequence at least 20, 30, 50, 60, 80, 90, 95, 96, 99, or 100% identical to a full-length sequence described herein, e.g., SEQ ID NO:1-6 or a sequence from the [Appendix], or a fragment thereof, e.g., a functional domain thereof. Exemplary AMPK pathway members include AMPK activating proteins, such as AMPKK, and AMPK suppressing proteins such as protein phosphatase 2C. Still other pathway members include AMPK substrates, for example, in liver cells, acetyl-CoA carboxylase (ACC) and 3-hydroxyl-3-methylglutaryl-CoA reductase (HMGR) are AMPK substrates.

In one embodiment, evaluating the interaction between the test compound and the polypeptide includes one or more of (i) evaluating binding of the test compound to the polypeptide; (ii) evaluating a biological activity of the polypeptide; (iii) evaluating an enzymatic activity (e.g., kinase activity) of the polypeptide. The *in vitro* contacting can include forming a reaction mixture that includes the test compound, the polypeptide, a substrate (e.g., a peptide substrate), and ATP (e.g., radiolabeled ATP) and evaluating transfer of a phosphate from the ATP to the substrate. Evaluating transfer of the phosphate can include, for example, detecting the phosphate (e.g., chemically or using a label, e.g., a radiolabel) or detecting a physical property of the substrate, e.g., a change in molecular weight, charge, or pI.

Where an organism is evaluated, the organism can be, for example, a mammal (e.g., a mouse, rat, primate, or other non-human), or other animal (e.g., Xenopus, zebrafish, or an invertebrate such as a fly or nematode). The organism can be a wild-type, mutant, RNAi-treated, or transgenic organism. Cells that are evaluated can be from such organisms (e.g., a wild-type, mutant, RNAi-treated, or transgenic organism). For example, a transgenic organism or cell can include a transgene that encodes a heterologous polypeptide, e.g., an AMPK polypeptide, e.g., a human AMPK polypeptide or a polypeptide that includes a sequence at least 20, 40, 60, 80, 90, 95, or 98% identical to at least 50, 100, or 150 amino acids of an AMPK polypeptide, e.g., a mammalian, e.g., human AMPK polypeptide.

In an embodiment using an organism assay, the organism is an adult organism. In a related embodiment, at least 30, 50, 60, 80% of the expected normal life span of the organism has elapsed prior to the organism being contacted with the test compound.

In an embodiment wherein the rate of aging of the cell is determined, the cell can be isolated from an organism that has been contacted with the test compound or can be cultured, e.g., prior to contact with the test compound. For example, the cell is contacted with the test compound in vitro.

The method can be repeated with one or more additional compounds to thereby evaluate a library of test compounds. The method can include one or more other features described herein.

The method may include: providing a cell that expresses a heterologous polypeptide including at least 100 amino acids of an AMPK pathway component (e.g., an AMPK pathway component from a species that is the same or different as the species of the cell or an artificial variant of a naturally-occurring AMPK pathway component); contacting a test compound to the cell; and evaluating an age-associated parameter of the cell.

In one embodiment, the cell is a cell from a model organism, e.g., a nematode, a fly, or a mammal, e.g., a rodent. The cell can be deficient in an endogenous AMPK activity, e.g., as a result of mutation or a treatment, e.g., RNAi treatment. The method can include one or more other features described herein.

A method may include: providing an organism that expresses a heterologous polypeptide in one or more cells, the heterologous polypeptide including at least 100 amino acids of an AMPK pathway component (e.g., an AMPK pathway component from a species that is the same or different as the species of the cell or an artificial variant of a naturally-occurring AMPK pathway component); administering a test compound to the organism; and evaluating an age-associated parameter of the organism.

In one embodiment, the organism is a model organism, e.g., a nematode, a fly, or a mammal, e.g., a rodent. The organism can be deficient in an endogenous AMPK activity. The method can include one or more other features described herein.

The method may include: identifying a candidate protein, wherein the candidate protein includes a sequence of at least 50, 100, or 250 amino acids that is at least 30, 50, 60, 70, 80, 90, 95, or 100% identical to an AMPK pathway component polypeptide, e.g., an AMPK polypeptide, e.g., an AMPK alpha subunit polypeptide (e.g., a human AMPK pathway component); altering the expression or activity of the candidate protein in a cell or in one or more cells of an organism; and evaluating the rate of aging of the cell or the organism. The method can be used to evaluate a protein.

In one embodiment, the identifying includes one or more of: searching a computer database, low stringency hybridization, nucleic acid amplification, or genetic complementation.

The alteration is a reduction in the expression of the candidate protein, e.g., using double stranded inhibitory RNA, antisense RNA, or a ribozyme. The alteration is an increase in the expression of the candidate protein. For example, the increase is produced by a transgene that encodes a copy of the candidate protein. In another example, the candidate protein is produced by an endogenous gene in the cell or the organism. The method can further include expressing a heterologous AMPK pathway component or segment thereof (e.g., from a mammal or human) in the cell or the organism. The method can further include one or more other features described herein.

In another aspect, the invention features a method of evaluating a protein. The method includes: a) identifying a candidate protein, wherein the candidate protein includes a sequence of at least 100 amino acids that is at least 50, 70, 80, 90, 95, or 100% identical to an AMPK subunit or is at least 30, 50, 70, 80, 90, 95, or 100% identical to an AMPK kinase domain; b) identifying one or more polymorphisms in the gene that encodes the candidate protein; and c) evaluating relationships between the presence of one or more of the polymorphisms and the longevity of the organism that contains the polymorphism to identify a correlation between the one or more polymorphisms and longevity of the organism, thereby evaluating the protein, as defined in the claims. The method includes: evaluating a compound for an effect on AMPK pathway component activity; and evaluating a compound for an effect on age-associated disease. Similarly it is possible to evaluate a plurality of compounds (e.g., from a library of compounds). For each compound of a plurality, of compounds, the method includes evaluating the compound for an effect on AMPK pathway component activity; and, optionally if the compound has an effect on AMPK pathway component activity, evaluating the compound for an effect on age-associated disease. In one embodiment, evaluating for an effect on AMPK pathway component activity includes evaluating AMPK pathway component mRNA expression. In another embodiment, evaluating for an effect on AMPK pathway component activity includes evaluating a AMPK pathway component polypeptide (e.g., evaluating AMPK pathway component enzymatic activity, e.g., kinase activity).

In one embodiment, evaluating for an effect on age-associated disease includes contacting the agent to a cell, e.g., a muscle cell, a neuronal cell, or a skin cell.

In one embodiment, evaluating for an effect on age-associated disease includes contacting the agent to a mammal, e.g., a mouse model of age-associated disease. For example, evaluating for an effect on age-associated disease includes testing the mammal with a cognitive test or evaluating the mammal for a symptom of an age-associated disease.

For example, in the case of Alzheimer's disease, the method (using a cell or organism) can include evaluating a secretase protein or mRNA or evaluating secretase activity. The method (using a cell or organism) can include evaluating a APP or a fragment thereof.

A method may include providing a computer model of the structure of a compound and the structure of an AMPK pathway component protein); evaluating compatibility of the models; and evaluating a compound for an effect on age-associated disease. For example, evaluating model compatibility includes evaluating an energy potential or steric compatibility. The method can include other features described herein.

Biomarkers

A method may include: providing a first organism and a second organism, wherein the first and second organism are derived from the same species and differ in endogenous AMPK pathway component activity in one or more cells; and comparing a property associated with a biomolecule in the first organism to a property associated with the biomolecule in the second organism to identify a biomolecule having a preselected value for said property, thereby identifying the biomolecule as AMPK-associated biomarker. The method can be used to identify a biomarker.

The first and second organisms are the same chronological age. The second organism is deficient in an AMPK activity (e.g., the deficiency of the second organism is effected by RNAi or a genetic mutation). The second organism has enhanced AMPK activity. The second organism can express, e.g., a constitutive AMPK polypeptide, e.g., a constitutive AMPK alpha subunit (e.g., mutated or truncated). In another example, the enhancement is effected by treating the organism with an agent that activates AMPK. The method can further include evaluating expression of the biomarker in organisms of different chronological age, or in a calorically restricted organism. The method can include other features described herein.

Genetic Polymorphisms

A method includes genotyping a human gene that encodes an AMPK pathway component, e.g., AMPK alpha, beta, or gamma subunit or another pathway component, and recording information about the genotype in association with information about an age-associated phenotype, e.g., an age-associated disorder or a symptom of aging.

A method includes a) determining the identity of at least one nucleotide in a gene that encodes an AMPK pathway component, of a subject (e.g., a human subject); and b) creating a record which includes information about the identity of the nucleotide and information relating to a parameter about an age-associated disease or an age-associated phenotype of the subject, wherein the parameter is other than the identify of the nucleotide. The method can be used, e.g., for gathering genetic information. In one embodiment, the determining includes evaluating a sample including human genetic material from the subject.

Another method includes: a) evaluating a parameter of a AMPK pathway component molecule from a mammalian subject; b) evaluating an age-related parameter (e.g., a parameter about an age-associated disease or an age-associated phenotype of the subject) wherein the age-related parameter is other than the evaluated parameter for the AMPK pathway component molecule; and c) recording information about the AMPK pathway component molecule parameter and information about the age-related parameter, wherein the information about the molecular parameter and age-related parameter are associated with each other in the database. For example, the age-related parameter is a phenotypic trait of the subject.

The AMPK pathway component molecule is a polypeptide and the AMPK pathway component parameter includes information about a AMPK pathway component polypeptide. The AMPK pathway component molecule is a nucleic acid and the AMPK pathway component parameter includes information about identity of a nucleotide in the AMPK pathway component gene (e.g., genomic nucleic acid, cDNA, or mRNA).

The subject is an embryo, blastocyst, or fetus.

Step b) is performed before or concurrent with step a). The human genetic material includes DNA and/or RNA.

The method can further include comparing the AMPK pathway component parameter to reference information, e.g., information about a corresponding nucleotide from a reference sequence. For example, the reference sequence is from a reference subject who has attained old age, e.g., at least 85, 90, 95, 98, or 100 years of age. In one embodiment, the reference subject did not exhibit age-associated disease, e.g., at least prior to the time at which a nucleic acid from the reference subject was obtained or at least prior to 85, 90, 95, 98, or 100 years of age. The reference subject was cognitively intact, e.g., at least prior to the time at which a nucleic acid from the reference subject was obtained or at least prior to 85, 90, 95, 98, or 100 years of age. The reference sequence is from a reference subject that has age-associated disease (e.g., at an age of onset that is early or late with respect to the norm for that disease). The method further includes comparing the nucleotide to a corresponding nucleotide from a genetic relative or family member (e.g., a parent, grandparent, sibling, progeny, prospective spouse, etc.).

The method further includes evaluating risk or determining diagnosis of age-associated disease in the subject as a function of the genotype.

The method further includes recording information about the AMPK pathway component parameter and age-related parameter, e.g., in a database. For example, the information is recorded in linked fields of a database (e.g., AMPK pathway component parameter is linked to at least one of: corresponding AMPK pathway component parameter and/or data regarding comparison with the reference sequence). The nucleotide can be located in an exon, intron, or regulatory region of the AMPK pathway component gene. For example, the nucleotide is a SNP. The identity of at least one SNP from Table 1 can be evaluated. A plurality of nucleotides (e.g., at least 10, 20, 50, 100, 500, or 1000 nucleotides are ' evaluated (e.g., consecutive or non-consecutive)) in the AMPK pathway component locus are evaluated. A single nucleotide is evaluated.

The method includes one or more of: evaluating a nucleotide position in the AMPK pathway component locus on both chromosomes of the subject; recording the information (e.g., as phased or unphased information); aligning the genotyped nucleotides of the sample and the reference sequence; and identifying nucleotides that differ between the subject nucleotides and the reference sequence.

The method can be repeated for a plurality of subjects (e.g., at least 10, 25, 50, 100, 250, 500 subjects).

The method can include comparing the information of step a) and step b) to information in a database, and evaluating the association of the genotyped nucleotide(s) with age-associated disease.

The age-related parameter is a biochemical parameter, e.g., an assessment of gene or protein expression. For example, the parameter can relate to an protein associated with old age or an age-related disease (e.g.,. a cancer specific antigen, an amyloid protein, growth hormone, insulin, IGF-1, Ab42, or tau). Other examples include non-protein components, e.g., metabolites, cofactors (such as vitamin B12) and nutrients. For example, the assessment can be of blood, plasma, serum, cerebrospinal fluid (CSF), a biopsy, urine, skin, and so forth. In another embodiment, the age-related parameter is an assessment of neurological function, e.g., cognitive function, motor control, reflex speed, etc. The age-related parameter includes a result of a mental examination, a memory test, a behavioral test, a personality test, or other cognitive test. For example, the age-related parameter includes information about a symptom of dementia. For example, the symptom of dementia includes at least one of the following: decline in mental status; loss of recent memory; inability to learn and remember new information; behavioral disorganization; diminished abstract thinking; diminished judgment; and personality changes (e.g., mood swings, irritability).

The age-related parameter is an anatomical feature, e.g., a feature of the brain, cardiovascular symptom, or a tumor. Exemplary methods for evaluating anatomical features include radiological methods, such as X-ray, and multi-dimensional imaging techniques such as MRI or computed topography.

In another example, the age-related parameter includes information about a genetic polymorphism associated with age-associated disease other than a nucleotide polymorphism present in the AMPK pathway component locus. For example, the genetic polymorphism is a polymorphism of a gene encoding: ApoE, presenilin 1, presenilin 2, or APP. The genetic polymorphism can be a nucleotide polymorphism, e.g., a SNP.

The method can further include making a decision about whether to provide an age-associated disease treatment as a function of the AMPK pathway component parameter.

A computer-readable database includes a plurality of records. Each record includes a) a first field which includes information about one or more nucleotides from a AMPK pathway component locus of a subject and; b) a second field which includes information about age-related parameter of the subject. For example, the age-related parameter includes information about a biochemical feature, anatomical feature, or cognitive assessment. For example, the age-related parameter is an age-associated disease diagnosis.

A related database has records that each include a) a first field which includes information about one or more nucleotides from a nucleic acid (e.g., a gene) that encodes an AMPK pathway component of a subject; and b) a second field which includes information about an age-related parameter, e.g., a parameter associated with an age-associated disease or phenotype of the subject. In one embodiment, the parameter relates to a disease of protein aggregation or protein misfolding, e.g., an amyloid disease (e.g., Alzheimer's or Parkinson's) or a neurodegenerative disease. For example, the disease can be mediated at least in part by polyglutamine aggregation, e.g., Huntington's disease.

A method may include a) determining the identity of at least one nucleotide in a gene that encodes an AMPK pathway component for a plurality of subjects who have age-associated disease or are associated with age-associated disease; and b) evaluating the distribution of one or more nucleotide identities for a given position in the gene among or between subjects of the plurality. Evaluating the distribution further includes comparing one or more nucleotide identities to corresponding nucleotides in reference subjects who do not have age-associated disease or who are not associated with age-associated disease. The method can include other features described herein. For example, the reference subjects can be long-lived individuals.

An animal (e.g., a non-human animal e.g., a non-human mammal, or an invertebrate, e.g., a nematode, or fly is disclosed that comprises a modification that alters lifespan regulation and a heterologous protein that includes at least 35 glutamines or a polyglutamine region. The modification that alters lifespan regulation can be a environmental, genetic, or epigenetic modification that affects AMPK activity or the activity of an AMPK pathway component.

For example, the heterologous protein can includes a polyglutamine repeat that includes at least 35 glutamines (e.g., at least 45, 50, 60, 70, or 80 glutamines). In one embodiment, the heterologous protein can also include all or part of a human protein that is a polyglutamine disease protein. For example, the heterologous protein includes at least 50 amino acid of the amino acid sequence of exon 1 of the human Huntingtin protein. Homologues of such human proteins can also be used. In another embodiment, the cell expresses an endogenous protein that includes a polyglutamine repeat that includes at least 35 glutamines. For example, the heterologous protein includes a fluorophore (e.g., the protein is a fluorescent protein, e.g., GFP, YFP, etc.) or other chromophore. For example, the protein can be intrinsically fluorescent.

An example of a genetic alteration is at least one substitution, insertion, or deletion in a gene that encodes an AMPK pathway component, e.g., a genomic copy of a gene. A genetic alteration can also be created by a transgene, e.g., that can over express a transcript, produce an anti-sense transcript, or produce dsRNA. Some genetic alterations also knock-out, e.g., create a deletion or other inactivating mutation in a gene. The animal can include a genetic alteration that alters one or more genes (e.g., two, or three) genes that are involved in age-regulation or are otherwise age-associated.

An example of an epigenetic alteration is e.g., RNA interference (e.g., using dsRNA or siRNA) that are specific for a transcript that encodes an AMPK pathway component.

A method of screening a compound is described (e.g., a small molecule, siRNA, drug, antibody, nucleic acid, gene therapy vector and so fourth). The method includes providing a cell or animal that includes a protein with a polyglutamine region that is prone to aggregation (e.g., more than a corresponding wild-type protein). The cell or animal also has an altered AMPK activity, e.g., by genetic, environmental, or epigenetic modification, e.g., as described herein. For example, the cell or animal can be treated with a pharmacological agent that affects AMPK activity (e.g., metformin), an siRNA specific for an AMPK pathway component message, an antibody, or a heterologous nucleic acid.

The compound is contacted to the cell or animal and a property of the cell or animal is evaluated. For example, the evaluated property can relate to protein aggregation, a neurological (e.g., cognitive) property, or a property of one or more of the AMPK pathway components (e.g., AMPK itself) .

Screening systems in which the control (e.g., no test compound is contacted) has a reduced lifespan or reduce age-associated properties) can provide a useful sensitized system for detecting the ability of a test compound to affect a cell or organism (e.g., to affect polyglutamine aggregation). On the other hand, screening systems in which the control (e.g., no test compound is contacted) has a enhanced lifespan or increase age-associated properties) also can provide a useful system for detecting the ability of a test compound to affect a cell or organism (e.g., to affect polyglutamine aggregation), e.g., by detecting synergies between the test agent and the compound, which may not be apparent in a wild-type scenario.

A non-human organism is disclosed that includes a deficiency in an age-associated protein that participates in the AMPK pathway (e.g., an AMPK pathway component) and a heterologous nucleic acid encoding a protein with a polyglutamine repeat region that includes at least 35 glutamines. The organism can be an invertebrate organism (e.g., a Drosophila or nematode) or a vertebrate organism (e.g., a non-human mammalian organism such as a rodent, e.g., a transgenic rodent). The deficiency is caused by a genetic mutation. In another embodiment, the deficiency is caused by RNAi. The age-associated protein can be a AMPK (e.g.,. AMPK alpha subunit), a protein that is directly or indirectly regulated by AMPK, or a protein that directly regulates AMPK.

Cultured cell preparation may include: an engineered mammalian cell that expresses a protein with a polyglutamine repeat region of at least 35 glutamines and includes a genetic alteration that enhances longevity or that sensitizes the cell (e.g., increases or decreases AMPK activity, etc.). The cell preparation can also include a test compound or a modulator (e.g., an agonist or antagonist) of an age-associated protein. The preparation can be used in a method for evaluating a test compound or a library of test compound. The method can include contacting a test compound to cells in the preparation; and evaluating the cells for aggregation of the protein with the polyglutamine repeats or a symptom of protein aggregation or a symptom of aging.

As used herein, a "polyglutamine region" of a protein is a region of the protein that includes at least 15 consecutive glutamine residues, and is at least 90 or 95% glutamine. Typically, the region is 100% glutamine and includes at least 30, 35, 40, 50, 60, 70, 80, or 90 residues. Regions with greater than 35 glutamines are more prone to aggregation. Absent other factors, the propensity for aggregation increases with repeat length.

An AMPK protein complex (e.g., of $\alpha$, $\beta$, and $\gamma$ subunits) and subunits, derivative, and functional domains thereof are collectively referred to as "AMPK polypeptides" or "AMPK proteins". AMPK pathway proteins, subunits, derivative, and functional domains thereof are collectively referred to as "AMPK pathway polypeptides" or "AMPK pathway proteins". AMPK pathway proteins also include AMPK proteins, AMPK activating proteins, and AMPK substrates. See below for examples. Nucleic acid molecules encoding such polypeptides or proteins are collectively referred to as "AMPK nucleic acids" or "AMPK pathway nucleic acids," respectively. Such nucleic acids include naturally occurring genomic and cDNA sequences, naturally occurring variants, and synthetic sequences (e.g., codon-optimized coding sequences).

As used herein, the term "nucleic acid molecule" includes DNA molecules (e.g., a cDNA or genomic DNA), RNA molecules (e.g., an mRNA) and analogs of the DNA or RNA. A DNA or RNA analog can be synthesized from nucleotide analogs. The nucleic acid molecule can be single-stranded or double-stranded, e.g., double-stranded DNA or a double-stranded RNA. The term "polypeptide" refers to a polymer of three or more amino acids linked by a peptide bond. The polypeptide may include one or more unnatural amino acids. Typically, the polypeptide includes only natural amino acids. The term "peptide" refers to a polypeptide that is between three and thirty-two amino acids in length. A "protein" can include one or more polypeptide chains. Accordingly, the term "protein" encompasses polypeptides and peptides. A protein or polypeptide can also include one or more modifications, e.g., a glycosylation, amidation, phosphorylation, and so forth.

The term "isolated nucleic acid molecule" or "purified nucleic acid molecule" includes nucleic acid molecules that are separated from other nucleic acid molecules present in the natural source of the nucleic acid. For example, with regards to genomic DNA, the term "isolated" includes nucleic acid molecules which are separated from the chromosome with which the genomic DNA is naturally associated. In some embodiments, an "isolated" nucleic acid is free of sequences which naturally flank the nucleic acid (i.e., sequences located at the 5' and/or 3' ends of the nucleic acid) in the genomic DNA of the organism from which the nucleic acid is derived. For example, in various embodiments, the isolated nucleic acid molecule can contain less than about 5 kb, 4 kb, 3 kb, 2 kb, 1 kb, 0.5 kb or 0.1 kb of 5' and/or 3' nucleotide sequences which naturally flank the nucleic acid molecule in genomic DNA of the cell from which the nucleic acid is derived. Examples of flanking sequences include adjacent genes, transposons, and regulatory sequences. Moreover, an "isolated" nucleic acid molecule, such as a cDNA molecule, can be substantially free of other cellular material, of culture medium when produced by recombinant techniques, or of chemical precursors or other chemicals when chemically synthesized.

As used herein, the term "hybridizes under low stringency, medium stringency, high stringency, or very high stringency conditions" describes conditions for hybridization and washing. Guidance for performing hybridization reactions can be found in Current Protocols in Molecular Biology, John Wiley & Sons, N.Y. (1989), 6.3.1-6.3.6. Aqueous and nonaqueous methods are described in that reference and either can be used. Specific hybridization conditions referred to herein are as follows: 1) low stringency hybridization conditions in 6X sodium chloride/sodium citrate (SSC) at about 45˚C, followed by two washes in 0.2X SSC, 0.1% SDS at least at 50˚C (the temperature of the washes can be increased to 55˚C for low stringency conditions); 2) medium stringency hybridization conditions in 6X SSC at about 45˚C, followed by one or more washes in 0.2X SSC, 0.1% SDS at 60˚C; 3) high stringency hybridization conditions in 6X SSC at about 45˚C, followed by one or more washes in 0.2X SSC, 0.1% SDS at 65˚C; and preferably 4) very high stringency hybridization conditions are 0.5 M sodium phosphate, 7% SDS at 65˚C, followed by one or more washes at 0.2X SSC, 1% SDS at 65˚C. Very high stringency conditions (4) are the preferred conditions and the ones that should be used unless otherwise specified. Methods of the invention can include use of an isolated nucleic acid molecule of the invention that hybridizes under a stringency condition described herein to a sequence described herein or use of a polypeptide encoded by such a sequence, e.g., the molecule can be a naturally occurring variant.

As used herein, a "naturally-occurring" nucleic acid molecule refers to an RNA or DNA molecule having a nucleotide sequence that occurs in Nature. For example a naturally occurring nucleic acid molecule can encode a natural protein.

As used herein, the terms "gene" and "recombinant gene" refer to nucleic acid molecules which include at least an open reading frame encoding an AMPK protein subunit or an AMPK pathway protein or subunit, derivative, or functional domain thereof. The gene can optionally further include non-coding sequences, e.g., regulatory sequences and introns. For example, a gene encodes a mammalian an AMPK protein subunit or an AMPK pathway protein or subunit, derivative, or functional domain thereof.

An "isolated" or "purified" polypeptide or protein is substantially free of cellular material or other contaminating proteins from the cell or tissue source from which the protein is derived, or substantially free from chemical precursors or other chemicals when chemically synthesized. "Substantially free" means that the protein of interest in the preparation is at least 10% pure. In an embodiment, the preparation of the protein has less than about 30%, 20%, 10% and more preferably 5% (by dry weight), of a contaminating component (e.g., a protein not of interest, chemical precursors, and so forth). When the protein or biologically active portion thereof is recombinantly produced, it is also preferably substantially free of culture medium, i.e., culture medium represents less than about 20%, more preferably less than about 10%, and most preferably less than about 5% of the volume of the protein preparation. The invention includes isolated or purified preparations of at least 0.01, 0.1, 1.0, and 10 milligrams in dry weight.

A "non-essential" amino acid residue is a residue that can be altered from the wild-type sequence of protein without abolishing or substantially altering activity, e.g., the activity is at least 20%, 40%, 60%, 70% or 80% of wild-type. An "essential" amino acid residue is a residue that, when altered from the wild-type sequence results in abolishing activity such that less than 20% of the wild-type activity is present. Conserved amino acid residues are frequently predicted to be particularly unamenable to alteration.

A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Thus, a predicted nonessential amino acid residue in a protein is preferably replaced with another amino acid residue from the same side chain family. Alternatively, in another embodiment, mutations can be introduced randomly along all or part of a coding sequence, such as by saturation mutagenesis, and the resultant mutants can be screened for biological activity to identify mutants that retain activity. Following mutagenesis, the encoded protein can be expressed recombinantly and the activity of the protein can be determined.

As used herein, a "biologically active portion" or a "functional domain" of a protein includes a fragment of a protein of interest which participates in an interaction, e.g., an intramolecular or an inter-molecular interaction, e.g., a binding or catalytic interaction. An inter-molecular interaction can be a specific binding interaction or an enzymatic interaction (e.g., the interaction can be transient and a covalent bond is formed or broken). An inter-molecular interaction can be between the protein and another protein, between the protein and another compound, or between a first molecule and a second molecule of the protein (e.g., a dimerization interaction). Biologically active portions/functional domains of a protein include peptides comprising amino acid sequences sufficiently homologous to or derived from the amino acid sequence of the protein which include fewer amino acids than the full length, natural protein, and exhibit at least one activity of the natural protein.

Biologically active portions can be identified by a variety of techniques including truncation analysis, site-directed mutagenesis, and proteolysis. Mutants or proteolytic fragments can be assayed for activity by an appropriate biochemical or biological (e.g., genetic) assay. In some embodiments, a functional domain is independently folded.

Exemplary biologically active portions can include at least a minimal enzymatic core domain that has an active site and detectable enzymatic activity in vitro.

Exemplary biologically active portions include between 5-100% of the reference protein, e.g., between 10-99, 10-95, 15-94, 15-90, 20-90, 25-80, 25-70, 25-60, 25-50, 25-40, 5-25, or 75-90% of the reference protein. Biologically active portions can include, e.g., internal deletions, insertions (e.g., of a heterologous sequence), terminal deletions, and substitutions

Typically, biologically active portions comprise a domain or motif with at least one activity of the protein, e.g., a kinase activity, e.g., kinase activity for an AMPK substrate. A biologically active portion/functional domain of a beta/gamma subunit can be a domain that is able to interact with an AMPK alpha subunit. A biologically active portion/functional domain of an AMPK protein subunit or an AMPK pathway protein can be a polypeptide which is, for example, 10, 25, 50, 100, 200 or more amino acids in length. Biologically active portions/functional domain of a protein can be used as targets for developing agents which modulate a lifespan regulation.

A variety of methods can be used to identify an AMPK family member or an AMPK pathway protein family member. For example, a known amino acid sequence of the AMPK protein or AMPK pathway protein can be searched against the GenBank sequence databases (National Center for Biotechnology Information, National Institutes of Health, Bethesda MD), e.g., using BLAST; against Pfam database of HMMs (Hidden Markov Models) (using default parameters for Pfam searching; against the SMART database; or against the ProDom database. For example, the hmmsf program, which is available as part of the HMMER package of search programs, is a family specific default program for MILPAT0063 and a score of 15 is the default threshold score for determining a hit. Alternatively, the threshold score for determining a hit can be lowered (e.g., to 8 bits). A description of the Pfam database can be found in Bateman et al. (2002) Nucleic Acids Res. 30(1):276-280 and Sonhammer et al. (1997) Proteins 28(3):405-420. A description of HMMs can be found, for example, in Gribskov et al.(1990) Meth. Enzymol. 183:146-159; Gribskov et al.(1987) Proc. Natl. Acad. Sci. USA 84:

4355-4358; Krogh et al. (1994) J. Mol. Biol. 235:1501-1531; and Stultz et al.(1993) Protein Sci. 2:305-314. The SMART database contains domains identified by profiling with the hidden Markov models of the HMMer2 search program (R. Durbin et al. (1998) Biological sequence analysis: probabilistic models of proteins and nucleic acids. Cambridge University Press). The database also is annotated and monitored. The ProDom protein domain database consists of an automatic compilation of homologous domains. (Corpet et al. (1999), Nucl. Acids Res. 27:263-267) Current versions of ProDom are built using recursive PSI-BLAST searches (Altschul et al. (1997) Nucleic Acids Res. 25:3389-3402; Gouzy et al. (1999) Computers and Chemistry 23:333-340.) of the SWISS-PROT 38 and TREMBL protein databases. The database automatically generates a consensus sequence for each domain.

Calculations of homology or sequence identity between sequences (the terms are used interchangeably herein) are performed as follows.

To determine the percent identity of two amino acid sequences, or of two nucleic acid sequences, the sequences are aligned for optimal comparison purposes (e.g., gaps can be introduced in one or both of a first and a second amino acid or nucleic acid sequence for optimal alignment and non-homologous sequences can be disregarded for comparison purposes). In a preferred embodiment, the length of a reference sequence aligned for comparison purposes is at least 30%, preferably at least 40%, more preferably at least 50%, 60%, and even more preferably at least 70%, 80%, 90%, 100% of the length of the reference sequence. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology").

The percent identity between the two sequences is a function of the number of identical positions shared by the sequences, taking into account the number of gaps, and the length of each gap, which need to be introduced for optimal alignment of the two sequences.

The comparison of sequences and determination of percent identity between two sequences can be accomplished using a mathematical algorithm. In a preferred embodiment, the percent identity between two amino acid sequences is determined using the Needleman and Wunsch ((1970) J. Mol. Biol. 48:444-453) algorithm which has been incorporated into the GAP program in the GCG software package, using either a Blossum 62 matrix or a PAM250 matrix, and a gap weight of 16, 14, 12, 10, 8, 6, or 4 and a length weight of 1, 2, 3, 4, 5, or 6. In yet another preferred embodiment, the percent identity between two nucleotide sequences is determined using the GAP program in the GCG software package, using the NWSgapdna.CMP matrix and a gap weight of 40, 50, 60, 70, or 80 and a length weight of 1, 2, 3, 4, 5, or 6. A particularly preferred set of parameters (and the one that should be used unless otherwise specified) are a Blossum 62 scoring matrix with a gap penalty of 12, a gap extend penalty of 4, and a frameshift gap penalty of 5.

The percent identity between two amino acid or nucleotide sequences can be determined using the algorithm of Meyers and Miller ((1989) CABIOS, 4:11-17) which has been incorporated into the ALIGN program (version 2.0), using a PAM120 weight residue table, a gap length penalty of 12 and a gap penalty of 4.

The nucleic acid and protein sequences described herein can be used as a "query sequence" to perform a search against public databases to, for example, identify other family members or related sequences. Such searches can be performed using the NBLAST and XBLAST programs (version 2.0) of Altschul, et al. (1990) J. Mol. Biol. 215: 403-10. BLAST nucleotide searches can be performed with the NBLAST program, score =100, wordlength = 12 to obtain nucleotide sequences homologous to nucleic acid molecules of the invention. BLAST protein searches can be performed with the XBLAST program, score = 50, wordlength = 3 to obtain amino acid sequences homologous to protein molecules of the invention. To obtain gapped alignments for comparison purposes, Gapped BLAST can be utilized as described in Altschul et al., (1997) Nucleic Acids Res. 25:3389-3402. When utilizing BLAST and Gapped BLAST programs, the default parameters of the respective programs (e.g., XBLAST and NBLAST) can be used.

Some polypeptides of the present invention can have an amino acid sequence substantially identical to an amino acid sequence described herein. In the context of an amino acid sequence, the term "substantially identical" is used herein to refer to a first amino acid that contains a sufficient or minimum number of amino acid residues that are i) identical to, or ii) conservative substitutions of aligned amino acid residues in a second amino acid sequence such that the first and second amino acid sequences can have a common structural domain and/or common functional activity. Methods of the invention can include use of a polypeptide that includes an amino acid sequence that contains a structural domain having at least about 60%, or 65% identity, likely 75% identity, more likely 85%, 90%, 92%, 94%, 95%, 96%, 97%, 98% or 99% identity to a domain of a polypeptide described herein.

In the context of nucleotide sequence, the term "substantially identical" is used herein to refer to a first nucleic acid sequence that contains a sufficient or minimum number of nucleotides that are identical to aligned nucleotides in a second nucleic acid sequence such that the first and second nucleotide sequences encode a polypeptide having common functional activity, or encode a common structural polypeptide domain or a common functional polypeptide activity. Methods of the invention can include use of a nucleic acid that includes a region at least about 60%, or 65% identity, likely 75% identity, more likely 85%, 90%. 91%, 92%, 93%, 94%, 95%, 96%, 97%, 98% or 99% identity to a nucleic acid sequence described herein, or use of a protein encoded by such nucleic acid.

"Subject," as used herein, refers to human and non-human animals. The term "non-human animals" of the invention includes all vertebrates, e.g., mammals, such as non-human primates (particularly higher primates), sheep, dog, rodent (e.g., mouse or rat), guinea pig, goat, pig, cat, rabbits, cow, and non-mammals, such as chickens, amphibians, reptiles, etc. In a preferred embodiment, the subject is a human. In another embodiment, the subject is an experimental animal or animal suitable as a disease model.

A "purifies preparation of cells", as used herein, refers to an in vitro preparation of cells. In the case cells from multicellular organisms (e.g., plants and animals), a purified preparation of cells is a subset of cells obtained from the organism, not the entire intact organism. In the case of unicellular microorganisms (e.g., cultured cells and microbial cells), it consists of a preparation of at least 10% and more preferably 50% of the subject cells.

The term "recombinant" when used with reference, e.g., to a cell, or nucleic acid, protein, or vector, indicates that the cell, nucleic acid, protein or vector, has been modified by the introduction of a heterologous nucleic acid or protein or the alteration of a native nucleic acid or protein, or that the cell is derived from a cell so modified. Thus, for example, recombinant cells express genes that are not found within the native (non-recombinant) form of the cell or express native genes that are otherwise abnormally expressed, under expressed or not expressed at all.

The term "heterologous" when used with reference to portions of a nucleic acid indicates that the nucleic acid comprises two or more subsequences that are not found in the same relationship to each other in nature. For instance, the nucleic acid is typically recombinantly produced, having two or more sequences from unrelated genes arranged to make a new functional nucleic acid, e.g., a promoter from one source and a coding region from another source. Similarly, a heterologous protein indicates that the protein comprises two or more subsequences that are not found in the same relationship to each other in nature (e.g., a fusion protein).

A "small organic molecule" is an organic molecule of having a molecular weight of less than 5, 2, 1, or 0.5kDa. In many embodiments, such small molecules do not include a peptide bond or a phosphodiester bond. For example, they can be non-polymeric. In some embodiments, the molecule has a molecular weight of at least 50, 100, 200, or 400 Daltons.

"Binding affinity" refers to the apparent dissociation constant or $K_D$. A ligand may, for example, have a binding affinity of at least $10^{-5}$, $10^{-6}$, $10^{-7}$ or $10^{-8}$ M for a particular target molecule. Higher affinity binding of a ligand to a first target relative to a second target can be indicated by a smaller numerical value $K_D^1$ for binding the first target than the numerical value $K_D^2$ for binding the second target. In such cases the ligand has specificity for the first target relative to the second target. The agent may bind specifically to the target, e.g., with an affinity that is at least 2, 5, 10, 100, or 1000 better than for a non-target. For example, an agent can bind to an AMPK pathway component, e.g., AMPK, with a $K_d$ of less than $10^{-5}$, $10^{-6}$, $10^{-7}$ or $10^{-8}$ M. If the agent binds specifically to AMPK, it may bind to a non-target kinase, e.g., to a cyclin dependent kinase with a $K_d$ of greater than 2, 5, 10, 100, or 1000 times its $K_d$ for AMPK.

Binding affinity can be determined by a variety of methods including equilibrium dialysis, equilibrium binding, gel filtration, ELISA, or spectroscopy (e.g., using a fluorescence assay). These techniques can be used to measure the concentration of bound and free ligand as a function of ligand (or target) concentration. The concentration of bound ligand ([Bound]) is related to the concentration of free ligand ([Free]) and the concentration of binding sites for the ligand on the target where (N) is the number of binding sites per target molecule by the following equation:

$$[\text{Bound}] = \text{N} \cdot [\text{Free}]/((1/\text{Ka}) + [\text{Free}])$$

Binding affinities can be measured in PBS at pH 7.0.

The term "chronological age" as used herein refers to time elapsed since a preselected event, such as biological age" as conception, a defined embryological or fetal stage, or, more preferably, birth.

In contrast, the term "biological age" refers to manifestations of the passage of time that is not linearly fixed with the amount of time elapsed. The manifestations of biological aging are varied and may depend on the species of organism, environmental conditions, and, as discussed herein, genotype. Exemplary manifestations of biological aging in mammals include endocrine changes (for example, puberty, menses, changes in fertility or fecundity, menopause, and secondary sex characteristics, such as balding,), metabolic changes (for example, changes in appetite and activity), and immunological changes (for example, changes in resistance to disease). The appearance of mammals also changes with biological age, for example, graying of hair, wrinkling of skin, and so forth. With respect to a different class of animals, the nematode *C. elegans* also has manifestations of biological aging, for example, changes in fecundity, activity, responsiveness to stimuli, and appearance (e.g., change in intestinal autofluorescence and flaccidity). In many cases, the remaining potential lifespan of an individual is a function of its biological age.

The term "deficient" when describing a variant protein or gene refers to a reduced ability to function relative to a reference protein or gene, typically the naturally occurring or "wild-type" form of the protein or gene. For example, a deficient protein may have one or more amino acid substitutions, insertions, or deletions relative to a reference protein.

A deficient protein may also be caused by other factors, e.g., reduced protein levels, altered post-translational modifications and so forth. For example, RNAi can be used to create a protein deficiency. Similarly, a deficient gene may have one or more nucleotide substitutions, insertions, or deletions relative to a reference gene. In addition, other factors can cause a genetic deficiency, e.g., altered chromosomal or extra-chromosomal position or altered chromosomal stability. In a further example, epigenetic factors such as altered methylation, can operate to produce deficiencies.

Other features and advantages of the invention will be apparent from the following detailed description, and from the claims.

Abbreviations

AICAR= 5'-aminoimidazole-4-carboxyamide-riboside
AMP = 5' adenosine monophosphate
ATP = adenosine triphosphate
AMPK = AMP-activated protein kinase
AMPKK = AMPK kinase
ACC = acetyl-CoA carboxylase
GLUT-4
HK = hexokinase
BLAST = Basic Local Alignment Search Tool
HMM = Hidden Markov Model
HMGR = 3-hydroxyl-3-methylglutaryl-CoA reductase (HMGR)
SSC = sodium chloride/sodium citrate

## BRIEF DESCRIPTION OF THE DRAWINGS

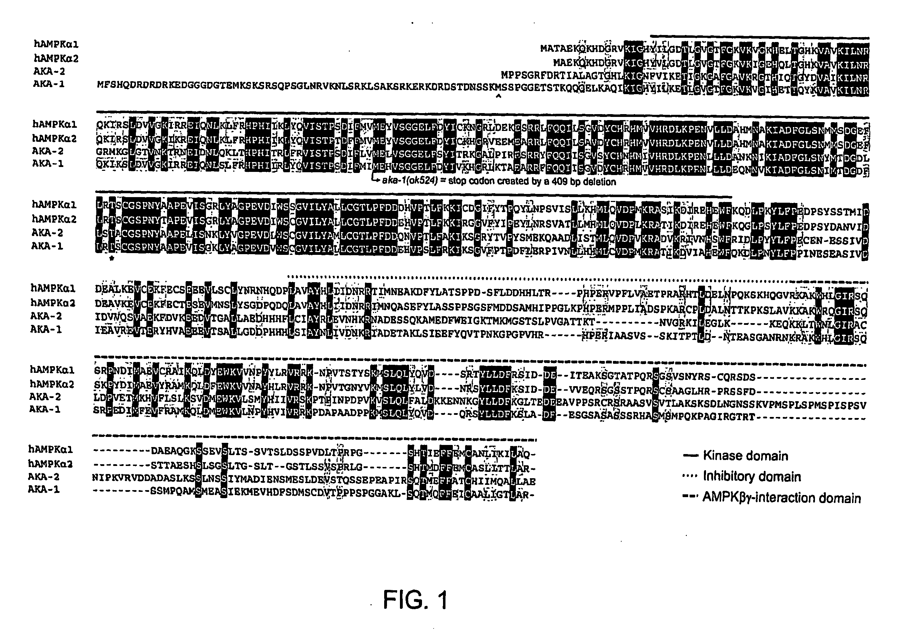

FIG. 1 is an alignment of nematode and human AMPK alpha subunits.

## DETAILED DESCRIPTION

Control of AMPK activity enables interventions that modulate lifespan regulation. Agents which alter lifespan regulation of a cell or organism are identified by screening for compounds that regulate AMPK activity or AMPK pathway activity. The agents so-identified and compounds known to alter AMPK pathway activity can be administered to a subject, e.g., to alter lifespan regulation in the subject or to affect a longevity-associated disorder, or a risk, symptom, predisposition thereof.

For example, lifespan regulation can be modulated to enhance, increase, or otherwise favor increased lifespan. A method to increase lifespan can include modulating AMPK activity or AMPK pathway activity to increase AMPK activity, increase AMPKK activity, increase AMP levels, increase phosphorylation of AMPK, or decrease dephosphorylation of AMPK by protein phosphatase, e.g., protein phosphatase 2C. Related methods can be used to activate physiological processes in an organism that are associated with an organism of reduced chronological age, e.g., a genetically identical or genetically normal organism of reduced chronological age.

Examples of methods for modulating AMPK activity and AMPK pathway activity in cells and organisms are described below as are method of identifying agent which modulate these activities.

AMPK Proteins

The following are exemplary human AMPK amino acid sequences:

| Identifier | Protein Sequence |
|---|---|
| >gi \| 5453964 \| ref \| NP_0 06242.1 \| protein kinase, AMP-activated, alpha 1 catalytic subunit; AMPK alpha 1; Protein kinase, AMP-activated, catalytic, alpha-1 [Homo sapiens] | MATAEKQKHDGRVKIGHYILGDTLGVGTFG KVKVGKHELTGHKVAVKILNRQKIRSLDVV GKIRREIQNLKLFRHPHIIKLYQVISTPSD IFMVMEYVSGGELFDYICKNGRLDEKESRR LFQQILSGVDYCHRHMVVHRDLKPENVLLD AHMNAKIADFGLSNMMSDGEFLRTSCGSPN YAAPEVISGRLYAGPEVDIWSSGVILYALL CGTLPFDDDHVPTLFKKICDGIFYTPQYLN PSVISLLKHMLQVDPMKRASIKDIREHEWF KQDLPKYLFPEDPSYSSTMIDDEALKEVCE KFECSEEEVLSCLYNRNHQDPLAVAYHLII DNRRIMNEAKDFYLATSPPDSFLDDHHLTR PHPERVPFLVAETPRARHTLDELNPQKSKH QGVRKAKWHLGIRSQSRPNDIMAEVCRAIK QLDYEWKVVNPYYLRVRRKNPVTSTYSKMS LQLYQVDSRTYLLDFRSIDDEITEAKSGTA TPQRSGSVSNYRSCQRSDSDAEAQGKSSEV SLTSSVTSLDSSPVDLTPRPGSHTIEFFEM CANLIKILAQ (SEQ ID NO:1) |
| gi \| 19923359 \| ref \| NP_0 06244.2 \| protein kinase, AMP-activated, beta 1 non-catalytic subunit; AMPK beta 1; Protein kinase, AMP-activated, noncatalytic, beta-1 [Homo sapiens] | MGNTSSERAALERHGGHKTPRRDSSGGTKD GDRPKILMDSPEDADLFHSEEIKAPEKEEF LAWQHDLEVNDKAPAQARPTVFRWTGGGKE VYLSGSFNNWSKLPLTRSHNNFVAILDLPE GEHQYKFFVDGQWTHDPSEPIVTSQLGTVN NIIQVKKTDFEVFDALMVDSQKCSDVSELS SSPPGPYHQEPYVCKPEERFRAPPILPPHL LQVILNKDTGISCDPALLPEPNHVMLNHLY ALSIKDGVMVLSATHRYKKKYVTTLLYKPI (SEQ ID NO:2) |
| >gi \| 4885561 \| ref \| NP_0 05390.1 \| protein kinase, AMP-activated, beta 2 | MGNTTSDRVSGERHGAKAARSEGAGGHAPG KEHKIMVGSTDDPSVFSLPDSKLPGDKEFV SWQQDLEDSVKPTQQARPTVIRWSEGGKEV FISGSFNNWSTKIPLIKSHNDFVAILDLPE |
| non-catalytic subunit; AMPK beta 2 [Homo sapiens] | GEHQYKFFVDGQWVHDPSEPVVTSQLGTIN NLIHVKKSDFEVFDALKLDSMESSETSCRD LSSSPPGPYGQEMYAFRSEERFKSPPILPP HLLQVILNKDTNISCDPALLPEPNHVMLNH LYALSIKDSVMVLSATHRYKKKYVTTLLYK PI (SEQ ID NO:3) |

(continued)

| Identifier | Protein Sequence |
|---|---|
| gi \| 1703037 \| sp \|P54619 \|AAKG_HUMAN 5'-AMP-activated protein kinase, gamma-1 subunit (AMPK gamma-1 chain) | METVISSDSSPAVENEHPQETPESNNSVYT SFMKSHRCYDLIPTSSKLVVFDTSLQVKKA FFALVTNGVRAAPLWDSKKQSFVGMLTITD FINILHRYYKSALVQIYELEEHKIETWREV YLQDSFKPLVCISPNASLFDAVSSLIRNKI HRLPVIDPESGNTLYILTHKRILKFLKLFI TEFPKPEFMSKSLEELQIGTYANIAMVRTT TPVYVALGIFVQHRVSALPVVDEKGRVVDI YSKFDVINLAAEKTYNNLDVSVTKALQHRS HYFEGVLKCYLHETLETIINRLVEAEVHRL VVVDENDVVKGIVSLSDILQALVLTGGEKK P (SEQ ID NO:4) |
| gi \| 14285344 \| sp \| Q9UGJ 0 \| AAKH_HUMAN 5'-AMP-activated protein kinase, gamma-2 subunit (AMPK gamma-2 chain) (AMPK gamma2) (H91620p) | MGSAVMDTKKKKDVSSPGGSGGKKNASQKR RSLRVHIPDLSSFAMPLLDGDLEGSGKHSS RKVDSPFGPGSPSKGFFSRGPQPRPSSPMS APVRPKTSPGSPKTVFPFSYQESPPRSPRR MSFSGIFRSSSKESSPNSNPATSPGGIRFF SRSRKTSGLSSSPSTPTQVTKQHTFPLESY KHEPERLENRIYASSSPPDTGQRFCPSSFQ SPTRPPLASPTHYAPSKAAALAAALGPAEA GMLEKLEFEDEAVEDSESGVYMRFMRSHKC YDIVPTSSKLVVFDTTLQVKKAFFALVANG VRAAPLWESKKQSFVGMLTITDFINILHRY YKSPMVQIYELEEHKIETWRELYLQETFKP LVNISPDASLFDAVYSLIKNKIHRLPVIDP ISGNALYILTHKRILKFLQLFMSDMPKPAF MKQNLDELGIGTYHNIAFIHPDTPIIKALN IFVERRISALPVVDESGKVVDIYSKFDVIN LAAEKTYNNLDITVTQALQHRSQYFEGVVK CNKLEILETIVDRIVRAEVHRLVVVNEADS IVGIISLSDILQALILTPAGAKQKETETE (SEQ ID NO:5) |
| gi \| 7705391 \| ref \| NP_05 7287.1 \| protein kinase, AMP-activated, gamma 2 non-catalytic subunit; H91620p protein [Homo sapiens] | MLIAVLLLPLRRRWRRALGPAEAGMLEKLE FEDEAVEDSESGVYMRFMRSHKCYDIVPTS SKLVVFDTTLQVKKAFFALVANGVRAAPLW ESKKQSFVGMLTITDFINILHRYYKSPMVQ IYELEEHKIETWRELYLQETFKPLVNISPD ASLFDAVYSLIKNKIHRLPVIDPISGNALY ILTHKRILKFLQLFMSDMPKPAFMKQNLDE LGIGTYHNIAFIHPDTPIIKALNIFVERRI SALPVVDESGKVVDIYSKFDVINLAAEKTY NNLDITVTQALQHRSQYFEGVVKCNKLEIL ETIVDRIVRAEVHRLVVVNEADSIVGIISL SDILQALILTPAGAKQKETETE (SEQ ID NO:6) |

The kinase region of human AMPK alpha includes amino acids 10 to 280, a region of about 270 amino acids. The C-terminal region of human AMPK alpha can interact with the beta and gamma subunit. For example, the C-terminal region of approximately amino acids 392 to 548 participates in beta and gamma binding. Mutations (e.g., insertions, deletions, and amino acid substations) can be created in these regions and assayed for constitutive activity, e.g., in a cell free or cell-based system, or can be assayed for binding to beta and gamma subunits, e.g., using a two-hybrid assay or co-purification (e.g., Crute et al. (1998) J. Biol. Chem. 273:35347-35354). For example, alanine substitutions can be introduced into the beta-gamma binding region, particularly at conserved amino acid positions to identifying a human AMPK variant that is defective in beta-gamma interaction. In another example, a stop codon can be introduced to produce an N-terminal fragment that is truncated at amino acid 313, or at 392, or at position between amino acid 280 and 392.

A fragment of the AMPK alpha subunit is constitutively active. This fragment includes, for example, amino acids 10 to 270 of SEQ ID NO:1.

AMPK activity can affect gene expression, e.g., by phosphorylating transcription factors such as HNF4$\alpha$ and ChREBP, and the transcriptional coactivator p300. AMPK may also regulate chromatin by phosphorylating histone H3. The numerous effects AMPK can have can be tissue dependent. For example, in the liver, AMPK may increase fatty acid oxidation (ketogenesis), decrease cholesterol synthesis, and decrease lipogenesis. In skeletal muscle, AMPK can increase fatty acid oxidation and increase glucose uptake. In adipocytes, AMPK can decrease lipogenesis and decrease lipolysis. In pancreatic islet, AMPK can modulate insulin secretion. See, e.g., Winder and Hardie (1999) Am. J. Physiol. 277:E1-E10. Substrates of AMPK can include ACC, MCD, and nitric oxide synthetase. See, e.g., J. Appl. Physiol. 91: 1017-1028 (2001).

## C. elegans AMPK Proteins

We identified T01C8.1 as an AMPK alpha homolog, i.e., *aka-1,* and PAR2.3 as another AMPK alpha homolog, i.e., *aka-2,* in *C. elegans.* We found that AMPK antagonizes insulin signaling in this animal. Other *C. elegans* homologs may also participate in lifespan regulation.

Exemplary *C. elegans* protein and nucleic acid sequences are in the [Appendix]

Other AMPK-related proteins include the yeast proteins Snf1p, Snf4p, and Sip1/Sip2/Gal83p.

It is possible to construct chimeric AMPK subunits, e.g., chimeric AMPK alpha subunits. A chimeric polypeptide includes at least one amino acid substitution that replace an amino acid at a given position with an amino acid from a corresponding position in a subunit of another species or isoforms. Typically a contiguous region is substituted, e.g., a region of between 10 and 300, or 50 and 300, or 70 and 250 amino acids. For example, the kinase region of T01C8.1 can be replaced with the kinase region of human AMPK alpha-1.

Additional exemplary sequences are provided in the [Appendix].

## AMPK Pathway members

AMPK Pathway members include AMPK activating proteins, such as AMPKK, and AMPK suppressing proteins such as protein phosphatase 2C, AMPK direct targets, and AMPK indirect targets.

Still other pathway members include AMPK substrates. For example, in liver cells, acetyl-CoA carboxylase (ACC) and 3-hydroxyl-3-methylglutaryl-CoA reductase (HMGR) are AMPK substrates. AMPK activation causes HMGR phosphorylation and inhibition of fatty acid and sterol synthesis. Additional exemplary substrates include malonyl-Co decarboxylase (MCD) and nitric oxide synthetase (NOS).

Peptide substrates that AMPK can phosphorylate include the SAMS substrate HMRSAMSGLHLVKRR (SEQ ID NO:7), a peptide from ACC, and a substrate peptide from HMGR, HLVKSHMIHNRSKINL (SEQ ID NO:8). AMPK may also phosphorylate substrates that generally include a hydrophobic amino acid at the -5 position, and to a lesser stringency a hydrophobic amino acid at the +4 position. The substrate serine is at the +1 position :

```
-5   -4   -3   -2   -1    0    +1   +2   +3   +4

Φ    X    Ψ    X    X   S/T   X    X    X    Φ
```

where X is any amino acid, Φ is hydrophobic, and Ψ is basic. See, e.g., Michell et al. (1996) J. Biol. Chem. 271:28445. Corresponding peptides from other substrates and homologous substrates from other organisms can also be used.

As a result of target phosphorylation, AMPK can cause increased fatty acid oxidation, glucose transport, gene transcription (e.g., GLUT4, hexokinase, and uncoupling protein 3 gene transcription), and increased oxidative enzyme activity. Examples of indirect targets of AMPK include GLUT4, hexokinase, and uncoupling protein 3.

Muscle contraction can increase the concentration of 5'AMP and decrease the concentration of creatine phosphate. These changes can activate AMPKK to phosphorylate AMPK. Phosphorylated AMPK can itself phosphorylate target proteins that decrease glucose uptake and acetyl-CoA carboxylase (ACC) which decreases malonyl-CoA and increases free fatty acid oxidation.

In particular embodiments, the AMPK pathway members are no more than two components removed from AMPK. For example, an upstream component that is no more than two components removed may act through one or two intermediaries to modulate AMPK activity. In some embodiments, the AMPK pathway members are no more than one component removed (e.g., no more than one intermediary). In some embodiments, the AMPK pathway members are intracellular components (e.g., cytoplasmic components or nuclear components).

Exemplary sequences include:

```
>gi|1703034|sp|P54645|AAK1_RAT 5'-AMP-activated protein
kinase, catalytic alpha-1 chain (AMPK alpha-1 chain)
MAEKQKHDGRVKIGHYILGDTLGVGTFGKVKVGKHELTGHKVAVKILNRQKIRSLDV
VGKIRREIQNLKLFRHPHIIKLYQVISTPSDIFMVMEYVSGGELFDYICKNGRLDEK
ESRRLFQQILSGVDYCHRHMVVHRDLKPENVLLDAHMNAKIADFGLSNMMSDGEFLR
TSCGSPNYAAPEVISGRLYAGPEVDIWSSGVILYALLCGTLPFDDDHVPTLFKKICD
GIFYTPQYLNPSVISLLKHMLQVDPMKRATIKDIREHEWFKQDLPKYLFPEDPSYSS
TMIDDEALKEVCEKFECSEEVLSCLYNRNHQDPLAVAYHLIIDNRRIMNEAKDFYL
ATSPPDSFLDDHHLTRPHPERVPFLVAETPRARHTLDELNPQKSKHQGVRKAKWHLG
IRSQSRPNDIMAEVCRAIKQLDYEWKVVNPYYLRVRRKNPVTSTFSKMSLQLYQVDS


RTYLLDFRSIDDEITEAKSGTATPQRSGSISNYRSCQRSDSDAEAQGKPSEVSLTSS
VTSLDSSPVDVAPRPGSHTIEFFEMCANLIKILAQ   (SEQ ID NO:12)



>gi|24181956|gb|AAN47137.1| AMP-activated protein kinase
gamma 3 subunit long form [Mus musculus]
MEPELEHTLPGTLTWSHSGGPESQEMDFLEQGENSWPSPAVATSSERTCAIRGVKAS
RWTRQEAVEEAEPPGLGEGAQSRPAAESTRQEATFPKATPLAQAVPLAEAETSPTGW
DLLLPDCAASAGGSSTGDLELTIEFPAPEAWDCELEGLGKDRPRPGPSPQAPLLGLS
WDDELQKPGAQVYMHFMQEHTCYDAMATSSKLVIFDTTLEIKKAFFAMVANGVRAAP
LWDSKKQSFVGMLTITDFILVLHRYYRSPLVQIYEIEEHKIETWREIYLQGCFKPLV
SISPNDSLFEAVYALIKNRIHRLPVLDPVSGTVLYILTHKRLLKFLHIFGALLPRPS
FLCRTIQDLGIGTFRDLAVVLETAPVLTALDIFVDRRVSALPVVNESGQVVGLYSRF
DVIHLAAQQTYNHLDMSVGEALRQRTLCLEGVLSCQPHESLGEVIDRIAREQVHRLV
LVDETQHLLGVVSLSDILQALVLSPAGIDALSA (SEQ ID NO:13)



>gi|23956234|ref|NP_114075.1| protein kinase, AMP-
activated, beta 1 non-catalytic subunit [Mus musculus]
MGNTSSERAALERQAGHKTPRRDSSGGAKDGDRPKILMDSPEDADIFHSEEIKAPEK
EEFLAWQHDLEANDKAPAQARPTVFRWTGGGKEVYLSGSFNNWSKLPLTRSQNNFVA
ILDLPEGEHQYKFFVDGQWTHDPSEPIVTSQLGTVNNIIQVKKTDFEVFDALMVDSQ
KCSDVSELSSSPPGPYHQEPYMSKPEERFKAPPILPPHLLQVILNKDTGISCDPALL
PEPNHVMLNHLYALSIKDGVMVLSATHRYKKKYVTTLLYKPI (SEQ ID NO:14)
```

>gi|1703037|sp|P54619|AAKG_HUMAN 5'-AMP-activated protein
kinase, gamma-1 subunit (AMPK gamma-1 chain) (AMPKg)
METVISSDSSPAVENEHPQETPESNNSVYTSFMKSHRCYDLIPTSSKLVVFDTSLQV
KKAFFALVTNGVRAAPLWDSKKQSFVGMLTITDFINILHRYYKSALVQIYELEEHKI
ETWREVYLQDSFKPLVCISPNASLFDAVSSLIRNKIHRLPVIDPESGNTLYILTHKR
ILKFLKLFITEFPKPEFMSKSLEELQIGTYANIAMVRTTTPVYVALGIFVQHRVSAL
PVVDEKGRVVDIYSKFDVINLAAEKTYNNLDVSVTKALQHRSHYFEGVLKCYLHETL
ETIINRLVEAEVHRLVVVDENDVVKGIVSLSDILQALVLTGGEKKP (SEQ ID
NO:15)

>gi|1176571|sp|P45894|YNA3_CAEEL Putative
serine/threonine-protein kinase PAR2.3 in chromosome III
MPPSGRFDRTIALAGTGHLKIGNFVIKETIGKGAFGAVKRGTHIQTGYDVAIKILNR
GRMKGLGTVNKTRNEIDNLQKLTHPHITRLFRVISTPSDIFLVMELVSGGELFSYIT
RKGALPIRESRRYFQQIISGVSYCHNHMIVHRDLKPENLLLDANKNIKIADFGLSNY
MTDGDLLSTACGSPNYAAPELISNKLYVGPEVDLWSCGVILYAMLCGTLPFDDQNVP
TLFAKIKSGRYTVPYSMEKQAADLISTMLQVDPVKRADVKRIVNHSWFRIDLPYYLF
PECENESSIVDIDVVQSVAEKTPPEKIIYFFIFRHFLKFDVKEEDVTGALLAEDHHH
FLCIAYRLEVNHKRNADESSQKAMEDFWEIGKTMKMGSTSLPVGATTKSEKSERNVA
KVVGKFSANVGRKILEGLKKE
QKKLTWNLGIRACLDPVETMKHVFLSLKSVDMEWKVLSMYHIIVRSKPTPINPDPVK
VSLQLFALDKKENNKGYLLDFKGLTEDEEAVPPSRCRSRAASVSVTLAKSKSDLNGN
SSKVPMSPLSPMSPISPSVNIPKVRVDDADASLKSSLNSSIYMADIENSMESLDEVS
TQSSEPEAPIRSQTMEFFATCHIIMQALLAE (SEQ ID NO:16)

>gi|22096326|sp|P80386|AAKB_RAT 5'-AMP-activated protein
kinase, beta-1 subunit (AMPK beta-1 chain) (AMPKb) (40
kDa subunit)
MGNTSSERAALERQAGHKTPRRDSSGGTKDGDRPKILMDSPEDADIFHTEEMKAPEK
EEFLAWQHDLEVNEKAPAQARPTVFRWTGGGKEVYLSGSFNNWSKLPLTRSQNNFVA
ILDLPEGEHQYKFFVDGQWTHDPSEPIVTSQLGTVNNIIQVKKTDFEVFDALMVDSQ
KCSDVSELSSSPPGPYHQEPYISKPEERFKAPPILPPHLLQVILNKDTGISCDPALL
PEPNHVMLNHLYALSIKDGVMVLSATHRYKKKYVTTLLYKPI (SEQ ID NO:17)

>gi|1155267|gb|AAC52355.1| 5'-AMP-activated protein
kinase alpha-1 catalytic subunit [Rattus norvegicus]

MAEKQKHDGRVKIGHYILGDTLGVGTFGKVKVGKHELTGHKVAVKILNRQKIRSLDV
VGKIRREIQNLKLFRHPHIIKLYQVISTPSDIFMVMEYVSGGELFDYICKNGRLDEK
ESRRLFQQILSGVDYCHRHMVVHRDLKPENVLLDAHMNAKIADFGLSNMMSDGEFLR
TSCGSPNYAAPEVISGRLYAGPEVDIWSSGVILYALLCGTLPFDDDHVPTLFKKICD
GIFYTPQYLNPSVISLLKHMLQVDPMKRATIKDIREHEWFKQDLPKYLFPEDPSYSS
TMIDDEALKEVCEKFECSEEEVLSCLYNRNHQDPLAVAYHLIIDNRRIMNEAKDFYL
ATSPPDSFLDDHHLTRPHPERVPFLVAETPRARHTLDELNPQKSKHQGVRKAKWHLG
IRSQSRPNDIMAEVCRAIKQLDYEWKVVNPYYLRVRRKNPVTSTFSKMSLQLYQVDS
RTYLLDFRSIDDEITEAKSGTATPQRSGSISNYRSCQRSDSDAEAQGKPSEVSLTSS
VTSLDSSPVDVAPRPGSHTIEFFEMCANLIKILAQ (SEQ ID NO:18)


>gi|22096265|sp|Q9R078|AAKB_MOUSE 5'-AMP-activated
protein kinase, beta-1 subunit (AMPK beta-1 chain)
(AMPKb)
MGNTSSERAALERQAGHKTPRRDSSGGAKDGDRPKILMDSPEDADIFHSEEIKAPEK
EEFLAWQHDLEAN
DKAPAQARPTVFRWTGGGKEVYLSGSFNNWSKLPLTRSQNNFVAILDLPEGEHQYKF
FVDGQWTHDPSEP
IVTSQLGTVNNIIQVKKTDFEVFDALMVDSQKCSDVSELSSSPPGPYHQEPYMSKPE
ERFKAPPILPPHL
LQVILNKDTGISCDPALLPEPNHVMLNHLYALSIKDGVMVLSATHRYKKKYVTTLLY
KPI (SEQ ID NO:19)


>gi|14285344|sp|Q9UGJ0|AAKH_HUMAN 5'-AMP-activated
protein kinase, gamma-2 subunit (AMPK gamma-2 chain)
(AMPK gamma2) (H91620p)
MGSAVMDTKKKKDVSSPGGSGGKKNASQKRRSLRVHIPDLSSFAMPLLDGDLEGSGK
HSSRKVDSPFGPGSPSKGFFSRGPQPRPSSPMSAPVRPKTSPGSPKTVFPFSYQESP
PRSPRRMSFSGIFRSSSKESSPNSNPATSPGGIRFFSRSRKTSGLSSSPSTPTQVTK
QHTFPLESYKHEPERLENRIYASSSPPDTGQRFCPSSFQSPTRPPLASPTHYAPSKA
AALAAALGPAEAGMLEKLEFEDEAVEDSESGVYMRFMRSHKCYDIVPTSSKLVVFDT
TLQVKKAFFALVANGVRAAPLWESKKQSFVGMLTITDFINILHRYYKSPMVQIYELE
EHKIETWRELYLQETFKPLVNISPDASLFDAVYSLIKNKIHRLPVIDPISGNALYIL
THKRILKFLQLFMSDMPKPAFMKQNLDELGIGTYHNIAFIHPDTPIIKALNIFVERR
ISALPVVDESGKVVDIYSKFDVINLAAEKTYNNLDITVTQALQHRSQYFEGVVKCNK


LEILETIVDRIVRAEVHRLVVVNEADSIVGIISLSDILQALILTPAGAKQKETETE
(SEQ ID NO:20)

>gi|14194425|sp|Q9Y478|AAKB_HUMAN 5'-AMP-activated
protein kinase, beta-1 subunit (AMPK beta-1 chain)
(AMPKb)
MGNTSSERAALERHGGHKTPRRDSSGGTKDGDRPKILMDSPEDADLFHSEEIKAPEK
EEFLAWQHDLEVNDKAPAQARPTVFRWTGGGKEVYLSGSFNNWSKLPLTRSHNNFVA
ILDLPEGEHQYKFFVDGQWTHDPSEPIVTSQLGTVNNIIQVKKTDFEVFDALMVDSQ
KCSDVSELSSSPPGPYHQEPYVCKPEERFRAPPILPPHLLQVILNKDTGISCDPALL
PEPNHVMLNHLYALSIKDGVMVLSATHRYKKKYVTTLLYKPI (SEQ ID NO:21)


>gi|3912957|sp|O43741|AAKC_HUMAN 5'-AMP-activated protein
kinase, beta-2 subunit (AMPK beta-2 chain)
MGNTTSDRVSGERHGAKAARSEGAGGHAPGKEHKIMVGSTDDPSVFSLPDSKLPGDK
EFVSWQQDLEDSVKPTQQARPTVIRWSEGGKEVFISGSFNNWSTKIPLIKSHNDFVA
ILDLPEGEHQYKFFVDGQWVHDPSEPVVTSQLGTINNLIHVKKSDFEVFDALKLDSM
ESSETSCRDLSSSPPGPYGQEMYAFRSEERFKSPPILPPHLLQVILNKDTNISCDPA
LLPEPNHVMLNHLYALSIKDSVMVLSATHRYKKKYVTTLLYKPI (SEQ ID NO:
22)


>gi|20178277|sp|Q13131|AAK1_HUMAN 5'-AMP-activated
protein kinase, catalytic alpha-1 chain (AMPK alpha-1
chain)
MATAEKQKHDGRVKIGHYILGDTLGVGTFGKVKVGKHELTGHKVAVKILNRQKIRSL
DVVGKIRREIQNLKLFRHPHIIKLYQVISTPSDIFMVMEYVSGGELFDYICKNGRLD
EKESRRLFQQILSGVDYCHRHMVVHRDLKPENVLLDAHMNAKIADFGLSNMMSDGEF
LRTSCGSPNYAAPEVISGRLYAGPEVDIWSSGVILYALLCGTLPFDDDHVPTLFKKI
CDGIFYTPQYLNPSVISLLKHMLQVDPMKRASIKDIREHEWFKQDLPKYLFPEDPSY
SSTMIDDEALKEVCEKFECSEEEVLSCLYNRNHQDPLAVAYHLIIDNRRIMNEAKDF
YLATSPPDSFLDDHHLTRPHPERVPFLVAETPRARHTLDELNPQKSKHQGVRKAKWH
LGIRSQSRPNDIMAEVCRAIKQLDYEWKVVNPYYLRVRRKNPVTSTYSKMSLQLYQV
DSRTYLLDFRSIDDEITEAKSGTATPQRSGSVSNYRSCQRSDSDAEAQGKSSEVSLT
SSVTSLDSSPVDLTPRPGSHTIEFFEMCANLIKILAQ (SEQ ID NO:23)

>gi|20178276|sp|P54646|AAK2_HUMAN 5'-AMP-activated
protein kinase, catalytic alpha-2 chain (AMPK alpha-2
chain)
MAEKQKHDGRVKIGHYVLGDTLGVGTFGKVKIGEHQLTGHKVAVKILNRQKIRSLDV
VGKIKREIQNLKLFRHPHIIKLYQVISTPTDFFMVMEYVSGGELFDYICKHGRVEEM
EARRLFQQILSAVDYCHRHMVVHRDLKPENVLLDAHMNAKIADFGLSNMMSDGEFLR
TSCGSPNYAAPEVISGRLYAGPEVDIWSCGVILYALLCGTLPFDDEHVPTLFKKIRG
GVFYIPEYLNRSVATLLMHMLQVDPLKRATIKDIREHEWFKQDLPSYLFPEDPSYDA
NVIDDEAVKEVCEKFECTESEVMNSLYSGDPQDQLAVAYHLIIDNRRIMNQASEFYL
ASSPPSGSFMDDSAMHIPPGLKPHPERMPPLIADSPKARCPLDALNTTKPKSLAVKK
AKWHLGIRSQSKPYDIMAEVYRAMKQLDFEWKVVNAYHLRVRRKNPVTGNYVKMSLQ
LYLVDNRSYLLDFKSIDDEVVEQRSGSSTPQRSCSAAGLHRPRSSFDSTTAESHSLS
GSLTGSLTGSTLSSVSPRLGSHTMDFFEMCASLITTLAR (SEQ ID NO:24

)>gi|3912959|sp|O54950|AAKG_MOUSE 5'-AMP-activated
protein kinase, gamma-1 subunit (AMPK gamma-1 chain)
(AMPKg)MESVAAESSPALENEHFQETPESNNSVYTSFMKSHRCYDLIPTSSKLVVF
DTSLQVKKAFFALVTNGVRAAPLWDSKKQCFVGMLTITDFINILHRYYKSALVQIYE
LEEHKIETWREVYLQDSFKPLVCISPNASSFDAVSSLIRNKIHRLPVIDPESGNTLY
ILTHKRILKFLKLFIIEFPKPEFMSKSLQELQIGTYANIAMVRTTTPVYVALGIFVQ
HRVSALPVVDEKGRVVDIYSKFDVINLAAEKTYNNLDVSVTKALXHRSHYFEGVLKC
YLHETLETIINRLVEAEVHRLVVVDEHXXVKGIVSLSDILQDLVLTGGEKKP (SEQ
ID NO:25)

>gi|14194424|sp|Q9UGI9|AAKI_HUMAN 5'-AMP-activated
protein kinase, gamma-3 subunit (AMPK gamma-3 chain)
(AMPK gamma3)
MSFLEQENSSSWPSPAVTSSSERIRGKRRAKALRWTRQKSVEEGEPPGQGEGPRSRP
TAESTGLEATFPKTTPLAQADPAGVGTPPTGWDCLPSDCTASAAGSSTDDVELATEF
PATEAWECELEGLLEERPALCLSPQAPFPKLGWDDELRKPGAQIYMRFMQEHTCYDA
MATSSKLVIFDTMLEIKKAFFALVANGVRAAPLWDSKKQSFVGMLTITDFILVLHRY
YRSPLVQIYEIEQHKIETWREIYLQGCFKPLVSISPNDSLFEAVYTLIKNRIHRLPV
LDPVSGNVLHILTHKRLLKFLHIFGSLLPRPSFLYRTIQDLGIGTFRDLAVVLETAP
ILTALDIFVDRRVSALPVVNECGQVVGLYSRFDVIHLAAQQTYNHLDMSVGEALRQR
TLCLEGVLSCQPHESLGEVIDRIAREQVHRLVLVDETQHLLGVVSLSDILQALVLSP
AGIDALGA (SEQ ID NO:26)

>gi|14194420|sp|Q9QZH4|AAKC_RAT 5'-AMP-activated protein kinase, beta-2 subunit (AMPK beta-2 chain)
MGNTTSERVSGERHGAKAARAEGGGHGPGKEHKIMVGSTDDPSVFSLPDSKLPGDKE
FVPWQQDLDDSVKPTQQARPTVIRWSEGGKEVFISGSFNNWSTKIPLIKSHNDFVAI
LDLPEGEHQYKFFVDGQWVHDPSEPVVTSQLGTINNLIHVKKSDFEVFDALKLDSME
SSETSCRDLSSSPPGPYGQEMYVFRSEERFKSPPILPPHLLQVILNKDTNISCDPAL
LPEPNHVMLNHLYALSTKDSVMVLSATHRYKKKYVTTLLYKPI (SEQ ID NO:27
)

>gi|2507205|sp|P80385|AAKG_RAT 5'-AMP-activated protein kinase, gamma-1 subunit (AMPK gamma-1 chain) (AMPKg)
MESVAAESAPAPENEHSQETPESNSSVYTTFMKSHRCYDLIPTSSKLVVFDTSLQVK
KAFFALVTNGVRAAPLWDSKKQSFVGMLTITDFINILHRYYKSALVQIYELEEHKIE
TWREVYLQDSFKPLVCISPNASLFDAVSSLIRNKIHRLPVIDPESGNTLYILTHKRI
LKFLKLFITEFPKPEFMSKSLEELQIGTYANIAMVRTTTPVYVALGIFVQHRVSALP
VVDEKGRVVDIYSKFDVINLAAEKTYNNLDVSVTKALQHRSHYFEGVLKCYLHETLE
AIINRLVEAEVHRLVVVDEHDVVKGIVSLSDILQALVLTGGEKKP (SEQ ID NO:
28)

>gi|17554376|ref|NP_498933.1| Protein kinase [Caenorhabditis elegans]
MPPSGRFDRTIALAGTGHLKIGNFVIKETIGKGAFGAVKRGTHIQTGYDVAIKILNR
GRMKGLGTVNKTRNEIDNLQKLTHPHITRLFRVISTPSDIFLVMELVSGGELFSYIT
RKGALPIRESRRYFQQIISGVSYCHNHMIVHRDLKPENLLLDANKNIKIADFGLSNY
MTDGDLLSTACGSPNYAAPELISNKLYVGPEVDLWSCGVILYAMLCGTLPFDDQNVP

TLFAKIKSGRYTVPYSMEKQAADLISTMLQVDPVKRADVKRIVNHSWFRIDLPYYLF
PECENESSIVDIDVVQSVAEKTPPEKIIYFFIFRHFLKFDVKEEDVTGALLAEDHHH
FLCIAYRLEVNHKRNADESSQKAMEDFWEIGKTMKMGSTSLPVGATTKSEKSERNVA
KVVGKFSANVGRKILEGLKKEQKKLTWNLGIRACLDPVETMKHVFLSLKSVDMEWKV
LSMYHIIVRSKPTPINPDPVKVSLQLFALDKKENNKGYLLDFKGLTEDEEAVPPSRC
RSRAASVSVTLAKSKSDLNGNSSKVPMSPLSPMSPISPSVNIPKVRVDDADASLKSS
LNSSIYMADIENSMESLDEVSTQSSEPEAPIRSQTMEFFATCHIIMQALLAE (SEQ
ID NO:29)

>gi|2766685|gb|AAB95475.1| AMP activated protein kinase
[Mus musculus]
MESVAAESSPALENEHFQETPESNNSVYTSFMKSHRCYDLIPTSSKLVVFDTSLQVK
KAFFALVTNGVRAAPLWDSKKQCFVGMLTITDFINILHRYYKSALVQIYELEEHKIE
TWREVYLQDSFKPLVCISPNASSFDAVSSLIRNKIHRLPVIDPESGNTLYILTHKRI
LKFLKLFIIEFPKPEFMSKSLQELQIGTYANIAMVRTTTPVYVALGIFVQHRVSALP
VVDEKGRVVDIYSKFDVINLAAEKTYNNLDVSVTKALXHRSHYFEGVLKCYLHETLE
TIINRLVEAEVHRLVVVDEHXXVKGIVSLSDILQDLVLTGGEKKP (SEQ ID NO:
30)

>gi|630705|pir||S44859 serine/threonine-specific protein
kinase (EC 2.7.1.-) PAR2.3 - Caenorhabditis elegans
MPPSGRFDRTIALAGTGHLKIGNFVIKETIGKGAFGAVKRGTHIQTGYDVAIKILNR
GRMKGLGTVNKTRNEIDNLQKLTHPHITRLFRVISTPSDIFLVMELVSGGELFSYIT
RKGALPIRESRRYFQQIISGVSYCHNHMIVHRDLKPENLLLDANKNIKIADFGLSNY
MTDGDLLSTACGSPNYAAPELISNKLYVGPEVDLWSCGVILYAMLCGTLPFDDQNVP
TLFAKIKSGRYTVPYSMEKQAADLISTMLQVDPVKRADVKRIVNHSWFRIDLPYYLF
PECENESSIVDIDVVQSVAEKTPPEKIIYFFIFRHFLKFDVKEEDVTGALLAEDHHH
FLCIAYRLEVNHKRNADESSQKAMEDFWEIGKTMKMGSTSLPVGATTKSEKSERNVA
KVVGKFSANVGRKILEGLKKEQKKLTWNLGIRACLDPVETMKHVFLSLKSVDMEWKV
LSMYHIIVRSKPTPINPDPVKVSLQLFALDKKENNKGYLLDFKGLTEDEEAVPPSRC
RSRAASVSVTLAKSKSDLNGNSSKVPMSPLSPMSPISPSVNIPKVRVDDADASLKSS
LNSSIYMADIENSMESLDEVSTQSSEPEAPIRSQTMEFFATCHIIMQALLAE (SEQ
ID NO:31)

>gi|7509905|pir||T31528 protein Y47D3A.15 -
Caenorhabditis elegans
MGNNQSGGPDARYGGPNDKAGLRRHRMMSETAKIAGQVLPNPDGGPPMIFDDGNEDK
SGECPVVFRWSFTQNAQPRVVHIVGSWDNWQTRIPMVKSTNDFSTIIDLQPGQYEYK
FQVDGSWVVDDNQGKAQDVHGNENNMINIQDSDFAVFEALDEDFQSSTAGEVLRGES
ESTKNHDTPNDRELEKLRSFTQEIPSMDMLRKAAGPPVIPPQLMQVLLNKETPESCD
PNVLPEPNHVMLNHMYALSIKDSVMVLSSTQRYRKKFVTTLLYKPV (SEQ ID
NO:32)

>gi|7508080|pir||T25899 protein T20F7.6 - Caenorhabditis
elegans
MSSFKDIHHQRISHMTGSKSTTMTESDEVLPKTPNDKEAFARLLWINQCYEAMPSSS
KMVVFDQGLLMHKAFNGLLAQSTRHVLLSDPDFGGKLDGILSVTDFIKVMLKIYRER
TKCEKESTELDMTQIANEEIGNLSIRQYRELVKKEGNLRPLVSVDASGSLLDAACIL

AEHRVHRIPVIDPLDGSALFILTHKRILKFLWLFGKHLAPLEYLHKSPKELGIGTWS
GIRVVFPDTQLVDCLDILLNKGVSGLPVVERETFKVVDMYSRFDAVGIALENRLDIT
VKEALAFKSQGGPMKNDERVVSVRDNESFWKAVNVLVDHNVHRLCAVNEHGGIEGVI
SLSDVINFMVVQPGSHLRNITAPKKHWARHHTGDMNDKLGKVPRMLKVVHTSPPFSS
FSWSSERFFSPTLPTLSTSRQIQAVRPTRHCQATRIATQNPQHIITEITSFYVFIPF
LRFFFLFCLTQPYNVARHIQKV (SEQ ID NO:33)


>gi|7504313|pir||T22739 protein F55F3.1 - Caenorhabditis
elegans


MGNNQPGGMYKRDRPYDSEKSGSHSRSRGGIPSSSPSNEDECPVQMKIAKGDDKSKF
PVVFKWNINNATPRQVYICGSWDGWNTKIPLVKSTSDFSTIVDLEPGKHEYKFMVDS
KWVVDDNQQKTGNNLGGENNVVMIDEADFEVFDALDKDLASSNAGEALRNSHPTKES
HDTPNDRELEKLHQFGQETPTRVDFNKAAAPPVLPPHLLQVILNKDTPVQCDPNVLP
EPDHVMLNHLYALSIKDGVMVLSATHRYRKKFVTTLLYKPI (SEQ ID NO:34)


>gi|7506849|pir||T29858 protein T01C8.1 - Caenorhabditis
elegans
MSSPGGETSTKQQQELKAQIKIGHYILKETLGVGTFGKVKVGIHETTQYKVAVKILN
RQKIKSLDVVGKIRREIQNLSLFRHPHIIRLYQVISTPSDIFMIMEHVSGGELFDYI
VKHGRLKTAEARRFFQQIISGVDYCHRHMVVHRDLKPENLLLDEQNNVKIADFGLSN
IMTDGDFLRTSCGSPNYAAPEVISGKLYAGPEVDVWSCGVILYALLCGTLPFDDEHV
PSLFRKIKSGVFPTPDFLERPIVNLLHHMLCVDPMKRATIKDVIAHEWFQKDLPNYL
FPPINESEASIVDIEAVREVTERYHVAEEEVTSALLGDDPHHHLSIAYNLIVDNKRI
ADETAKLSIEEFYQVTPNKGPGPVHRHPERIAASVSSKITPTLDNTEASGANRNKRA
KWHLGIRSQSRPEDIMFEVFRAMKQLDMEWKVLNPYHVIVRRKPDAPAADPPKMSLQ
LYQVDQRSYLLDFKSLADEESGSASASSSRHASMSMPQKPAGIRGTRTSSMPQAMSM
EASIEKMEVHDFSDMSCDVTPPPSPGGAKLSQTMQFFEICAALIGTLAR (SEQ ID
NO:35)

>gi|728758|sp|Q09137|AAK2_RAT 5'-AMP-ACTIVATED PROTEIN
KINASE, CATALYTIC ALPHA-2 CHAIN (AMPK ALPHA-2
CHAIN)MAEKQKHDGRVKIGHYVLGDTLGVGTFGKVKIGEHQLTGHKVAVKILNRQK
IRSLDVVGKIKREIQNLKLFRHPHIIKLYQVISTPTDFFMVMEYVSGGELFDYICKH
GRVEEVEARRLFQQILSAVDYCHRHMVVHRDLKPENVLLDAQMNAKIADFGLSNMMS
DGEFLRTSCGSPNYAAPEVISGRLYAGPEVDIWSCGVILYALLCGTLPFDDEHVPTL
FKKIRGGVFYIPEYLNRSIATLLMHMLQVDPLKRATIKDIREHEWFKQDLPSYLFPE
DPSYDANVIDDEAVKEVCEKFECTESEVMNSLYSGDPQDQLAVAYHLIIDNRRIMNQ
ASEFYLASSPPTGSFMDDMAMHIPPGLKPHPERMPPLIADSPKARCPLDALNTTKPK
SLAVKKAKWHLGIRSQSKPYDIMAEVYRAMKQLDFEWKVVNAYHLRVRRKNPVTGNY
VKMSLQLYLVDNRSYLLDFKSIDDEVVEQRSGSSTPQRSCSAAGLHRPRSSVDSSTA
ENHSLSGSLTGSLTGSTLSSASPRLGSHTMDFFEMCASLITALAR (SEQ ID NO:
36)

>gi|488376|emb|CAA82620.1| AMP-activated protein kinase
[Rattus norvegicus]
MAEKQKHDGRVKIGHYVLGDTLGVGTFGKVKIGEHQLTGHKVAVKILNRQKIRSLDV
VGKIKREIQNLKLFRHPHIIKLYQVISTPTDFFMVMEYVSGGELFDYICKHGRVEEV

EARRLFQQILSAVDYCHRHMVVHRDLKPENVLLDAQMNAKIADFGLSNMMSDGEFLR
TSCGSPNYAAPEVISGRLYAGPEVDIWSCGVILYALLCGTLPFDDEHVPTLFKKIRG
GVFYIPEYLNRSIATLLMHMLQVDPLKRATIKDIREHEWFKQDLPSYLFPEDPSYDA
NVIDDEAVKEVCEKFECTESEVMNSLYSGDPQDQLAVAYHLIIDNRRIMNQASEFYL
ASSPPTGSFMDDMAMHIPPGLKPHPERMPPLIADSPKARCPLDALNTTKPKSLAVKK
AKWHLGIRSQSKPYDIMAEVYRAMKQLDFEWKVVNAYHLRVRRKNPVTGNYVKMSLQ
LYLVDNRSYLLDFKSIDDEVVEQRSGSSTPQRSCSAAGLHRPRSSVDSSTAENHSLS
GSLTGSLTGSTLSSASPRLGSHTMDFFEMCASLITALAR (SEQ ID NO:37)

>gi|758367|gb|AAA64745.1| AMP-activated protein kinase
MAEKQKHDGRVKIGHYVLGDTLGVGTFGKVKIGEHQLTGHKVAVKILNRQKIRSLDV
VGKIKREIQNLKLFRHPHIIKLYQVISTPTDFFMVMEYVSGGELFDYICKHGRVEEM
EARRLFQQILSAVDYCHRHMVVHRDLKPENVLLDAHMNAKIADFGLSNMMSDGEFLR
TSCGSPNYTAPEVISGRLYAGPEVDIWSCGVILYALLCGTLPFDDEHVPTLFKKIRG
GVFYIPEYLNRSVATLLMHMLQVDPLKRATIKDIREHEWFKQGLPSYLFPEDPSYDA
NVIDDEAVKEVCEKFECTESEVMNSLYSGDPQDQLAVAYHLIIDNRRIMNQASEFYL
ASSPPSGSFMDDSAMHIPPGLKPHPERMPPLIADSPKARCPLDALNTTKPKSLAVKK
AKWRQGIRSQSKPYDIMAEVYRAMKQLDFEWKVVNAYHLRVRRKNPVTGNYVKMSLQ
LYLVDNRSYLLDFKSIDDEVVEQRSGSSTPQRSCSAAGLHRPRSSFDSTTAESHSLS
GSLTGSLTGSTLSSVSPRLGSHTMDFFEMCASLITTLAR (SEQ ID NO:38)

>gi|21450520|gb|AAM54161.1|U00025_3 protein PAR2.3b
[Caenorhabditis elegans]
MTDGDLLSTACGSPNYAAPELISNKLYVGPEVDLWSCGVILYAMLCGTLPFDDQNVP
TLFAKIKSGRYTVPYSMEKQAADLISTMLQVDPVKRADVKRIVNHSWFRIDLPYYLF
PECENESSIVDIDVVQSVAEKTPPEKIIYFFIFRHFLKFDVKEEDVTGALLAEDHHH
FLCIAYRLEVNHKRNADESSQKAMEDFWEIGKTMKMGSTSLPVGATTKSEKSERNVA
KVVGKFSANVGRKILEGLKKEQKKLTWNLGIRACLDPVETMKHVFLSLKSVDMEWKV
LSMYHIIVRSKPTPINPDPVKVSLQLFALDKKENNKGYLLDFKGLTEDEEAVPPSRC
RSRAASVSVTLAKSKSDLNGNSSKVPMSPLSPMSPISPSVNIPKVRVDDADASLKSS
LNSSIYMADIENSMESLDEVSTQSSEPEAPIRSQTMEFFATCHIIMQALLAE (SEQ
ID NO:39)

>gi|13548459|emb|CAC35836.1| protein Y111B2A.8
[Caenorhabditis elegans]
MKAHKCYDLIPTSSKLVVFDTHLPVRKAFYALVYNGVRAAPLWDTDNQRFTGMLTIT
DFIKILCKHYDKGDNSERIRALEDQQISHWRDQFELDGTLRPFVYIDPNESLHRAVE
LLCESKVHRLPVLDRKTGNITYILTHKRIMKFLSLYMRDLPRPSFMSCTPRELGIGA
WGDILCCHVDTPIHDALELFLKNRVSALPLIDENGRVVDIYAKFDVISLAAESSYDK
LDCTVQEALQHRSEWFEGVQTCLETDSLFQVLEAIVKAEVHRLIVTDQDKKVVGVVS
LSDILKNLVLDPCQKPPPPPPQSQQAGGGGPPTRNASGTSTGGASSSDSPPHSIPEG
IEVEDDDDDDEEAPPPSIDCSTPGPSSAAT (SEQ ID NO:40)

>gi|5832839|emb|CAB55074.1| protein Y47D3A.15
[Caenorhabditis elegans]
MGNNQSGGPDARYGGPNDKAGLRRHRMMSETAKIAGQVLPNPDGGPPMIFDDGNEDK
SGECPVVFRWSFTQNAQPRVVHIVGSWDNWQTRIPMVKSTNDFSTIIDLQPGQYEYK
FQVDGSWVVDDNQGKAQDVHGNENNMINIQDSDFAVFEALDEDFQSSTAGEVLRGES

ESTKNHDTPNDRELEKLRSFTQEIPSMDMLRKAAGPPVIPPQLMQVLLNKETPESCD
PNVLPEPNHVMLNHMYALSIKDSVMVLSSTQRYRKKFVTTLLYKPV (SEQ ID
NO:41)

>gi|3877643|emb|CAB04480.1| protein F55F3.1
[Caenorhabditis elegans]
MGNNQPGGMYKRDRPYDSEKSGSHSRSRGGIPSSSPSNEDECPVQMKIAKGDDKSKF
PVVFKWNINNATPRQVYICGSWDGWNTKIPLVKSTSDFSTIVDLEPGKHEYKFMVDS
KWVVDDNQQKTGNNLGGENNVVMIDEADFEVFDALDKDLASSNAGEALRNSHPTKES
HDTPNDRELEKLHQFGQETPTRVDFNKAAAPPVLPPHLLQVILNKDTPVQCDPNVLP
EPDHVMLNHLYALSIKDGVMVLSATHRYRKKFVTTLLYKPI (SEQ ID NO:42)

>gi|458887|gb|AAA50618.1| protein PAR2.3a [Caenorhabditis elegans]
MPPSGRFDRTIALAGTGHLKIGNFVIKETIGKGAFGAVKRGTHIQTGYDVAIKILNR
GRMKGLGTVNKTRNEIDNLQKLTHPHITRLFRVISTPSDIFLVMELVSGGELFSYIT
RKGALPIRESRRYFQQIISGVSYCHNHMIVHRDLKPENLLLDANKNIKIADFGLSNY
MTDGDLLSTACGSPNYAAPELISNKLYVGPEVDLWSCGVILYAMLCGTLPFDDQNVP
TLFAKIKSGRYTVPYSMEKQAADLISTMLQVDPVKRADVKRIVNHSWFRIDLPYYLF
PECENESSIVDIDVVQSVAEKTPPEKIIYFFIFRHFLKFDVKEEDVTGALLAEDHHH
FLCIAYRLEVNHKRNADESSQKAMEDFWEIGKTMKMGSTSLPVGATTKSEKSERNVA
KVVGKFSANVGRKILEGLKKEQKKLTWNLGIRACLDPVETMKHVFLSLKSVDMEWKV
LSMYHIIVRSKPTPINPDPVKVSLQLFALDKKENNKGYLLDFKGLTEDEEAVPPSRC
RSRAASVSVTLAKSKSDLNGNSSKVPMSPLSPMSPISPSVNIPKVRVDDADASLKSS
LNSSIYMADIENSMESLDEVSTQSSEPEAPIRSQTMEFFATCHIIMQALLAE (SEQ
ID NO:43)


>gi|24636001|gb|AAF60550.2| protein Y41G9A.3
[Caenorhabditis elegans]
MNNTTGRLRRNKATTFESPSIPKAFFDLQHHFIFSKRKAPVDGIEAIQRDGASISEH
AVVKYANDERPDEKEKQDLENMFKTVLRIGVRNNRVAKNIPSTIPENSDIVYTHLLQ
LSQCYEAMARNNKLIVFTNDISVRKAFNGLIYNCMRTGLVADSQTLEITGVLSVTDF
IMVLMMLWKYRENLDELKGTPLSHEDFRQMDIAYMPISRWKGCLETKGQLKPFINIG
LKESIFRAVELLTKYRIHRLPVMDEKTGDCAYILTHRRILHYIWKHCALLPKPECLS
QRVVDLEIGSWKNLIFANEQTPLIECLDMLIDNNISGIPIVQKNTLKVLEVYTRFDA
ASAAFSDHIDLSVSVTRAIQERDYQNGIRRDGVVTANYTTTLWSLIEIFIDKNVHRI
FMVDDRTILKGIISLSDVIEF
LVLRPTKKNGVTTGEPMEK (SEQ ID NO:44)


>gi|21629470|gb|AAM69096.1|U58726_2 protein T01C8.1b
[Caenorhabditis elegans]
MFSHQDRDRDRKEDGGGDGTEMKSKSRSQPSGLNRVKNLSRKLSAKSRKERKDRDST
DNSSKMSSPGGETSTKQQQELKAQIKIGHYILKETLGVGTFGKVKVGIHETTQYKVA
VKILNRQKIKSLDVVGKIRREIQNLSLFRHPHIIRLYQVISTPSDIFMIMEHVSGGE
LFDYIVKHGRLKTAEARRFFQQIISGVDYCHRHMVVHRDLKPENLLLDEQNNVKIAD
FGLSNIMTDGDFLRTSCGSPNYAAPEVISGKLYAGPEVDVWSCGVILYALLCGTLPF
DDEHVPSLFRKIKSGVFPTPDFLERPIVNLLHHMLCVDPMKRATIKDVIAHEWFQKD
LPNYLFPPINESEASIVDIEAVREVTEFQRYHVAEEEVTSALLGDDPHHHLSIAYNL


IVDNKRIADETAKLSIEEFYQVTPNKGPGPVHRHPERIAASVSSKITPTLDNTEASG
ANRNKRAKWHLGIRSQSRPEDIMFEVFRAMKQLDMEWKVLNPYHVIVRRKPDAPAAD
PPKMSLQLYQVDQRSYLLDFKSLADEESGSASASSSRHASMSMPQKPAGIRGTRTSS
MPQAMSMEASIEKMEVHDFSDMSCDVTPPPSPGGAKLSQTMQFFEICAALIGTLAR
(SEQ ID NO:45)

>gi|21629469|gb|AAM69095.1|U58726_1 protein T01C8.1a
[Caenorhabditis elegans]
MFSHQDRDRDRKEDGGGDGTEMKSKSRSQPSGLNRVKNLSRKLSAKSRKERKDRDST
DNSSKMSSPGGETSTKQQQELKAQIKIGHYILKETLGVGTFGKVKVGIHETTQYKVA
VKILNRQKIKSLDVVGKIRREIQNLSLFRHPHIIRLYQVISTPSDIFMIMEHVSGGE
LFDYIVKHGRLKTAEARRFFQQIISGVDYCHRHMVVHRDLKPENLLLDEQNNVKIAD
FGLSNIMTDGDFLRTSCGSPNYAAPEVISGKLYAGPEVDVWSCGVILYALLCGTLPF
DDEHVPSLFRKIKSGVFPTPDFLERPIVNLLHHMLCVDPMKRATIKDVIAHEWFQKD
LPNYLFPPINESEASIVDIEAVREVTERYHVAEEEVTSALLGDDPHHHLSIAYNLIV
DNKRIADETAKLSIEEFYQVTPNKGPGPVHRHPERIAASVSSKITPTLDNTEASGAN
RNKRAKWHLGIRSQSRPEDIMFEVFRAMKQLDMEWKVLNPYHVIVRRKPDAPAADPP
KMSLQLYQVDQRSYLLDFKSLADEESGSASASSSRHASMSMPQKPAGIRGTRTSSMP
QAMSMEASIEKMEVHDFSDMSCDVTPPPSPGGAKLSQTMQFFEICAALIGTLAR
(SEQ ID NO:46)


>gi|13072981|gb|BG277558.1|BG277558 ux45f03.y1
Soares_NMMAX_maxillary_process Mus musculus cDNA clone
IMAGE:3513341 5' similar to TR:Q9R078 Q9R078 5'-AMP-
ACTIVATED PROTEIN KINASE BETA SUBUNIT ;, mRNA sequence
CATCAGCATTGAGCTCGCCGCGCTCGTGCGGCAGGTGGACCACAAGACGCCGGGGAT
GGACTGTTCCGGGGGCGCCATGGATGGGGACAGGCCCAAGATCCTCATGGACAGCTC
TGAACACGTCGACATCTTCCACTCCGAATACATCCTTGCTCTGAAGAAAGAGGAATT
CCTGTCCTGACTGCATGACCTGTAAGCGAATGATAAAGCTTCCTGCCAGGCCCGGCC
CACCGTGTATCGATGGACAGGGGGTGGAAAGGAAGTCTACTTGTCTGGGTCCTTCAA
CAACTGGAGCAACCTTACCCTCACGATAAGCCATAATAAGTTTGTAGTCATGCTGTA
CCTGCCTGAATGAGAGCATCGATCCAAGTTCTGCGTGCATGGAGAGTGTACCCATGA
TCCTTGTGATAGC (SEQ ID NO:47)


>gi|12649678|gb|BG146270.1|BG146270 mab73f04.y1
NCI_CGAP_BC3 Mus musculus cDNA clone IMAGE:3976063 5'
similar to TR:Q9R078 Q9R078 5'-AMP-ACTIVATED PROTEIN
KINASE BETA SUBUNIT ;, mRNA sequence
GTTCCAGTCGCGGTCGCCCGAGCCAGATTCCCAGCCATGGGCAACACGAGCAGCGAG
CGCGCCGCGCTGGAGCGGCAGGCGGGCCACAAGACGCCGCGGAGGGACAGCTCCGGG
GGCGCCAAGGATGGGGACAGGCCCAAGATCCTCATGGACAGCCCTGAAGACGCCGAC
ATCTTCCACTCCGAAGAGATCAAGGCTCCAGAGAAAGAGGAATTCCTGGCCTGGCAG
CACGACCTGGAAGCGAATGATAAAGCCCCCGCCCAGGCCCGGCCCACCGTGTTTCGA
TGGACAGGGGGTGGAAAGGAAGTCTACTTGTCTGGGTCCTTCAACAACTGGAGCAAG
CTTCCCCTCACGAGA (SEQ ID NO:48)

>gi|11134070|gb|BF226344.1|BF226344 uz48h07.y1
NCI_CGAP_Mam6 Mus musculus cDNA clone IMAGE:3672349 5'
similar to TR:Q9R078 Q9R078 5'-AMP-ACTIVATED PROTEIN
KINASE BETA SUBUNIT ;, mRNA sequence
ACGCGTCCGGCCATGGGCAACACGAGCAGCGAGCGCGCCGCGCTGGAGCGGCAGGCG
GGCCACAAGACGCCGCGGAGGGACAGCTCCGGGGGCGCCAAGGATGGGGACAGGCCC
AAGATCCTCATGGACAGCCCTGAAGACGCCGACATCTTCCACTCCGAAGAGATCAAG
GCTCCAGAGAAAGAGGAATTCCTGGCCTGGCAGCACGACCTGGAAGCGAATGATAAA
GCCCCCGCCCAGGCCCGGCCCACCGTGTTTCGATGGACAGGGGGTGGAAAGGAAGTC
TACTTGTCTGGGTCCTTCAACAACTGGAGCAAGCTTCCCCTCACGAGAAGCCAGAAT
AACTTTGTAGCCATCCTGGACCTGCCTGAAGGAGAGCATCAGTACAAGTTCTTCGTG
GATGGACAGTGGACCCATGATCCTTTTGAGCCAATAGTAACC  (SEQ ID NO:49)

>gi|8215681|gb|AF214519.1|AF214519 Homo sapiens AMP-
activated protein kinase gamma subunit (PRKAG3) mRNA,
complete cds
ATGAGCTTCCTAGAGCAAGAAAACAGCAGCTCATGGCCATCACCAGCTGTGACCAGC
AGCTCAGAAAGAATCCGTGGGAAACGGAGGGCCAAAGCCTTGAGATGGACAAGGCAG
AAGTCGGTGGAGGAAGGGGAGCCACCAGGTCAGGGGGAAGGTCCCCGGTCCAGGCCA
ACTGCTGAGTCCACCGGGCTGGAGGCCACATTCCCCAAGACCACACCCTTGGCTCAA
GCTGATCCTGCCGGGGTGGGCACTCCACCAACAGGGTGGGACTGCCTCCCCTCTGAC
TGTACAGCCTCAGCTGCAGGCTCCAGCACAGATGATGTGGAGCTGGCCACGGAGTTC
CCAGCCACAGAGGCCTGGGAGTGTGAGCTAGAAGGCCTGCTGGAAGAGAGGCCTGCC
CTGTGCCTGTCCCCGCAGGCCCCATTTCCCAAGCTGGGCTGGGATGACGAACTGCGG
AAACCCGGCGCCCAGATCTACATGCGCTTCATGCAGGAGCACACCTGCTACGATGCC
ATGGCAACTAGCTCCAAGCTAGTCATCTTCGACACCATGCTGGAGATCAAGAAGGCC
TTCTTTGCTCTGGTGGCCAACGGTGTGCGGGCAGCCCCTCTATGGGACAGCAAGAAG
CAGAGCTTTGTGGGGATGCTGACCATCACTGACTTCATCCTGGTGCTGCATCGCTAC
TACAGGTCCCCCCTGGTCCAGATCTATGAGATTGAACAACATAAGATTGAGACCTGG
AGGGAGATCTACCTGCAAGGCTGCTTCAAGCCTCTGGTCTCCATCTCTCCTAATGAT
AGCCTGTTTGAAGCTGTCTACACCCTCATCAAGAACCGGATCCATCGCCTGCCTGTT
CTTGACCCGGTGTCAGGCAACGTACTCCACATCCTCACACACAAACGCCTGCTCAAG
TTCCTGCACATCTTTGGTTCCCTGCTGCCCCGGCCCTCCTTCCTCTACCGCACTATC
CAAGATTTGGGCATCGGCACATTCCGAGACTTGGCTGTGGTGCTGGAGACAGCACCC
ATCCTGACTGCACTGGACATCTTTGTGGACCGGCGTGTGTCTGCACTGCCTGTGGTC
AACGAATGTGGTCAGGTCGTGGGCCTCTATTCCCGCTTTGATGTGATTCACCTGGCT
GCCCAGCAAACCTACAACCACCTGGACATGAGTGTGGGAGAAGCCCTGAGGCAGAGG
ACACTATGTCTGGAGGGAGTCCTTTCCTGCCAGCCCCACGAGAGCTTGGGGGAAGTG
ATCGACAGGATTGCTCGGGAGCAGGTACACAGGCTGGTGCTAGTGGACGAGACCCAG
CATCTCTTGGGCGTGGTCTCCCTCTCCGACATCCTTCAGGCACTGGTGCTCAGCCCT
GCTGGCATCGATGCCTCGGGGCCTGAGAAGATCTGAGTCCTCAATCCCAAGCCAAC
TGCACACTGGAAGCCAATGAAGGAATTGAGAACAGCTTCATTTCCCCAACCCCAATT
TGCTGGTTCAGCTATGATTCAGGCTTCTTCAGCCTTCCAAAATTGCCTTTGCCTTAC
TTGTGCTCCCAGAACCCTTCGGGCATGCCCAGTGCACCATGGGATGATGAAATTAAG
GAGAACAGCTGAGTCAAGCTTGGAGGTCCCTGAACCAGAGGCACTAGGATTACCCCA
GGGCCATCTGTGCTCCATGCCCGCCCATCCCCTTGCCGCCTGACTGGGTCGGATGGC
CCCAGTGGGTTTAGTCAGGGCTTCTGGATTCCTCGGTTTCTGGGCTACCTATGGCTT
CAGCCTTCAGCTCCTGGGAGTCCCAGCTGTTGTTCCCAGCAACGTCGCCACTGCCCT

CCTACTCTCCAGGCTTTGTCATTTCAAGGCTGCTGAAATGCTGCATTTCAGGGGCCA
CCATGGAGCAGCCGTTATTTATAGAACTGCCTGTTGGAGGTGGGGAGTCCTCCCTCC
ATTCTTGTCCAGAAAACTCCTTAGCTCTCGCAGTGAGCCATGTTCTTAGTCTCCAGG
GATGGATGGCCTTGTATATGGACCCCTGAGAATGAGCAATTGAGAAAACAAAACAAA
AGGAACAATCCATGAACTTAGATTTTATTGGTTTCACTCAAAATGCTGCAGTCATTT
GACCTG (SEQ ID NO:50)

EP 1 576 180 B1

```
>gi|6688198|emb|AJ249976.1|HSA249976 Homo sapiens mRNA
for AMP-activated protein kinase gamma2 subunit (AMPK
gamma2 gene)
GCGGCTCCAGTCCGAAGGCAGCGGCCGGGGGAGGGAAGGAGGGGACCGAACCCCCGA
GGAGTTTCGCAGAATCAACTTCTGGTTAGAGTTATGGGAAGCGCGGTTATGGACACC
AAGAAGAAAAAAGATGTTTCCAGCCCCGGCGGGAGCGGCGGCAAGAAAAATGCCAGC
CAGAAGAGGCGTTCGCTGCGCGTGCACATTCCGGACCTGAGCTCCTTCGCCATGCCG
CTCCTGGACGGAGACCTGGAGGGTTCCGGAAAGCATTCCTCTCGAAAGGTGGACAGC
CCCTTCGGCCCGGGCAGCCCCTCCAAAGGGTTCTTCTCCAGAGGCCCCCAGCCCCGG
CCCTCCAGCCCCATGTCTGCACCTGTGAGGCCCAAGACCAGCCCCGGCTCTCCCAAA
ACCGTGTTCCCGTTCTCCTACCAGGAGTCCCCGCCACGCTCCCTCGACGCATGAGC
TTCAGTGGGATCTTCCGCTCCTCCTCCAAAGAGTCTTCCCCCAACTCCAACCCTGCT
ACCTCGCCCGGGGGCATCAGGTTTTTCTCCCGCTCCAGAAAAACCTCCGGCCTCTCC
TCCTCTCCGTCAACACCCACCCAAGTGACCAAGCAGCACACGTTTCCCCTGGAATCC
TATAAGCACGAGCCTGAACGGTTAGAGAATCGCATCTATGCCTCGTCTTCCCCCCCG
GACACAGGGCAGAGGTTCTGCCCGTCTTCCTTCCAGAGCCCGACCAGGCCTCCACTG
GCATCACCGACACACTATGCTCCCTCCAAAGCCGCGGCGCTGGCGGCGGCCCTGGGA
CCCGCGGAAGCCGGCATGCTGGAGAAGCTGGAGTTCGAGGACGAAGCAGTAGAAGAC
TCAGAAAGTGGTGTTTACATGCGATTCATGAGGTCACACAAGTGTTATGACATCGTT
CCAACCAGTTCAAAGCTTGTTGTCTTTGATACTACATTACAAGTTAAAAAGGCCTTC
TTTGCTTTGGTAGCCAACGGTGTCCGAGCAGCGCCACTGTGGGAGAGTAAAAAACAA
AGTTTTGTAGGAATGCTAACAATTACAGATTTCATAAATATACTACATAGATACTAT
AAATCACCTATGGTACAGATTTATGAATTAGAGGAACATAAAATTGAAACATGGAGG
GAGCTTTATTTACAAGAAACATTTAAGCCTTTAGTGAATATATCTCCAGATGCAAGC
CTCTTCGATGCTGTATACTCCTTGATCAAAAATAAAATCCACAGATTGCCCGTTATT
GACCCTATCAGTGGGAATGCACTTTATATACTTACCCACAAAAGAATCCTCAAGTTC
CTCCAGCTTTTTATGTCTGATATGCCAAAGCCTGCTTTCATGAAGCAGAACCTGGAT
GAGCTTGGAATAGGAACGTACCACAACATTGCCTTCATACATCCAGACACTCCCATC
ATCAAAGCCTTGAACATATTTGTGGAAAGACGAATATCAGCTCTGCCTGTTGTGGAT
GAGTCAGGAAAAGTTGTAGATATTTATTCCAAATTTGATGTAATTAATCTTGCTGCT
GAGAAAACATACAATAACCTAGATATCACGGTGACCCAGGCCCTTCAGCACCGTTCA
CAGTATTTTGAAGGTGTTGTGAAGTGCAATAAGCTGGAAATACTGGAGACCATCGTG
GACAGAATAGTAAGAGCTGAGGTCCATCGGTTGGTGGTGGTAAATGAAGCAGATAGT
ATTGTGGGTATTATTTCCCTGTCGGACATTCTGCAAGCCCTGATCCTCACACCAGCA
GGTGCCAAACAAAAGGAGACAGAAACGGAGTGACCGCCGTGAATGTAGACGCCCTAG
GAGGAGAACTTGAACAAAGTCTCTGGGTCACGTTTTGCCTCATGAACACTGGCTGCA
AGTGGTTAAGAATGTATATCAGGGTTTAACGATAGGTATTTCTTCCAGTGATGTTGA
AATTAAGCTTAAAAAAGAAAGATTTTATGTGCTTGAAAATTCAGGCTTGCATTAAAA
AACTGTTTTCAGACCTTGTCTGAAGGATTTTAAATGCTGTATGTCATTAAAGTGCAC
TGTGTCCCTG (SEQ ID NO:51)
```

>gi|4115828|dbj|AB022017.1| Homo sapiens mRNA for AMP-
activated protein kinase alpha-1, complete cds
GCAGACTCAGTTCCTGGAGAAAGATGGCGACAGCCGAGAAGCAGAAACACGACGGGC
GGGTGAAGATCGGCCACTACATTCTGGGTGACACGCTGGGGGTCGGCACCTTCGGCA
AAGTGAAGGTTGGCAAACATGAATTGACTGGGCATAAAGTAGCTGTGAAGATACTCA
ATCGACAGAAGATTCGGAGCCTTGATGTGGTAGGAAAAATCCGCAGAGAAATTCAGA
ACCTCAAGCTTTTCAGGCATCCTCATATAATTAAACTGTACCAGGTCATCAGTACAC
CATCTGATATTTTCATGGTGATGGAATATGTCTCAGGAGGAGAGCTATTTGATTATA
TCTGTAAGAATGGAAGGCTGGATGAAAAAGAAAGTCGGCGTCTGTTCCAACAGATCC
TTTCTGGTGTGGATTATTGTCACAGGCATATGGTGGTCCATAGAGATTTGAAACCTG
AAAATGTCCTGCTTGATGCACACATGAATGCAAAGATAGCTGATTTTGGTCTTTCAA
ACATGATGTCAGATGGTGAATTTTTAAGAACAAGTTGTGGCTCACCCAACTATGCTG
CACCAGAAGTAATTTCAGGAAGATTGTATGCAGGCCCAGAGGTAGATATATGGAGCA
GTGGGGTTATTCTCTATGCTTTATTATGTGGAACCCTTCCATTTGATGATGACCATG
TGCCAACTCTTTTTAAGAAGATATGTGATGGATCTTCTATACCCCTCAATATTTAA
ATCCTTCTGTGATTAGCCTTTTGAAACATATGCTGCAGGTGGATCCCATGAAGAGGG
CCTCAATCAAAGATATCAGGGAACATGAATGGTTTAAACAGGACCTTCCAAAATATC
TCTTTCCTGAGGATCCATCATATAGTTCAACCATGATTGATGATGAAGCCTTAAAAG
AAGTATGTGAAAAGTTTGAGTGCTCAGAAGAGGAAGTTCTCAGCTGTCTTTACAACA
GAAATCACCAGGATCCTTTGGCAGTTGCCTACCATCTCATAATAGATAACAGGAGAA
TAATGAATGAAGCCAAAGATTTCTATTTGGCGACAAGCCCACCTGATTCTTTTCTTG
ATGATCATCACCTGACTCGGCCCCATCCTGAAAGAGTACCATTCTTGGTTGCTGAAA
CACCAAGGGCACGCCATACCCTTGATGAATTAAATCCACAGAAATCCAAACACCAAG
GTGTAAGGAAAGCAAAATGGCATTTAGGAATTAGAAGTCAAAGTCGACCAAATGATA
TTATGGCAGAAGTATGTAGAGCAATCAAACAATTGGATTATGAATGGAAGGTTGTAA
ACCCATATTATTTGCGTGTACGAAGGAAGAATCCTGTGACAAGCACTTACTCCAAAA
TGAGTCTACAGTTATACCAAGTGGATAGTAGAACTTATCTACTGGATTTCCGTAGTA
TTGATGATGAAATTACAGAAGCCAAATCAGGGACTGCTACTCCACAGAGATCGGGAT
CAGTTAGCAACTATCGATCTTGCCAAAGGAGTGATTCAGATGCTGAGGCTCAAGGAA
AATCCTCAGAAGTTTCTCTTACCTCATCTGTGACCTCACTTGACTCTTCTCCTGTTG
ACCTAACTCCAAGACCTGGAAGTCACACAATAGAATTTTTTGAGATGTGTGCAAATC
TAATTAAAATTCTTGCACAATAAACAGAAAACTTTGCTTATTTCTTTTGCAGCAATA
AGCATGCATAATAAGTCACAGCCAAATGCTTCCATTTGTAATCAAGTTATACATAAT
TATAACCGAGGGCTGGCGTTTTGGAATCGAATTCGACAGGGATTGGAACATGATTT
ATAGTTAAAAGCCTAATATCGAGAAATGAATTAAGATCA  (SEQ ID NO:52)

>gi|1335855|gb|U42412.1|HSU42412 Human 5'-AMP-activated
protein kinase, gamma-1 subunit mRNA, complete cds
GCGCCCTTAAAGATGGTGAGGGGGCTCATGCTCTGAGTAGAAGGTGGTGACCTCCAG
GAGCGGTGGGATGATGAGGGCCCGGGCGCCTCTTGCAATGGAGACGGTCATTTCTTC
AGATAGCTCCCCAGCTGTGGAAAATGAGCATCCTCAAGAGACCCCAGAATCCAACAA
TAGCGTGTATACTTCCTTCATGAAGTCTCATCGCTGCTATGACCTGATTCCCACAAG
CTCCAAATTGGTTGTATTTGATACGTCCCTGCAGGTGAAGAAAGCTTTTTTTGCTTT
GGTGACTAACGGTGTACGAGCTGCCCCTTTATGGGATAGTAAGAAGCAAAGTTTTGT
GGGCATGCTGACCATCACTGATTTCATCAATATCCTGCACCGCTACTATAAATCAGC
CTTGGTACAGATCTATGAGCTAGAAGAACACAAGATAGAAACTTGGAGAGAGGTGTA
TCTCCAGGACTCCTTTAAACCGCTTGTCTGCATTTCTCCTAATGCCAGCTTGTTTGA
TGCTGTCTCTTCATTAATTCGGAACAAGATCCACAGGCTGCCAGTTATTGACCCAGA

ATCAGGCAATACTTTGTACATCCTCACCCACAAGCGCATTCTGAAGTTCCTCAAATT
GTTTATCACTGAGTTCCCCAAGCCAGAGTTCATGTCCAAGTCTCTGGAAGAGCTACA
GATTGGCACCTATGCCAATATTGCTATGGTTCGCACTACCACCCCCGTCTATGTGGC
TCTGGGGATTTTTGTACAGCATCGAGTCTCAGCCCTGCCAGTGGTGGATGAGAAGGG
GCGTGTGGTGGACATCTACTCCAAGTTTGATGTTATCAATCTGGCAGCAGAAAAGAC
CTACAACAACCTAGATGTATCTGTGACTAAAGCCTTGCAACATCGATCACATTACTT
TGAGGGTGTTCTCAAGTGCTACCTGCATGAGACTCTGGAGACCATCATCAACAGGCT
AGTGGAAGCAGAGGTTCACCGACTTGTAGTGGTGGATGAAAATGATGTGGTCAAGGG
AATTGTATCACTGTCTGACATCCTGCAGGCCCTGGTGCTCACAGGTGGAGAGAAGAA
GCCCTGAGCTGGGGGAAGGGGTCATGCAGCACCAGGGGATATGCCCAACTCACTGCC
TGCTGGAAGCTCTGTGGGAATCAGATGAAACTTGAGGGAATTGTGACTCTGTTCCCT
GTTCAGGGTCCCCTGCCCTTCTATCTGGGAGCTAGGGAAGGTATGGGGGAGGAAAGA
GAATGGATTTATAGCTACCCTTACCCTCACACATACACTTGAAAAAACTTTCAGCCT
AGCCAGTTCTAGCCCCTGTCCTCTTAGATATATCCCCCTTTCTGGGTGAACTATAGG
CTCTGTGCCTCTCAGACAAATTCTGATCTCTAAGAGATCCCCAGACCTCACTTGCCT
CTGCCTCCATCTTGGCCCTGATTCAACCCTAAGATAATAGCACAACAAAATTCTTCA
TAAAGATATTTTTATTCACCTGTTCCGTGCTATATGGAGGAGGCCAAGTCCATTTAG
TGACATTTCTTCCCATAATGTGAGTGGGGAGGATTGTGG (SEQ ID NO:53)

>gi|2916801|emb|AJ224538.1|HSA224538 Homo sapiens mRNA
for AMP-activated protein kinase beta 2 subunit
ATGGGAAACACCACCAGCGACCGGGTGTCCGGGGAGCGCCACGGCGCCAAGGCTGCA
CGCTCCGAGGGCGCAGGCGGCCATGCCCCGGGGAAGGAGCACAAGATCATGGTGGGG
AGTACGGACGACCCCAGCGTGTTCAGCCTCCCTGACTCCAAGCTCCCTGGGGACAAA
GAGTTTGTATCATGGCAGCAGGATTTGGAGGACTCCGTAAAGCCCACACAGCAGGCC
CGGCCCACTGTTATCCGCTGGTCTGAAGGAGGCAAGGAGGTCTTCATCTCTGGGTCT
TTCAACAATTGGAGCACCAAGATTCCACTGATTAAGAGCCATAATGACTTTGTTGCC
ATCCTGGACCTCCCTGAGGGAGAGCACCAATACAAGTTCTTTGTGGATGGACAGTGG
GTTCATGATCCATCAGAGCCTGTGGTTACCAGTCAGCTTGGCACAATTAACAATTTG
ATCCATGTCAAGAAATCTGATTTTGAGGTGTTCGATGCTTTAAAGTTAGATTCTATG
GAAAGTTCTGAGACATCTTGTAGAGACCTTTCCAGCTCACCCCCAGGGCCTTATGGT
CAAGAAATGTATGCGTTTCGATCTGAGGAAAGATTCAAATCCCCACCCATCCTTCCT
CCTCATCTACTTCAAGTTATTCTTAACAAAGACACTAATATTTCTTGTGACCCAGCC
TTACTCCCTGAGCCCAACCATGTTATGCTGAACCATCTCTATGCATTGTCCATTAAG
GACAGTGTGATGGTCCTTAGCGCAACCCATCGCTACAAGAAGAAGTATGTTACTACT
CTGCTATACAAGCCCATTTGA (SEQ ID NO:54)


>gi|2916799|emb|AJ224515.1|HSA224515 Homo sapiens mRNA
for AMP-activated protein kinase beta 1
ATGGGCAATACCAGCAGTGAGAGGGCGGCGCTGGAGCGGCATGCTGGCCATAAGACG
CCCCGGAGGGACAGCTCGGGGGGCACCAAGGACGGGGACAGGCCCAAGATCCTGATG
GACAGCCCCGAAGACGCCGACCTCTTCCACTCCGAGGAAATCAAGGCACCAGAGAAG
GAGGAATTCCTGGCCTGGCAGCATGATCTGGAAGTGAATGATAAAGCTCCCGCCCAG
GCTCGGCCAACGGTGTTTCGATGGACGGGGGGCGGAAAGGAAGTTTACTTATCTGGG
TCCTTCAACAACTGGAGTAAACTTCCCCTCACCAGAAGCCACAATAACTTTGTAGCC
ATCCTGGATCTGCCGGAAGGAGAGCATCAGTACAAGTTCTTTGTGGATGGTCAGTGG
ACGCACGACCCTTCCGAGCCCATAGTAACCAGCCAGCTTGGCACAGTTAACAACATA
ATTCAAGTGAAGAAAACTGACTTTGAGGTATTTGATGCTTTAATGGTGGATTCCCAA


AAGTGCTCCGATGTGTCTGAGCTGTCCAGTTCTCCCCCAGGACCCTACCATCAGGAG
CCCTACGTCTGCAAACCCGAAGAGCGCTTTCGGGCACCCCCTATTCTCCCCCCACAT
CTCCTCCAGGTCATCCTGAACAAGGACACGGGGATTTCCTGTGATCCAGCTTTGCTT
CCTGAGCCCAATCACGTCATGCTGAACCACCTATACGCGCTGTCTATCAAGGATGGA
GTGATGGTGCTCAGCGCAACCCACCGGTACAAGAAGAAGTACGTCACCACCTTGTTA
TACAAGCCCATATGA (SEQ ID NO:55)

```
>gi|3043597|dbj|AB011109.1| Homo sapiens mRNA for
KIAA0537 protein, complete cds
AATCTCCCAAGGCCTAAGGAGGCAAGAGGCCTGCAAATCGCCTCCTGCTCAGCAAAC
GGGTTGCTCAGCAGGCCCGGGGTCCTGGTCCACCCCAGGTCCCTGGTTTGCCCACCT
CCGATGGCGGCCTTCGCTGGCAGGGTGGGCGCCTCTGGGGAGCCAGCTCCGTCCCGG
CGCCTTTAGAGCCCCATCTCTTCCACGTCCCTGGCCTTCCTCCCCTTCCAGGCGGCT
GTCCCCGCCGGGGTCCAGATGGTGTCGGAGGGCCGGCGGTTCGACGGCGGGCCCGGG
GTTCAGCCTCCCGGCCTCCCTCCGTCCCTGACTCTCCTTTCTTCGGAGAGGGCGCGG
GGGCCGGGGCCAAAGCGCCGCTCTTGGGGTTCTCCTGGACTCGGAGTTGCCCCAGGC
GGGCGCAGCTCTGCCCCGCGGGGTGCCAGCCTCGGGCGGGCAAGGTCCGTGAGTCAC
CGCCTGTAACCGAACACCAGGCCTCCCTGCCCCCTCCCCAGCTCCGGCCGCCAGGC
TGCGGCGACACCTACAAGAAAATGAAGGGGCGCCCAGGCCCGCGGCGGCCCCGGCCG
TATCGCGAGCAGGTCCCGGCGGCCCCCGGCTCGCGGCGCTCTTTCTTCCCCGGCCCC
GGGGCTCGGCCAGCCGCAACCGCCGCCCCGGCGCCAGCAGGAATCCAGGCCGAGCGA
CCGGCCCCGGAGCCCGAGGCGGCGGAGGGCCCGCGGTAGCTGCGACTGGCGAGCCCG
AGAGCGCCCGGGGAGGGGGCGCCCGGCTTGGAATTTCCCGGTCCCTTCCGGCCCAGC
GAGGACAAAGCACTCCTGGCCGCCGCCGCCGCCGCCGCCGTGGCCTACGCCGCGCCG
CACAAAGGGCGAGTCGCGACACGCTCCCATCCCCCTCCCAGCTCACGGCGGCCCCGG
CCCCGGGTGGCTGCAGGGAGGTGGGGGAAGCCCTGGCTGCACCGCCCCTCGCTCCCC
CTCCCCTGGGGCCGCGCGAGCGCCGCCCCGCCCCGTCTGCGCGTCCTCCCGGGGAG
GGGTTGGGGGGCGCGGCGCCCCACATAACACTCCCCCTCCTGCGCTGCGAGCCACCC
TCTCCCCTCCCTCCTGCAAACACCACCGCCTCCCCTGCCACCGCCGCCACCTCGCCC
GACGCTCCACAGCTCGCCGCGGCCGGGGGGCGGTGCGCGGACCGTGCGCGCCGCGGG
CGCCAGATGTGCAGTCCCCGCCGCCGCCAGTGACCGAGCCGCAGTCCGAGCGGTATC
GGGCCGCCTCCCTGATGCTGCGGGGGCGACCTTGAGCGTACAGCGGCTTCCCTCGGT
GGGGACCCCGACATCCCAGCGCTGTGCCCGGTCTTGCCCTCTGTAGCCCGGCTCGCC
CCGCGCTTGGACATGGAAGGGGCCGCCGCGCCTGTGGCGGGGGACCGCCCCGACTTG
GGGCTGGGGGCGCCGGGCTCTCCCCGAGAGGCGGTGGCGGGGGCGACTGCAGCCCTG
GAGCCCAGGAAGCCGCACGGGGTGAAGCGGCATCACCACAAGCACAACTTGAAGCAC
CGCTACGAGCTGCAGGAGACCCTGGGCAAAGGCACCTACGGCAAAGTCAAGCGGGCC
ACCGAGAGGTTTTCTGGCCGAGTGGTTGCTATAAAATCCATTCGTAAGGACAAAATT
AAGGATGAACAAGACATGGTTCACATCAGACGAGAGATTGAGATCATGTCATCTCTC
AACCATCCTCATATCATCAGTATTTATGAAGTGTTTGAGAACAAAGATAAGATTGTG
ATCATCATGGAATATGCCAGCAAAGGGGAGCTGTACGATTACATCAGTGAGCGGCGA
CGCCTCAGTGAGAGGGAGACCCGGCACTTCTTCCGGCAGATCGTCTCTGCTGTGCAC
TATTGTCACAAGAACGGTGTGGTCCACCGGGACTTGAAGCTGGAAAATATACTGCTC
GATGACAACTGCAATATTAAGATTGCTGACTTTGGGCTTTCCAACCTGTACCAGAAG
GATAAGTTCTTACAAACGTTTTGTGGGAGTCCACTCTATGCATCTCCTGAGATTGTC
AATGGGAGACCTTACCGAGGGCCAGAGGTGGACAGCTGGGCCCTGGGTGTGTTGCTT
TACACTCTTGTTTATGGAACAATGCCCTTCGATGGTTTCGATCACAAAAACCTCATT
CGGCAAATCAGCAGCGGAGAGTACCGGGAGCCAACACAGCCCTCAGATGCTCGAGGA
```

CTCATACGGTGGATGCTGATGGTGAACCCCGATCGCCGGGCCACTATTGAGGACATT
GCCAACCACTGGTGGGTGAACTGGGGCTATAAGAGCAGCGTGTGTGACTGTGATGCC
CTCCATGACTCTGAGTCCCCACTCCTGGCTCGGATCATTGACTGGCACCACCGTTCC
ACAGGGCTGCAGGCTGACACCGAAGCCAAAATGAAGGGCCTGGCCAAACCCACGACC
TCTGAGGTCATGCTAGAGCGGCAGCGGTCGCTGAAGAAATCCAAGAAAGAGAATGAC
TTTGCTCAGTCTGGTCAGGATGCAGTGCCTGAAAGCCCATCCAAGTTGAGTTCTAAG
AGGCCCAAGGGGATCCTGAAGAAGCGAAGCAACAGCGAGCATCGCTCTCACAGCACT
GGCTTCATTGAAGGTGTAGTTGGTCCTGCCTTACCCTCTACTTTCAAGATGGAGCAG
GACTTGTGCAGGACTGGCGTGCTCCTCCCAAGCTCACCAGAGGCAGAGGTGCCGGGA
AAACTCAGCCCCAAGCAGTCGGCCACGATGCCCAAGAAAGGCATCTTGAAAAAGACC
CAGCAGAGAGAATCAGGTTACTACTCTTCCCCAGAGCGCAGTGAGTCTTCGGAGCTG
TTGGACAGTAATGATGTGATGGGCAGCAGCATCCCCTCCCCCAGCCCCCCGGACCCA
GCCAGGGTAACCTCCCACAGCCTCTCCTGCCGGAGGAAGGGCATCTTGAAACACAGC
AGCAAATACTCAGCGGGCACCATGGACCCAGCCCTGGTCAGCCCTGAAATGCCCACA
CTGGAATCCCTGTCAGAGCCTGGTGTCCCTGCCGAGGGCCTCTCCCGGAGCTACAGC
CGCCCTTCCAGTGTCATCAGCGATGACAGCGTGCTGTCCAGCGACTCTTTTGACTTG
CTGGATTTGCAGGAGAATCGCCCTGCCCGCCAGCGCATCCGCAGCTGCGTCTCTGCA
GAAAACTTCCTCCAGATCCAGGACTTTGAGGGGCTCCAGAACCGGCCCCGGCCCCAG
TACCTGAAGCGGTACCGGAACCGGCTGGCAGACAGCAGCTTCTCCCTCCTCACAGAC
ATGGATGATGTGACTCAGGTCTACAAGCAAGCGCTGGAGATCTGCAGCAAGCTCAAC
TAGCATTCCAGGGCGCCCAGGGGCGGGCGGGGGTACGAGGGAGGAAGGGGAGCAAGA
CTTGGGCTCACAGGCTGGTTACCTCTTTGCTGGCTGTGACAACAGACTGAAAAAGGA
TTGGCACTGTCTCACTTGGCCAAGTTTGCAGCCTTGAGCCAACACCTAAAAGGGAGA
GGTGGGCTCTTCTGCCAGTTCTGTCAATTGTCAGTCAGAATTTGGGCCCTGTTTGGC
ATTTGCTTTATGGCACCTCCTAGAGGACCAGCTGTCCAGGGGAGGTGGTATTGACCG
GCACTCAGTGGGTGGAGAGGAAGCATATGTGGAAGGAGCATTTCCTTAGAAATGCTT
CATTCATCCAGATGCTTCTGGAGGAGGGGCAGGAGACACTTGGGCTGTTTGCCTTGG
GCGAGCCCAAAGAACTTGCCCCTTTTCTCCTTGCATTAGCAAGCTAGGTCTGGCTGC
GTGGAGCTGGCAAGTAGATTTCAGCAACTTGAGCTTGAGTTGATGATCAATTAATAG
TGGCTGCCAGTTGTGCTGGCGTAAGTGGCCCACATCATGGGGAAGGAGTGCTGTGAT
TGACTAGTAATGGCTACCACGGGAAAGGGAAGGGGAAGCAGTAGCACTAATGCTATG
TAGTTGTCATCTTTGATCTGGCTAGGCCCTGGGAATCGGGTTTAGTCATCCTGTGGG
ATCTGTGTTAACTCTTTCATGCCACTGGTGAGGCATTTGTTAATTTGCTACTCAACT
TTGAGGAAAGACAGGGCCTTGGTCAGAGAGAGAATGCTCTGAACTCTGCTAAGGACA
TAGAGTCAGCCCATGGTGATTTAGCTCCTTGCTGTTCACCTCCTCTTTCCTGATGTC
TGCCTTGCTCTACAGCACAACCTCTTGAGGGTGGACAGGGAGAAAGATGATGGTGTC
AGAGGTCAAAACTATTATATATGACAGGGCACAAGATGGTCTGTGATCTTTGCACAG
ATGAATGGAAGTTGATGCACACCAACAAGAGGCAACTTGTCACTTTCTTTCTCAATA
TTAACTGGAATGCTGCCTCTTGGGTTCTCACCTGCATGGATGCTTTGAGTTGGATGT
GATACTGTCCATATTCTCCAGAGGATTACCTGGCTGAACCATTGGCTCTGTTCACCA
GTGACAGATGGTTTCCCCATCCACTGAGTGTAGCATCCTCAGAGGTAGGCAAGTTTG
CTTCTAGGGAGTTAGCATGTAGATGGGATATTGGGATGAGGAAAGGAAAATCAGGTA
GATGGTGCTTTTTTTCCCCCAAATCTAAGTATTCTATGTCATGGTTTTAAACTTTGC
CATGAACTCCTGGGCTTTGGGGGAAGAGAAAGTTCCATTCATTTAAATGAATAAGGT
GTTGAAAGAGTGCAGGGGGTTGGGAGGAAGCATGTAAGAGAGGGAACATTTCCTTAG
ATGTTACCCAGATGGTTCTGGGGGAGACAGAAAGAGGTGCGGCAGGACTCTTATCT
TAAAAAGTAAACAAAACAAAACAAAACAAAACAAAAAAACTAGATATGTAATTTCTA
AACACCCAGATCACAATGACAAGATGCCACTCCAACCATGGGACACCTTCATGATAC

TAGGTTTGTACTTCCTGGTCTCTGGGATGACTTCAGATTCTGCTGGCCAAGGCAAAT
TGAACTCAGTTCAAGATGGCCACCACTGGTAGACGTGTAGATAGAAAAGAGGACTGG
TCTTGGGAACATCTTTGGAAAAACCAACAAACAATAGTTCTAGGGAGATGAGAAAAA
AATTCACCTTACAGTGCTAAGAAAGTGCATTAGAATGGAATTGCCCTTTCCTTAAGG
AGACAGTTTGGGCTCTCCCCTTGCCACCGGCTCTGGTGTTTTGGCTTATGCGTTCCT
TCAGGTTGAGCTGAGCAGTGTGTTATGGGAAGCTGCTCAATTTCCTTTCATTCAATT
CCACCTCCTTCCTGAACTCTAATAGAGGTTAAAAGGGAAAAAAAAAATTCTGTAGAT
AGCAAATTGTGTGTGTGGGGGGGGGTGGGGGTGTGGGTGCATGGAGGACAACCTGCA
ACTCTGAGCTCCCTACTTCCTGCCTCATTTCATGCAGTCTTTTCTGAACAGCCTATG
CTGCTGCCCTGCTGGCCCCTTGTGCACGGCAGCTGGCCGTGTCCGTAGCTGTCAGTA
TGACTTAGATCTAGCTCCTACCTACTGGTTGATGTGTTTTTTCCTTTTGCCAAGTGA
TTGAGTCTGTTTAGTAGTTTCCATCATTCTAGTCTTTAAGTAAAAATGACACTATTG
AGGAAAGTCAGTCTACTCCCTTCTTCCTCCCCCCAAACACGTGTTCTCTTTTGTCAG
GAAACTCAGCCAGTGGGCTGTGGCAGAGAAAGTCCTCCACTCAGAGGCAGAGACTGA
GTTAAGTCATAGGTGGCCTTAGGCATCTGCATTGTTTGCAGGGGTTAAGTTTTCCTT
CCAGTGAGGGCTGGAGGGATGAATTAGCTGGTACCTGAAGCCCCGCTTAGCTCTGAC
ACTCTGCCAACATCCTCTGATTCTAGGTGTGGTGTTGACTGTCCTTTCAAGGAAAAA
CTTGCAATAGAGGGAAAAGCCATTAAAGCAGCTCCCTGCTTCATCATTAAGTCCTGT
CATCCCTACCAGCCAATCCCAGTCAAAGAAGTTATGCTTTATTCACTTCTGTGGAAT
TACAAGTGAGAGACACTTTTAGGACCTGATGGACAAAGCAGGAGATTCACTGTCAGC
TTTCCTGGTCCTCTCCTTACTTCTGTGGGCCTTGCACCGTCTTAGTTTACACATCTG
CCAAAGGGGTAGAATTACACTTCTTTTTACAGGTAAATGTCAAGGCACAATCAGTTT
TCAGGAAGTGCTTCAAGACCCCAGGTGAAATGAAAATGCTAAGTACCCTCTGAATGG
CCATGCCTGTTACCAGGTGCTGCTTCTTCAGATGATGGGGAGCACTTTTCAGGGTGA
AATTCAGGCGAGTTTTGCCCAGGCCTGCTGTCTTGAGTACAAATGTGAATGATCGAC
TGACTGCTTGTTGCCAAACTGGAAATGTTCTGTAGGGATTTACTGGCATGGTATCAT
TCCTAGAAGAAAAAAGAGAGAAACTTGACTGCACATTAAAAAAAAAAAAAATCCACA
TTGTGACTTTTATTTAATTTCTATTTTTTTTGGTAATAAAAAGTTGACTTTTTTATT
TGAATTTGTCTTTTTTATTTATTGGTCTGAAAGGCATTTCAAAGGTATTATAATAAT
ATATTGGTGTAATTTAATTGGTGCAACATGCTTTATGGCTCCTGTCAAAATTGGTTT
TCACTCATTTGATTGGTTTGAGCCCAGAACAGCCTACAGGGGAAAAACAAGCTGGAT
AACCACCCAAAGTGTTTGTATTTTCGTTGGAAACTGATTTTTGTTTCATTTTGGTTT
TTGTTTCTGTTTTTATTTTTAAATTAAATAAATTGCAATGAACTG (SEQ ID NO:
56)

```
>PAR2.3a
GGTTTAATTA CCCAAGTTTG AGGGTATGGT AGTACCAATA GGTTCACAAC
ATCGTCAAAA AAATATGCCT CCAAGTGGAC GTTTCGATAG AACAATCGCC
TTGGCTGGCA CTGGACATTT GAAAATTGGA AACTTTGTCA TCAAAGAGAC
AATTGGAAAA GGAGCATTTG GAGCTGTTAA AAGAGGAACA CATATTCAAA
CCGGATACGA CGTCGCCATT AAAATTCTGA ATCGTGGAAG AATGAAAGGA
CTCGGAACAG TTAACAAAAC TCGAAATGAG ATTGATAATC TTCAGAAACT
CACACATCCA CATATCACAC GTCTCTTCCG TGTTATCAGT ACTCCTTCGG
ACATATTTTT GGTCATGGAA CTAGTTTCTG GTGGAGAACT TTTCAGTTAT
ATAACTAGAA AAGGTGCTCT TCCAATCAGA GAAAGTCGAC GATACTTTCA
ACAAATTATT TCTGGGGTCA GTTATTGTCA TAATCATATG ATTGTGCATA
GAGACTTGAA GCCCGAAAAT CTTCTTCTG   (SEQ ID NO:57)
```

>Spliced T01C8.1b

```
ATGTTTTCTC ATCAAGATCG AGACCGCGAT AGGAAGGAGG ACGGTGGTGG
AGACGGTACT GAGATGAAAT CCAAGAGTCG GAGTCAGCCA AGTGGACTTA
ATCGCGTGAA AAATCTTTCA AGAAAGCTAT CGGCAAAGTC AAGAAAGGAA
CGCAAGGACC GCGATAGCAC AGACAACAGT TCGAAAATGT CGTCTCCAGG
AGGAGAAACG TCGACGAAGC AGCAACAAGA GCTCAAAGCT CAAATCAAAA
TCGGACATTA CATTCTCAAG GAGACACTCG GAGTTGGTAC TTTTGGTAAA
GTAAAAGTTG GAATCCATGA GACAACTCAA TACAAAGTGG CTGTCAAGAT
TCTGAACCGT CAGAAGATCA AGTCACTGGA TGTCGTTGGA AAGATTCGCC
GCGAAATCCA AAACCTCTCG CTCTTCCGCC ATCCGCATAT CATCCGCCTC
TACCAAGTCA TCAGTACACC TTCTGACATT TTCATGATTA TGGAGCACGT
TTCCGGCGGG GAGCTCTTTG ACTACATTGT TAAGCACGGA CGGCTGAAGA
CCGCAGAAGC TCGTCGCTTC TTTCAACAAA TCATTTCCGG CGTTGACTAC
TGTCATCGTC ATATGGTTGT CCATAGAGAT TTGAAGCCAG AGAATTTGTT
GCTCGATGAG CAGAACAATG TGAAGATTGC GGACTTTGGA CTTTCAAATA
TTATGACGGA TGGTGACTTC TTACGCACCA GCTGCGGATC GCCAAATTAT
GCTGCCCCTG AGGTTATTAG CGGAAAGTTG TACGCAGGTC CGAAGTTGA
TGTATGGTCG TGTGGAGTCA TTTTGTATGC ACTTCTTTGT GGAACCCTGC
CATTTGATGA TGAGCACGTG CCAAGTCTTT TCAGAAAAAT TAAATCTGGC
GTATTCCCAA CTCCAGACTT TCTGGAGCGC CCAATTGTAA ATCTGCTTCA
CCATATGCTC TGCGTAGACC CGATGAAGAG GGCCACCATC AAGGACGTCA
TTGCTCACGA GTGGTTCCAG AAGGATTTGC CGAACTACTT GTTCCCACCA
ATCAACGAGA GTGAGGCTTC CATTGTTGAT ATTGAGGCTG TCCGAGAGGT
CACTGAGTTT CAGCGCTATC ATGTCGCCGA AGAGGAAGTC ACCTCAGCAT
TGCTGGGAGA TGATCCACAT CACCATTTGT CGATTGCATA TAACTTGATT
GTTGATAACA AGAGAATTGC CGATGAGACT GCCAAGTTGT CAATTGAGGA
GTTTTATCAA GTGACGCCGA ATAAGGGACC TGGACCAGTT CATCGCCATC
CAGAGCGCAT TGCAGCGTCA GTCAGCAGCA AGATCACACC AACTCTCGAC
AACACGGAAG CCAGTGGCGC GAACCGCAAC AAACGTGCCA AGTGGCATCT
GGGTATCCGA TCCCAAAGTC GTCCGGAAGA CATCATGTTC GAGGTGTTCC
GTGCGATGAA GCAGCTGGAC ATGGAGTGGA AGGTGCTCAA CCCATATCAT
GTGATTGTTC GTCGCAAGCC TGATGCACCC GCTGCCGACC CGCCAAAGAT
GTCGTTGCAG TTGTACCAAG TGGATCAGAG AAGTTACTTG TTGGATTTCA
AGAGTTTGGC CGACGAGGAG TCTGGATCCG CCAGTGCATC TTCATCCCGA
CACGCATCAA TGTCTATGCC GCAGAAGCCG GCCGGAATTC GTGGAACTAG
AACGTCAAGC ATGCCACAGG CGATGAGTAT GGAGGCGAGT ATTGAGAAAA
TGGAAGTGCA TGATTTTTCG GACATGTCGT GCGATGTGAC ACCACCACCA
TCTCCTGGAG GAGCTAAGCT TTCCCAGACT ATGCAATTCT TTGAGATTTG
CGCCGCATTG ATTGGAACAC TGGCTCGTTA A (SEQ ID NO:58)
```

```
>Spliced T01C8.1a
ATGTTTTCTC ATCAAGATCG AGACCGCGAT AGGAAGGAGG ACGGTGGTGG
AGACGGTACT GAGATGAAAT CCAAGAGTCG GAGTCAGCCA AGTGGACTTA
ATCGCGTGAA AAATCTTTCA AGAAAGCTAT CGGCAAAGTC AAGAAAGGAA
CGCAAGGACC GCGATAGCAC AGACAACAGT TCGAAAATGT CGTCTCCAGG
AGGAGAAACG TCGACGAAGC AGCAACAAGA GCTCAAAGCT CAAATCAAAA
TCGGACATTA CATTCTCAAG GAGACACTCG GAGTTGGTAC TTTTGGTAAA
GTAAAAGTTG GAATCCATGA GACAACTCAA TACAAAGTGG CTGTCAAGAT
```

```
TCTGAACCGT CAGAAGATCA AGTCACTGGA TGTCGTTGGA AAGATTCGCC
GCGAAATCCA AAACCTCTCG CTCTTCCGCC ATCCGCATAT CATCCGCCTC
TACCAAGTCA TCAGTACACC TTCTGACATT TTCATGATTA TGGAGCACGT
TTCCGGCGGG GAGCTCTTTG ACTACATTGT TAAGCACGGA CGGCTGAAGA
CCGCAGAAGC TCGTCGCTTC TTTCAACAAA TCATTTCCGG CGTTGACTAC
TGTCATCGTC ATATGGTTGT CCATAGAGAT TTGAAGCCAG AGAATTTGTT
GCTCGATGAG CAGAACAATG TGAAGATTGC GGACTTTGGA CTTTCAAATA
TTATGACGGA TGGTGACTTC TTACGCACCA GCTGCGGATC GCCAAATTAT
GCTGCCCCTG AGGTTATTAG CGGAAAGTTG TACGCAGGTC CCGAAGTTGA
TGTATGGTCG TGTGGAGTCA TTTTGTATGC ACTTCTTTGT GGAACCCTGC
CATTTGATGA TGAGCACGTG CCAAGTCTTT TCAGAAAAAT TAAATCTGGC
GTATTCCCAA CTCCAGACTT TCTGGAGCGC CCAATTGTAA ATCTGCTTCA
CCATATGCTC TGCGTAGACC CGATGAAGAG GGCCACCATC AAGGACGTCA
TTGCTCACGA GTGGTTCCAG AAGGATTTGC CGAACTACTT GTTCCCACCA
ATCAACGAGA GTGAGGCTTC CATTGTTGAT ATTGAGGCTG TCCGAGAGGT
CACTGAGCGC TATCATGTCG CCGAAGAGGA AGTCACCTCA GCATTGCTGG
GAGATGATCC ACATCACCAT TTGTCGATTG CATATAACTT GATTGTTGAT
AACAAGAGAA TTGCCGATGA GACTGCCAAG TTGTCAATTG AGGAGTTTTA
TCAAGTGACG CCGAATAAGG GACCTGGACC AGTTCATCGC CATCCAGAGC
GCATTGCAGC GTCAGTCAGC AGCAAGATCA CACCAACTCT CGACAACACG
GAAGCCAGTG GCGCGAACCG CAACAAACGT GCCAAGTGGC ATCTGGGTAT
CCGATCCCAA AGTCGTCCGG AAGACATCAT GTTCGAGGTG TTCCGTGCGA
TGAAGCAGCT GGACATGGAG TGGAAGGTGC TCAACCCATA TCATGTGATT
GTTCGTCGCA AGCCTGATGC ACCCGCTGCC GACCCGCCAA AGATGTCGTT
GCAGTTGTAC CAAGTGGATC AGAGAAGTTA CTTGTTGGAT TTCAAGAGTT
TGGCCGACGA GGAGTCTGGA TCCGCCAGTG CATCTTCATC CCGACACGCA
TCAATGTCTA TGCCGCAGAA GCCGGCCGGA ATTCGTGGAA CTAGAACGTC
AAGCATGCCA CAGGCGATGA GTATGGAGGC GAGTATTGAG AAAATGGAAG
TGCATGATTT TTCGGACATG TCGTGCGATG TGACACCACC ACCATCTCCT
GGAGGAGCTA AGCTTTCCCA GACTATGCAA TTCTTTGAGA TTTGCGCCGC
ATTGATTGGA ACACTGGCTC GTTAA  (SEQ ID NO:59)
```

## Screening Assays

In one aspect, the invention provides assays for screening for a test compound, or more typically, a library of test compounds, to evaluate an effect of the test compound on AMPK pathway activity *in vitro,* in a cell, or in an organism or to evaluate interaction between the test compound and an AMPK pathway component.

**Test Compounds. A** "test compound" can be any chemical compound, for example, a macromolecule (e.g.,

a polypeptide, a protein complex, or a nucleic acid) or a small molecule (e.g., an amino acid, a nucleotide, an organic or inorganic compound). The test compound can have a formula weight of less than about 10,000 grams per mole, less than 5,000 grams per mole, less than 1,000 grams per mole, or less than about 500 grams per mole. The test compound can be naturally occurring (e.g., a herb or a nature product), synthetic, or both. Examples of macromolecules are proteins, protein complexes, and glycoproteins, nucleic acids, e.g., DNA, RNA and PNA (peptide nucleic acid). Examples of small molecules are peptides, peptidomimetics (e.g., peptoids), amino acids, amino acid analogs, polynucleotides, polynucleotide analogs, nucleotides, nucleotide analogs, organic or inorganic compounds e.g., heteroorganic or organometallic compounds. A test compound can be the only substance assayed by the method described herein. Alternatively, a collection of test compounds can be assayed either consecutively or concurrently by the methods described herein.

In one preferred embodiment, high throughput screening methods involve providing a combinatorial chemical or peptide library containing a large number of potential therapeutic compounds (potential modulator or ligand compounds). Such "combinatorial chemical libraries" or "ligand libraries" are then screened in one or more assays, as described herein, to identify those library members (particular chemical species or subclasses) that display a desired characteristic activity. The compounds thus identified can serve as conventional "lead compounds" or can themselves be used as potential or actual therapeutics.

A combinatorial chemical library is a collection of diverse chemical compounds generated by either chemical synthesis or biological synthesis, by combining a number of chemical "building blocks" such as reagents. For example, a linear combinatorial chemical library such as a polypeptide library is formed by combining a set of chemical building blocks (amino acids) in every possible way for a given compound length (i.e., the number of amino acids in a polypeptide compound). Millions of chemical compounds can be synthesized through such combinatorial mixing of chemical building blocks.

Preparation and screening of combinatorial chemical libraries is well known to those of skill in the art. Such combinatorial chemical libraries include, but are not limited to, peptide libraries *(see, e.g.,* U.S. 5,010,175, Furka, Int. J. Pept. Prot. Res. 37:487-493 (1991) and Houghton et al., Nature 354:84-88 (1991)). Other chemistries for generating chemical diversity libraries can also be used. Such chemistries include, but are not limited to: peptoids (e.g., PCT Publication No. WO 91/19735), encoded peptides (e.g., PCT Publication No. WO 93/20242), random bio-oligomers (e.g., PCT Publication No. WO 92/00091), benzodiazepines (e.g., U.S. 5,288,514), diversomers such as hydantoins, benzodiazepines and dipeptides (Hobbs et al., Proc. Nat. Acad. Sci. USA 90:6909-6913 (1993)), vinylogous polypeptides (Hagihara et al., J. Amer. Chem. Soc. 114:6568 (1992)), nonpeptidal peptidomimetics with glucose scaffolding (Hirschmann et al., J. Amer. Chem. Soc. 114:9217-9218 (1992)), analogous organic syntheses of small compound libraries (Chen et al., J. Amer. Chem. Soc. 116:2661 (1994)), oligocarbamates (Cho et al., Science 261:1303 (1993)), and/or peptidyl phosphonates (Campbell et al., J. Org. Chem. 59:658 (1994)), nucleic acid libraries (*see* Ausubel, Berger and Sambrook, all *supra*), peptide nucleic acid libraries (*see, e.g.,* U.S. 5,539,083), antibody libraries *(see, e.g.,* Vaughn et al., Nature Biotechnology, 14(3):309-314 (1996) and PCT/US96/10287), carbohydrate libraries *(see, e.g.,* Liang et al., Science, 274:1520-1522 (1996) and U.S. 5,593,853), small organic molecule libraries *(see, e.g.,* benzodiazepines, Baum C&EN, Jan 18, page 33 (1993); isoprenoids, U.S. 5,569,588; thiazolidinones and metathiazanones, U.S. 5,549,974; pyrrolidines, U.S.s 5,525,735 and 5,519,134; morpholino compounds, U.S. 5,506,337; benzodiazepines, 5,288,514, and the like). Additional examples of methods for the synthesis of molecular libraries can be found in the art, for example in: DeWitt et al. (1993) Proc. Natl. Acad. Sci. U.S.A. 90:6909; Erb et al. (1994) Proc. Natl. Acad. Sci. USA 91:11422; Zuckermann et al. (1994). J. Med. Chem. 37:2678; Cho et al. (1993) Science 261:1303; Carrell et al. (1994) Angew. Chem. Int. Ed. Engl. 33:2059; Carell et al. (1994) Angew. Chem. Int. Ed. Engl. 33:2061; and Gallop et al. (1994) J. Med. Chem. 37:1233.

Some exemplary libraries are used to generate variants from a particular lead compound. One method includes generating a combinatorial library in which one or more functional groups of the lead compound are varied, e.g., by derivatization. Thus, the combinatorial library can include a class of compounds which have a common structural feature (e.g., scaffold or framework). Examples of lead compounds which can be used as starting molecules for library generation include: biguanides such as metformin; thiazolidinediones, e.g., rosiglitazone and pioglitazone; an AMP analog such as AICAR= 5'-aminoimidazole-4-carboxyamide-ribosid; leptin and leptin-related molecules; adiponectin and Adiponectin-related molecules.

Devices for the preparation of combinatorial libraries are commercially available (*see, e.g.,* 357 MPS, 390 MPS, Advanced Chem Tech, Louisville KY, Symphony, Rainin, Woburn, MA, 433A Applied Biosystems, Foster City, CA, 9050 Plus, Millipore, Bedford, MA). In addition, numerous combinatorial libraries are themselves commercially available (*see, e.g.,* ComGenex, Princeton, N.J., Asinex, Moscow, Ru, Tripos, Inc., St. Louis, MO, ChemStar, Ltd, Moscow, RU, 3D Pharmaceuticals, Exton, PA, Martek Biosciences, Columbia, MD, etc.).

The test compounds of the present invention can also be obtained from: biological libraries; peptoid libraries (libraries of molecules having the functionalities of peptides, but with a novel, non-peptide backbone which are resistant to enzymatic degradation but which nevertheless remain bioactive; see, e.g., Zuckermann, R.N. et al. (1994) J. Med. Chem. 37:2678-85); spatially addressable parallel solid phase or solution phase libraries; synthetic library methods requiring deconvolution; the 'one-bead one-compound' library method; and synthetic library methods using affinity chromatography selection. The biological libraries include libraries of nucleic acids and libraries of proteins. Some nucleic acid libraries encode a diverse set of proteins (e.g., natural and artificial proteins; others provide, for example, functional RNA and DNA molecules such as nucleic acid aptamers or ribozymes. A peptoid library can be made to include structures similar to a peptide library. (See also Lam (1997) Anticancer Drug Des. 12:145). A library of proteins may be produced by an expression library or a display library (e.g., a phage display library).

Libraries of compounds may be presented in solution (e.g., Houghten (1992) Biotechniques 13:412-421), or on beads (Lam (1991) Nature 354:82-84), chips (Fodor (1993) Nature 364:555-556), bacteria (Ladner, U.S. 5,223,409), spores (Ladner U.S. 5,223,409), plasmids (Cull et al. (1992) Proc Natl Acad Sci USA 89:1865-1869) or on phage (Scott and Smith (1990) Science 249:386-390; Devlin (1990) Science 249:404-406; Cwirla et al. (1990) Proc. Natl. Acad. Sci. 87:6378-6382; Felici (1991) J. Mol. Biol. 222:301-310; Ladner *supra.*).

Any assay herein, e.g., an in vitro assay or an in vivo assay, can be performed individually, e.g., just with the test compound, or with appropriate controls. For example, a parallel assay without the test compound, or other parallel assays without other reaction components, e.g., without a target or without a substrate. Alternatively, it is possible to compare assay results to a reference, e.g., a reference value, e.g., obtained from the literature, a prior assay, and so forth. Appropriate correlations and art known statistical methods can be used to evaluate an assay result.

*In vitro* **Assays.** AMPK activity can be assayed *in vitro.* See, e.g., Hardie et al. (1997) Eur J. Biochem 246: 259; Hardie et al., (1998) Annu Rev Biochem 67:851; Vavvas et al. (1997) J Biol Chem 272:13256; and Winder et al. (1996) Am J Physiol Endocrinol Metab 270:E299. The reaction mixture can include radiolabeled ATP, e.g., [$^{32}$P]ATP and an artificial peptide substrate, e.g., a 15-amino acid peptide called "SAMS" which is an amino acid sequence from the acetyl-CoA carboxylase (ACC) enzyme. The SAMS peptide can include the sequence: HMRSAMSGLHLVKRR (SEQ ID NO:7). See, e.g., Davies et al. (1989) Eur. J. Biochem, 186:123. A peptide from glycogen synthase can also be used, e.g., a peptide that includes the sequence PLSRTLSVAAKK (SEQ ID NO:9).

An increase in AMPK activity will cause an increase in phosphorylation of the peptide. In some implementations, the reaction mixture can include AMP or creatine phosphate. Phosphorylation can be detected, e.g., using a scintillation counter after separation of free ATP from the peptide.

Another type of *in vitro* assay evaluates the ability of a test compound to modulate interaction between a first AMPK pathway component and a second AMPK pathway component, e.g., between AMPK alpha and beta-gamma. This type of assay can be accomplished, for example, by coupling one of the components, with a radioisotope or enzymatic label such that binding of the labeled component to the other AMPK pathway component can be determined by detecting the labeled compound in a complex. A AMPK pathway component can be labeled with $^{125}$I, $^{35}$S, $^{14}$C, or $^{3}$H, either directly or indirectly, and the radioisotope detected by direct counting of radioemmission or by scintillation counting. Alternatively, a component can be enzymatically labeled with, for example, horseradish peroxidase, alkaline phosphatase, or luciferase, and the enzymatic label detected by determination of conversion of an appropriate substrate to product. Competition assays can also be used to evaluate a physical interaction between a test compound and a target.

Soluble and/or membrane-bound forms of isolated proteins (e.g., AMPK pathway components and their receptors or biologically active portions thereof) can be used in the cell-free assays of the invention. When membrane-bound forms of the protein are used, it may be desirable to utilize a solubilizing agent. Examples of such solubilizing agents include non-ionic detergents such as n-octylglucoside, n-dodecylglucoside, n-dodecylmaltoside, octanoyl-N-methylglucamide, decanoyl-N-methylglucamide, Triton® X-100, Triton® X-114, Thesit®, Isotridecypoly(ethylene glycol ether)$_n$, 3-[(3-cholamidopropyl)dimethylamminio]-1-propane sulfonate (CHAPS), 3-[(3-cholamidopropyl)dimethylamminio]-2-hydroxy-1-propane sulfonate (CHAPSO), or N-dodecyl=N,N-dimethyl-3-ammonio-1-propane sulfonate. In another example, the AMPK pathway component (e.g., GLUT-4) can reside in a membrane, e.g., a liposome or other vesicle.

Cell-free assays involve preparing a reaction mixture of the target protein (e.g., the AMPK pathway component) and the test compound under conditions and for a time sufficient to allow the two components to interact and bind, thus forming a complex that can be removed and/or detected.

The interaction between two molecules can also be detected, e.g., using a fluorescence assay in which at least one molecule is fluorescently labeled. One example of such an assay includes fluorescence energy transfer (FET or FRET for fluorescence resonance energy transfer) (see, for example, Lakowicz et al., U.S. 5,631,169; Stavrianopoulos, et al., U.S. 4,868,103). A fluorophore label on the first, 'donor' molecule is selected such that its emitted fluorescent energy will be absorbed by a fluorescent label on a second, 'acceptor' molecule, which in turn is able to fluoresce due to the absorbed energy. Alternately, the 'donor' protein molecule may simply utilize the natural fluorescent energy of tryptophan residues. Labels are chosen that emit different wavelengths of light, such that the 'acceptor' molecule label may be differentiated from that of the 'donor'. Since the efficiency of energy transfer between the labels is related to the distance separating the molecules, the spatial relationship between the molecules can be assessed. In a situation in which binding occurs between the molecules, the fluorescent emission of the 'acceptor' molecule label in the assay should be maximal. A FET binding event can be conveniently measured through standard fluorometric detection means well known in the art (e.g., using a fluorimeter).

Another example of a fluorescence assay is fluorescence polarization (FP). For FP, only one component needs to be labeled. A binding interaction is detected by a change in molecular size of the labeled component. The size change alters the tumbling rate of the component in solution and is detected as a change in FP. See, e.g., Nasir et al. (1999) Comb Chem HTS 2:177-190; Jameson et al. (1995) Methods Enzymol 246:283; Seethala et al.. (1998) Anal Biochem. 255:257. Fluorescence polarization can be monitored in multi-well plates. See, e.g, Parker et al. (2000) Journal of Biomolecular Screening 5:77 - 88; and Shoeman, *et al..* (1999) 38, 16802-16809.

In another embodiment, determining the ability of the AMPK pathway component protein to bind to a target molecule can be accomplished using real-time Biomolecular Interaction Analysis (BIA) (see, e.g., Sjolander, S. and Urbaniczky, C. (1991) Anal. Chem. 63:2338-2345 and Szabo et al. (1995) Curr. Opin. Struct. Biol. 5:699-705). "Surface plasmon resonance" or "BIA" detects biospecific interactions in real time, without labeling any of the interactants (e.g., BIAcore). Changes in the mass at the binding surface (indicative of a binding event) result in alterations of the refractive index of light near the surface (the optical phenomenon of surface plasmon resonance (SPR)), resulting in a detectable signal which can be used as an indication of real-time reactions between biological molecules.

In one embodiment, the AMPK pathway component is anchored onto a solid phase. The AMPK pathway component/test compound complexes anchored on the solid phase can be detected at the end of the reaction, e.g., the binding reaction. For example, the AMPK pathway component can be anchored onto a solid surface, and the test compound, (which is not anchored), can be labeled, either directly or indirectly, with detectable labels discussed herein.

It may be desirable to immobilize either the AMPK pathway component or an anti-AMPK pathway component antibody to facilitate separation of complexed from uncomplexed forms of one or both of the proteins, as well as to accommodate automation of the assay. Binding of a test compound to a AMPK pathway component protein, or interaction of a AMPK pathway component protein with a second component in the presence and absence of a candidate compound, can be accomplished in any vessel suitable for containing the reactants. Examples of such vessels include microtiter plates, test tubes, and micro-centrifuge tubes. In one embodiment, a fusion protein can be provided which adds a domain that allows one or both of the proteins to be bound to a matrix. For example, glutathione-S-transferase/AMPK pathway component fusion proteins or glutathione-S-transferase/target fusion proteins can be adsorbed onto glutathione sepharose beads (Sigma Chemical, St. Louis, MO) or glutathione derivatized microtiter plates, which are then combined with the test compound or the test compound and either the non-adsorbed target protein or AMPK pathway component protein, and the mixture incubated under conditions conducive to complex formation (e.g., at physiological conditions for salt and pH). Following incubation, the beads or microtiter plate wells are washed to remove any unbound components, the matrix immobilized in the case of beads, complex determined either directly or indirectly, for example, as described above. Alternatively, the complexes can be dissociated from the matrix, and the level of AMPK pathway component binding or activity determined using standard techniques.

Other techniques for immobilizing either a AMPK pathway component protein or a target molecule on matrices include using conjugation of biotin and streptavidin. Biotinylated AMPK pathway component protein or target molecules can be prepared from biotin-NHS (N-hydroxy-succinimide) using techniques known in the art (e.g., biotinylation kit, Pierce Chemicals, Rockford, IL), and immobilized in the wells of streptavidin-coated 96 well plates (Pierce Chemical).

In order to conduct the assay, the non-immobilized component is added to the coated surface containing the anchored component. After the reaction is complete, unreacted components are removed (e.g., by washing) under conditions such that any complexes formed will remain immobilized on the solid surface. The detection of complexes anchored on the solid surface can be accomplished in a number of ways. Where the previously non-immobilized component is pre-labeled, the detection of label immobilized on the surface indicates that complexes were formed. Where the previously non-immobilized component is not pre-labeled, an indirect label can be used to detect complexes anchored on the surface, e.g., using a labeled antibody specific for the immobilized component (the antibody, in turn, can be directly labeled or indirectly labeled with, e.g., a labeled anti-Ig antibody).

In one embodiment, this assay is performed utilizing antibodies reactive with a AMPK pathway component protein or target molecules but which do not interfere with binding of the AMPK pathway component protein to its target molecule. Such antibodies can be derivatized to the wells of the plate, and unbound target or the AMPK pathway component protein trapped in the wells by antibody conjugation. Methods for detecting such complexes, in addition to those described above for the GST-immobilized complexes, include immunodetection of complexes using antibodies reactive with the AMPK pathway component protein or target molecule, as well as enzyme-linked assays which rely on detecting an enzymatic activity associated with the AMPK pathway component protein or target molecule.

Alternatively, cell free assays can be conducted in a liquid phase. In such an assay, the reaction products are separated from unreacted components, by any of a number of standard techniques, including but not limited to: differential centrifugation (see, for example, Rivas, G., and Minton, A.P., (1993) Trends Biochem Sci 18:284-7); chromatography (gel filtration chromatography, ion-exchange chromatography); electrophoresis (see, e.g., Ausubel, F. et al., eds. Current Protocols in Molecular Biology 1999, J. Wiley: New York.); and immunoprecipitation (see, for example, Ausubel, F. et al., eds. (1999) Current Protocols in Molecular Biology, J. Wiley: New York). Such resins and chromatographic techniques are known to one skilled in the art (see, e.g., Heegaard, N.H., (1998) J Mol Recognit 11:141-8; Hage, D.S., and Tweed, S.A. (1997) J Chromatogr B Biomed Sci Appl. 699:499-525). Further, fluorescence energy transfer may also be conveniently utilized, as described herein, to detect binding without further purification of the complex from solution.

In a preferred embodiment, the assay includes contacting the AMPK pathway component protein or biologically active portion thereof with a known compound which binds a AMPK pathway component to form an assay mixture, contacting the assay mixture with a test compound, and determining the ability of the test compound to interact with a AMPK pathway component protein, wherein determining the ability of the test compound to interact with the AMPK pathway component protein includes determining the ability of the test compound to preferentially bind to the AMPK pathway component or biologically active portion thereof, or to modulate the activity of a target molecule, as compared to the known compound.

The target products of the invention can, *in vivo,* interact with one or more cellular or extracellular macromolecules, such as proteins. For the purposes of this discussion, such cellular and extracellular macromolecules are referred to herein as "binding partners." Compounds that disrupt such interactions can be useful in regulating the activity of the target product. Such compounds can include, but are not limited to molecules such as antibodies, peptides, and small molecules. The preferred targets/products for use in this embodiment are the AMPK pathway components. In an alternative embodiment, the invention provides methods for determining the ability of the test compound to modulate the activity of a AMPK pathway component protein through modulation of the activity of a downstream effector of a AMPK pathway component target molecule. For example, the activity of the effector molecule on an appropriate target can be determined, or the binding of the effector to an appropriate target can be determined, as previously described.

To identify compounds that interfere with the interaction between the target product and its cellular or extracellular binding partner(s), a reaction mixture containing the target product and the binding partner is prepared, under conditions and for a time sufficient, to allow the two products to form complex. In order to test an inhibitory agent, the reaction mixture is provided in the presence and absence of the test compound. The test compound can be initially included in the reaction mixture, or can be added at a time subsequent to the addition of the target and its cellular or extracellular binding partner. Control reaction mixtures are incubated without the test compound or with a placebo. The formation of any complexes between the target product and the cellular or extracellular binding partner is then detected. The formation of a complex in the control reaction, but not in the reaction mixture containing the test compound, indicates that the compound interferes with the interaction of the target product and the interactive binding partner. Additionally, complex formation within reaction mixtures containing the test compound and normal target product can also be compared to complex formation within reaction mixtures containing the test compound and mutant target product. This comparison can be important in those cases wherein it is desirable to identify compounds that disrupt interactions of mutant but not normal target products.

These assays can be conducted in a heterogeneous or homogeneous format. Heterogeneous assays involve anchoring either the target product or the binding partner onto a solid phase, and detecting complexes anchored on the solid phase at the end of the reaction. In homogeneous assays, the entire reaction is carried out in a liquid phase. In either approach, the order of addition of reactants can be varied to obtain different information about the compounds being tested. For example, test compounds that interfere with the interaction between the target products and the binding partners, e.g., by competition, can be identified by conducting the reaction in the presence of the test substance. Alternatively, test compounds that disrupt preformed complexes, e.g., compounds with higher binding constants that displace one of the components from the complex, can be tested by adding the test compound to the reaction mixture after complexes have been formed. The various formats are briefly described below.

In a heterogeneous assay system, either the target product or the interactive cellular or extracellular binding partner, is anchored onto a solid surface (e.g., a microtiter plate), while the non-anchored species is labeled, either directly or indirectly. The anchored species can be immobilized by non-covalent or covalent attachments. Alternatively, an immobilized antibody specific for the species to be anchored can be used to anchor the species to the solid surface.

In order to conduct the assay, the partner of the immobilized species is exposed to the coated surface with or without the test compound. After the reaction is complete, unreacted components are removed (e.g., by washing) and any complexes formed will remain immobilized on the solid surface. Where the non-immobilized species is pre-labeled, the detection of label immobilized on the surface indicates that complexes were formed. Where the non-immobilized species is not pre-labeled, an indirect label can be used to detect complexes anchored on the surface; e.g., using a labeled antibody specific for the initially non-immobilized species (the antibody, in turn, can be directly labeled or indirectly labeled with, e.g., a labeled anti-Ig antibody). Depending upon the order of addition of reaction components, test compounds that inhibit complex formation or that disrupt preformed complexes can be detected.

Alternatively, the reaction can be conducted in a liquid phase in the presence or absence of the test compound, the reaction products separated from unreacted components, and complexes detected; e.g., using an immobilized antibody specific for one of the binding components to anchor any complexes formed in solution, and a labeled antibody specific for the other partner to detect anchored complexes. Again, depending upon the order of addition of reactants to the liquid phase, test compounds that inhibit complex or that disrupt preformed complexes can be identified.

In an alternate embodiment of the invention, a homogeneous assay can be used. For example, a preformed complex of the target product and the interactive cellular or extracellular binding partner product is prepared in that either the target products or their binding partners are labeled, but the signal generated by the label is quenched due to complex formation (see, e.g., U.S. 4,109,496 that utilizes this approach for immunoassays). The addition of a test substance that competes with and displaces one of the species from the preformed complex will result in the generation of a signal above background. In this way, test substances that disrupt target product-binding partner interaction can be identified.

In yet another aspect, the AMPK pathway component proteins can be used as "bait proteins" in a two-hybrid assay or three-hybrid assay (see, e.g., U.S. 5,283,317; Zervos et al. (1993) Cell 72:223-232; Madura et al. (1993) J. Biol. Chem. 268:12046-12054; Bartel et al. (1993) Biotechniques 14:920-924; Iwabuchi et al. (1993) Oncogene 8: 1693-1696; and Brent WO94/10300), to identify other proteins, which bind to or interact with the AMPK pathway component ("AMPK pathway component-binding proteins" or "AMPK pathway component-bp") and are involved in AMPK pathway component activity. Such AMPK pathway component-bps can be activators or inhibitors of signals by the AMPK pathway component proteins or AMPK pathway component targets as, for example, downstream elements of a AMPK pathway component-mediated signaling pathway.

The two-hybrid system is based on the modular nature of most transcription factors, which consist of separable DNA-binding and activation domains. Briefly, the assay utilizes two different DNA constructs. In one construct, the gene that codes for a AMPK pathway component protein is fused to a gene encoding the DNA binding domain of a known transcription factor (e.g., GAL-4). In the other construct, a DNA sequence, from a library of DNA sequences, that encodes an unidentified protein ("prey" or "sample") is fused to a gene that codes for the activation domain of the known transcription factor. (Alternatively the: AMPK pathway component protein can be the fused to the activator domain.) If the "bait" and the "prey" proteins are able to interact, *in vivo,* forming a AMPK pathway component-dependent complex, the DNA-binding and activation domains of the transcription factor are brought into close proximity. This proximity allows transcription of a reporter gene (e.g., lacZ) which is operably linked to a transcriptional regulatory site responsive to the transcription factor. Expression of the reporter gene can be detected and cell colonies containing the functional transcription factor can be isolated and used to obtain the cloned gene which encodes the protein which interacts with the AMPK pathway component protein. In another embodiment, the two-hybrid assay is used to monitor an interaction between two components of the AMPK pathway that are known to interact. The two hybrid assay is conducted in the presence of a test compound, and the assay is used to determine whether the test compound enhances or diminishes the interaction between the components.

In another embodiment, modulators of an AMPK pathway component gene expression are identified. For example, a cell or cell free mixture is contacted with a candidate compound and the expression of the AMPK pathway component mRNA or protein evaluated relative to the level of expression of AMPK pathway component mRNA or protein in the absence of the candidate compound. When expression of the AMPK pathway component mRNA or protein is greater in the presence of the candidate compound than in its absence, the candidate compound is identified as a stimulator of AMPK pathway component mRNA or protein expression. Alternatively, when expression of the AMPK pathway component mRNA or protein is less (statistically significantly less) in the presence of the candidate compound than in its absence, the candidate compound is identified as an inhibitor of the AMPK pathway component mRNA or protein expression. The level of the AMPK pathway component mRNA or protein expression can be determined by methods for detecting AMPK pathway component mRNA or protein.

Organismal Assays. Still other methods for evaluating a test compound include organismal based assays, e.g., using a mammal (e.g., a mouse, rat, primate, or other non-human), or other animal (e.g., Xenopus, zebrafish, or an invertebrate such as a fly or nematode). In some cases, the organism is a transgenic organism, e.g., an organism which includes a heterologous AMPK pathway component, (e.g., from a mammal, e.g., a human). The test compound can be administered to the organism once or as a regimen (regular or irregular). A parameter of the organism is then evaluated, e.g., an age-associated parameter or a parameter of the AMPK pathway. Test compounds that are indicated as of interest result in a change in the parameter relative to a reference, e.g., a parameter of a control organism. Other parameters (e.g., related to toxicity, clearance, and pharmacokinetics) can also be evaluated.

In some embodiment, the test compound is evaluated using an animal that has a particular disorder, e.g., an age associated disorder, or using an animal that is otherwise sensitized to developing a particular disorder, e.g., an age associated disorder. These disorders provide a sensitized system in which the test compound's effects on physiology can be observed. Exemplary disorders include: denervation, disuse atrophy; metabolic disorders (e.g., disorder of obese and/or diabetic animals such as db/db mouse and ob/ob mouse); cerebral, liver ischemia; cisplatin/taxol/vincristine models; various tissue (xenograft) transplants; transgenic bone models; Pain syndromes(include inflammatory and neuropathic disorders); Paraquot, genotoxic, oxidative stress models; pulmonary obstruction (e.g., asthma models); and tumor models. In a preferred embodiment, the animal model is an animal that has an altered phenotype when calorically restricted. For example, F344 rats provide a useful assay system for evaluating a test compound. When calorically restricted, F344 rats have a 0 to 10% incidence of nephropathy. However, when fed ad libitum, they typically have a 60 to 100% incidence of nephropathy. Additional animal models that may be used are listed in Table 1:

**Table 1: Exemplary Animal Models**

| | Mean Lifespan (months) | | |
| --- | --- | --- | --- |
| *Model* | *Ad lib* | **CR** | **Predisposition** |
| SH Rat | 18 | 30 | Hypertension |
| SA Mouse | 10 | 15 | Amyloid |
| NZB Mouse | 12 | 16 | SLE |
| *kd/kd* Mouse | 8 | 18 | Nephritis |
| MRL/1 Mouse | 6 | >15 | Autoimmune |
| *ob/ob* Mouse | 14 | 26 | Diabetes |

To evaluate a test compound, it is administered to the animal (e.g., an F344 rat or an animal listed in Table 1), and a parameter of the animal is evaluated, e.g., after a period of time. The animal can be fed ad libitum or normally (e.g., not under caloric restriction, although some parameters can be evaluated under such conditions). Typically, a cohort of such animals is used for the assay. Generally, a test compound can be indicated as favorably altering lifespan regulation in the animal if the test compound affects the parameter in the direction of the phenotype of a similar animal subject to caloric restriction. Such test compounds may cause at least some of the lifespan regulatory effects of caloric restriction, e.g., a subset of such effects, without having to deprive the organism of caloric intake.

In one embodiment, the parameter is an age-associated or disease associated parameter, e.g., a symptom of the disorder associated with the animal model (e.g., the disorder in the "Predisposition" column of Table 1). For example, the test compound can be administered to the SH Rat, and blood pressure is monitored. A test compound that is favorably indicated can cause an amelioration of the symptom relative to a similar reference animal not treated with the compound. In a related embodiment, the parameter is a parameter of the AMPK activity, e.g., fatty acid oxidation and ketogenesis, inhibition of cholesterol synthesis, lipogenesis, and triglyceride synthesis, lipolysis, stimulation of glucose uptake, and modulation of insulin secretion.

Still other *in vivo* models and organismal assays include evaluating an animal for a metabolic parameter, e.g., a parameter relevant to an insulin disorder. Exemplary metabolic parameters include: glucose concentration, insulin concentration, and insulin sensitivity. Another set of metabolic parameters are parameters associated with the function of the growth hormone (GH)/insulin-like growth factor (IGF-1) axis, e.g., GH concentration, IGF-1 concentration, GHSH concentration, and so forth. Another exemplary system features tumors, e.g., in an animal model. The tumors can be spontaneous or induced. For example, the tumors can be developed from cells that have a variety of genetic constitutions, e.g., they can be p53+ or p53-. It is also possible to use organisms that an autoimmune disorder, e.g., an NZB mouse, which is predisposed to SLE. To evaluate features of bone disease, it is possible, for example, to use an animal that has an ovariectomy as a model,. e.g., for osteoporosis. Similarly, for joint disease, the model can be based on adjuvant arthritis (e.g., mice can be immunized with cartilage proteoglycans, high mobility group proteins, streptococcal cell wall material, or collagens); for kidney disease, kd/kd mice can be used. Animal models of cognition, particularly learning and memory are also available. Animal models of diabetes and its complications are also available, e.g., the streptozotocin model. Canine models can be used, for example, for evaluating stroke and ischemia.

In assessing whether a test compound is capable of inhibiting the AMPK pathway for the purpose of altering life span regulation, a number of age-associated parameters or biomarkers can be monitored or evaluated. Exemplary age associated parameters include: (i) lifespan of the cell or the organism; (ii) presence or abundance of a gene transcript or gene product in the cell or organism that has a biological age-dependent expression pattern; (iii) resistance of the cell or organism to stress; (iv) one or more metabolic parameters of the cell or organism; (v) proliferative capacity of the cell or a set of cells present in the organism; and (vi) physical appearance or behavior of the cell or organism.

Characterization of molecular differences between two such organisms, e.g., one reference organism and one organism treated with an AMPK pathway modulator can reveal a difference in the physiological state of the organisms. The reference organism and the treated organism are typically the same chronological age. Generally, organisms of the same chronological age may have lived for an amount of time within 15, 10, 5, 3,2 or 1% of the average lifespan of a wild-type organism of that species. In a preferred embodiment, the organisms are adult organisms, e.g. the organisms have lived for at least an amount of time in which the average wild-type organism has matured to an age at which it is competent to reproduce.

In some embodiments, the organismal screening assay is performed before the organisms exhibit overt physical features of aging. For example, the organisms may be adults that have lived only 10, 30, 40, 50, 60, or 70% of the average lifespan of a wild-type organism of the same species.

Age-associated changes in metabolism, immune competence, and chromosomal structure have been reported. Any of these changes can be evaluated, either in a test subject (e.g., for an organism based assay), or for a patient (e.g., prior, during or after treatment with a therapeutic described herein.

In another embodiment, a marker associated with caloric restriction is evaluated in a subject organism of a screening assay (or a treated subject). Although these markers may not be age-associated, they may be indicative of a physiological state that is altered when the AMPK pathway is modulated. The marker can be an mRNA or protein whose abundance changes in calorically restricted animals. WO 01/12851 and US 6,406,853 describe exemplary markers.

In a related aspect, the invention features a method of evaluating a test compound using a plurality of biomarkers. This can be done by profiling the sample. The method includes providing a cell or organism and a test compound; contacting the test compound to the cell; obtaining a subject expression profile for the contacted cell; and comparing the subject expression profile to one or more reference profiles. The profiles include a value representing the level of expression of molecules previously determined to be correlated with AMPK pathway activity (see, e.g., below). In a preferred embodiment, the subject expression profile is compared to a target profile, e.g., a profile for a normal cell or for desired condition of a cell. The test compound is evaluated favorably if the subject expression profile is more similar to the target profile than an expression profile obtained from an uncontacted cell.

In one embodiment, expression of a gene regulated by a nuclear protein that is regulated by the AMPK pathway is evaluated. For example, the gene may be directly regulated by a phosphorylated transcription factor such as HNF4$\alpha$, ChREBP or a transcriptional coactivator, such as p300. It is possible to evaluate the activity of a compound on the AMPK pathway by evaluating expression of a gene regulated by one of these factors. For example, Naiki et al. J Biol Chem. 2002 Apr 19;277(16):14011-9 describes genes regulated by HNF4$\alpha$. Uyeda et al. Biochem Pharmacol. 2002 Jun 15; 63(12):2075-80. describes genes regulated by ChREBP. Genes that are regulated by p300 are also known.

Genes whose activity is affected by the phosphorylation state of a histone (e.g., histone H3) can also be evaluated.

Similarity of profiles can be determined by a variety of metric, including Euclidean distance in a n-dimensional space, where n is the number of different values within the profile. Other metrics, for example, include weighting factors that basis different values according to their importance for the comparison.

Profiles, e.g., profiles obtained from nucleic acid array or protein arrays can be used to compare samples and/or cells in a variety of states as described in Golub et al. ((1999) Science 286:531). In one embodiment, multiple expression profiles from different conditions and including replicates or like samples from similar conditions are compared to identify nucleic acids whose expression level is predictive of the sample and/or condition. Each candidate nucleic acid can be given a weighted "voting" factor dependent on the degree of correlation of the nucleic acid's expression and the sample identity. A correlation can be measured using a Euclidean distance or the Pearson correlation coefficient.

**Structure-Activity Relationships and Structure-Based Design.** It is also possible to use structure-activity relationships (SAR) and structure-based design principles to find compounds that have improved effects on AMPK pathway activity. SARs provide information about the activity of related compounds in at least one relevant assay. Correlations are made between structural features of a compound of interest and an activity. For example, it may be possible by evaluating SARs for a family of compounds that activate AMPK (e.g., metformin, a thiazolidinedione, or AICAR) to identify one or more structural features required for activity. A library of compounds can then be produced that vary these features, and then the library is screened. Structure-based design can include determining a structural model of the physical interaction of AMPK activator and its target. The structural model can indicate how an antagonist of the target can be engineered.

Both the SAR and the structure-based design approach can be used to identify a pharmacophore. Pharmacophores are a highly valuable and useful concept in drug discovery and drug-lead optimization. A pharmacophore is defined as a distinct three dimensional (3D) arrangement of chemical groups essential for biological activity. Since a pharmaceutically active molecule must interact with one or more molecular structures within the body of the subject in order to be effective, and the desired functional properties of the molecule are derived from these interactions, each active compound must contain a distinct arrangement of chemical groups which enable this interaction to occur. The chemical groups, commonly termed descriptor centers, can be represented by (a) an atom or group of atoms; (b) pseudo-atoms, for example a center of a ring, or the center of mass of a molecule; (c) vectors, for example atomic pairs, electron lone pair directions, or the normal to a plane. Once formulated a pharmacophore can be used to search a database of chemical compound, e.g., for those having a structure compatible with the pharmacophore. See, for example, U.S. 6,343,257 ; Y. C. Martin, 3D Database Searching in Drug Design, J. Med. Chem. 35, 2145(1992); and A. C. Good and J. S. Mason, Three Dimensional Structure Database Searches, Reviews in Comp. Chem. 7, 67(1996). Database search queries are based not only on chemical property information but also on precise geometric information.

Computer-based approaches can use database searching to find matching templates; Y. C. Martin, Database searching in drug design, J. Medicinal Chemistry, vol. 35, pp 2145-54 (1992). Existing methods for searching 2-D and 3-D databases of compounds are applicable. Lederle of American Cyanamid (Pearl River, N.Y.) has pioneered molecular shape-searching, 3D searching and trend-vectors of databases. Commercial vendors and other research groups also provide searching capabilities (MACSS-3D, Molecular Design Ltd. (San Leandro, Calif.); CAVEAT, Lauri, G. et al.,

University of California (Berkeley, Calif.); CHEM-X, Chemical Design, Inc. (Mahwah, N.J.)). Software for these searches can be used to analyze databases of potential drug compounds indexed by their significant chemical and geometric structure (e.g., the Standard Drugs File (Derwent Publications Ltd., London, England), the Bielstein database (Bielstein Information, Frankfurt, Germany or Chicago), and the Chemical Registry database (CAS, Columbus, Ohio)).

Once a compound is identified that matches the pharmacophore, it can be tested for activity, e.g., for binding to a component of AMPK pathway and/or for a biological activity, e.g., modulation of AMPK activity, e.g., AMPK activation. See, e.g., "Screening Methods".

Combinatorial Systems

The organisms described herein may be deficient in the activity of any protein that is associated with aging, e.g., associated with the regulation of lifespan. Some exemplary genes and homologs of genes which encode proteins that are associated with the regulation of lifespan are listed in Table 2. For example, mutant or otherwise altered (e.g., RNAi treated or transgenic) organisms that are altered for an AMPK pathway component activity can also include an alteration in a component that is listed in Table 2 or that directly interacts with a component in Table 2.

Other types of combinatorial systems include environmental treatment of an organism that is mutant or otherwise altered (e.g., RNAi treated or transgenic) with respect to an AMPK pathway component activity. Exemplary environmental treatments include stress (e.g., oxidative stress, genotoxic stress, $H_2O_2$, heavy metal exposure), caloric restriction, and treatment with a drug, e.g., a histone deacetylase inhibitor.

**Table 2: Exemplary Age-associated Components**

| Organism | Gene name | Description | Exemplary homologs |
|---|---|---|---|
| *S. cerevisiae* | SIR2 | NAD-dependent deacetylase | Murine Sir2alpha (GenBank AccNo: AF214646), human SIRT1 (GenBank Acc No: AF083106) human Sir2 SIRT3 GenBank Accession No: AF083108; human Sir2 SIRT4 GenBank Accession No: AF083109; human Sir2 SIRTS GenBank Accession No: AF083110 |
| *S. cerevisiae* | SIR3 | Regulator of chromatin silencing | |
| *S. cerevisiae* | SIR4 | Regulator of chromatin silencing | |
| *S. cerevisiae* | RPD3 | Histone deacetylase | |
| *S. cerevisiae* | FOB1 | Suppresses rDNA replication | |
| *S. cerevisiae* | SGS1 | Wemers-like DNA helicase | |
| *S. cerevisiae* | SNF1 | Kinase involved in carbon source utilization | |
| *S. cerevisiae* | SIP2 | SNF1 co-repressor | |
| *S. cerevisiae* | SNF4 | SNF1 co-activator | |
| *S. cerevisiae* | NPTI | Involved in NAD synthesis | |
| *S. cerevisiae* | RTG2 | Sensor of mitochondrial disfunction | |
| *S. cerevisiae* | Coq7 | Regulator of ubiquinone synthesis | |
| *C. elegans* | Daf-2 | Insulin/IGF-1 receptor homolog | insulin or IGF receptor |
| *C. elegans* | Age-1 | PI(3) kinase | PI(3) kinase |
| *C. elegans* | Pdk-1 | | PDK-1 |
| *C. elegans* | Daf-18 | Phosphatase | PTEN |
| *C. elegans* | Daf-16 | Forkhead/winged-helix family transcription factor | AFX,FKHR,FKHRL1 |
| *C. elegans* | Ceinsulin-1 | Insulin/IGF-1-like homolog | insulin or IGF molecules |
| *C. elegans* | Ctl-1 | Cytosolic catalase | |

(continued)

| Organism | Gene name | Description | Exemplary homologs |
|---|---|---|---|
| *C. elegans* | MEV-1 | Cytochrome B subunit of mitochondrial succinate dehydrogenase | Cytochrome B subunit of mitochondrial succinate dehydrogenase |
| *C. elegans* | Sod-3 | Mn-superoxide dismutase | superoxide dismutase |
| *C. elegans* | Clk-1 | Regulator of ubiquinone synthesis | |
| *C. elegans* | Tkr-1 | Tyrosine kinase | |
| *C. elegans* | Spe-10 | Unknown (sperm defective) | |
| *C. elegans* | Spe-26 | Unknown (sperm defective) | |
| *C. elegans* | Old-1 | Receptor tyrosine kinase | |
| *C. elegans* | Kin-29 | Serine Threonine Kinase | |
| *Drosophila* | Indy<br><br>GenBank accession no. AE003519 | Carboxylate transporter | hNaDC-1, accession No. U26209, SDCT2, accession no. AF081825, NaDC-1, accession no. U12186, mNaDC-1, accession no. AF 201903, human solute carrier family 13, member 2 GenBank NP_003975.1, human sodium-dependent high-affinity dicarboxylate transporter 3, human carrier family 13 (sodium/sulfate symporters), member 1, human hypothetical protein XP_091606, human carrier family 13 (sodium/sulfate symporters) member 4 (GenBank NP_036582), |
| *Drosophila* | Cu/Zn-SOD | superoxide dismutase | |
| *Drosophila* | Methuselah | Putative G-protein-coupled 7 transmembrane domain receptor | |
| *Mus musculus* | p66shc | Signaling adaptor | |
| *Mus musculus* | PROP1 | Homeodomain protein | |
| *Mus musculus* | Growth hormone | | |
| *Mus musculus* | Growth hormone Releasing hormone receptor | | |

RNAi

It is also possible to regulate AMPK pathway activity using a double-stranded RNA (dsRNA) that mediates RNA interference (RNAi). The dsRNA can be delivered to cells or to an organism. Endogenous components of the cell or organism can trigger RNA interference (RNAi) which silences expression of genes that include the target sequence. dsRNA can be produced by transcribing a cassette in both directions, for example, by including a T7 promoter on either side of the cassette. The insert in the cassette is selected so that it includes a sequence complementary to a nucleic acid encoding an AMPK pathway component. The sequence need not be full length, for example, an exon, or at least 50 nucleotides. The sequence can be from the 5' half of the transcript, e.g., within 1000, 600, 400, or 300 nucleotides of the ATG. See also, the HiScribe™ RNAi Transcription Kit (New England Biolabs, MA) and Fire, A. (1999) Trends Genet. 15, 358-363. dsRNA can be digested into smaller fragments. See, e.g., US Patent Application 2002-0086356 and 2003-0084471. In one embodiment, an siRNA is used. siRNAs are small double stranded RNAs (dsRNAs) that optionally include overhangs. For example, the duplex region is about 18 to 25 nucleotides in length, e.g., about 19, 20, 21, 22, 23, or 24 nucleotides in length. Typically the siRNA sequences are exactly complementary to the target mRNA.

dsRNAs (and siRNA's in particular) can be used to silence gene expression in mammalian cells. See, e.g., Clemens, J. C. et al. (2000) Proc. Natl. Sci. USA 97, 6499-6503; Billy et al. (2001) Proc. Natl. Sci. USA 98, 14428-14433; Elbashir et al. (2001) Nature. 411 (683 6):494-8; Yang, D. et al. (2002) Proc. Natl. Acad. Sci. USA 99, 9942-9947.

For example, dsRNA molecules complementary to a nucleic acid encoding protein phosphatase 2C can be used to enhance activity of the AMPK pathway. Such molecules can be used to contact cells ex vivo or *in vivo* to regulate the AMPK pathway in those cells. dsRNA molecules can thus be used to reduce the activity of inhibitors of the AMPK pathway to increase AMPK activity.

dsRNA molecules can be used to provide cells and organisms (e.g., mammalian cells and organisms, and nematode mammalian cells and organisms) that are deficient in an AMPK activity, e.g., the AMPK alpha subunit, or AMPKK.. Such cells and organisms are useful tools for evaluating heterologous AMPK molecules and test compounds for activity, e.g., an activity that modulates lifespan regulation.

Artificial Transcription Factors

Artificial transcription factors can also be used to regulate genes that are regulated by an AMPK pathway component (e.g., AMPK) or to regulate genes that encode an AMPK pathway component (e.g., AMPK). The artificial transcription factor can be designed or selected from a library. The artificial transcription factor can include zinc finger domains. The artificial transcription factor can be prepared by selection in vitro (e.g., using phage display, U.S. 6,534,261) or in vivo, or by design based on a recognition code (see, e.g., WO 00/42219 and U.S. 6,511,808). See, e.g., Rebar et al. (1996) Methods Enzymol 267:129; Greisman and Pabo (1997) Science 275:657; Isalan et al. (2001) Nat. Biotechnol 19:656; and Wu et al. (1995) Proc. Nat. Acad. Sci. USA 92:344 for, among other things, methods for creating libraries of varied zinc finger domains.

Optionally, the zinc finger protein can be fused to a transcriptional regulatory domain, e.g., an activation domain to activate transcription or a repression domain to repress transcription. The zinc finger protein can itself be encoded by a heterologous nucleic acid that is delivered to a cell or the protein itself can be delivered to a cell (see, e.g., U.S. 6,534,261. The heterologous nucleic acid that includes a sequence encoding the zinc finger protein can be operably linked to an inducible promoter, e.g., to enable fine control of the level of the zinc finger protein in the cell.

Stem Cell Therapy

It is also possible to modify cells, e.g., stem cells, using nucleic acid recombination, e.g., to insert a transgene, e.g., a transgene encoding a polypeptide or polypeptides that increases AMPK pathway activity, e.g., a constitutively activated AMPK alpha subunit or an AMPK alpha subunit that is unable to interact with the beta or gamma subunit. The modified stem cell can be administered to a subject. Methods for cultivating stem cells in vitro are described, e.g., in US Application 2002-0081724. In some examples, the stem cells can be induced to differentiate in the subject and express the transgene. For example, the stem cells can be differentiated into liver, adipose, or skeletal muscle cells. The stem cells can be derived from a lineage that produces cells of the desired tissue type, e.g., liver, adipose, or skeletal muscle cells.

AMPK Pathway Nucleic Acids, Proteins and Vectors

Method described herein can include use of routine techniques in the field of molecular biology, biochemistry, classical genetics, and recombinant genetics. Basic texts disclosing the general methods of use in this invention include Sambrook & Russell, Molecular Cloning: A Laboratory Manual, 3rd Edition, Cold Spring Harbor Laboratory, N.Y. (2001); Kriegler, Gene Transfer and Expression: A Laboratory Manual (1990); and Current Protocols in Molecular Biology (Ausubel et al., eds., 1994)).

Nucleic acids, polymorphic variants, orthologs, and alleles that encode AMPK pathway components can be isolated, e.g., by screening libraries, by analyzing a sequence database, and/or by synthetic gene construction. For example, expression libraries can be used to screen such nucleic acids, e.g., by detecting expressed homologs immunologically with antisera or purified antibodies made against *C. elegans* or mammalian AMPK pathway components or by complementation, e.g., of a *C. elegans* phenotype, e.g., a *C. elegans* that is deficient for a AMPK activity, e.g., deficient for a T01C8.1 activity.

To make a cDNA library, one can choose a source that is rich in the RNA of choice. The mRNA is then made into cDNA using reverse transcriptase, ligated into a recombinant vector, and transfixed into a recombinant host for propagation, screening and cloning. Methods for making and screening cDNA libraries are well known *(see,* e.g., Gubler & Hoffman, Gene 25:263-269 (1983); Sambrook *et al., supra;* Ausubel *et al., supra*).

For a genomic library, the DNA is extracted from the tissue and either mechanically sheared or enzymatically digested to yield fragments of about 12-20 kb. The fragments are then separated by gradient centrifugation from undesired sizes and are constructed in bacteriophage lambda vectors. These vectors and phage are packaged *in vitro.* Recombinant phage are analyzed by plaque hybridization as described in Benton & Davis, Science 196:180-182 (1977). Colony hybridization is carried out as generally described in Grunstein et al., Proc. Natl. Acad. Sci. USA., 72:3961-3965 (1975).

An alternative method for identifying AMPK pathway component-encoding nucleic acid and their orthologs, alleles, mutants, polymorphic variants, and conservatively modified variants combines the use of synthetic oligonucleotide primers and amplification of an RNA or DNA template (*see* U.S. 4,683,195 and 4,683,202; PCR Protocols: A Guide to Methods and Applications (Innis et al., eds, 1990)). Methods such as polymerase chain reaction (PCR) and ligase chain reaction (LCR) can be used to amplify nucleic acid sequences directly from mRNA, from cDNA, from genomic libraries or cDNA libraries. Degenerate oligonucleotides can be designed to amplify homologs using the sequences provided herein. Restriction endonuclease sites can be incorporated into the primers. Polymerase chain reaction or other *in vitro* amplification methods may also be useful, for example, to clone nucleic acid sequences that code for proteins to be expressed, to make nucleic acids to use as probes for detecting the presence of AMPK pathway component encoding mRNA in physiological samples, for nucleic acid sequencing, or for other purposes. Genes amplified by the PCR reaction can be purified from agarose gels and cloned into an appropriate vector.

Another method for identifying AMPK pathway components uses a computer database search. A variety of tools can be used to evaluate sequence information in a database such as GenBank® or SWISSPROT. For example an amino acid sequence can be used to query a database using an alignment processor such as BLAST. In another example, a profile is used to evaluate a database. An exemplary profiling method is Pfam, which uses Hidden Markov Models (HMM) to scan amino acid sequences. To identify the presence of a domain in a protein sequence, and make the determination that a polypeptide or protein of interest has a particular profile, the amino acid sequence of the protein can be searched against the Pfam database of HMMs (e.g., the Pfam database, release 9) using the default parameters. Profiles for various protein families are indexed in the Pfam database and other database, such as INTERPRO. For example, the hmmsf program, which is available as part of the HMMER package of search programs, is a family specific default program for MILPAT0063 and a score of 15 is the default threshold score for determining a hit. Alternatively, the threshold score for determining a hit can be lowered (e.g., to 8 bits).

Gene expression of AMPK pathway components can also be analyzed by techniques known in the art, e.g., reverse transcription and amplification of mRNA, isolation of total RNA or poly A$^+$ RNA, northern blotting, dot blotting, *in situ* hybridization, RNase protection, nucleic acid array technology, e.g., and the like.

The gene for AMPK pathway components can be cloned into vectors before transformation into prokaryotic or eukaryotic cells for replication and/or expression. These vectors are typically prokaryote vectors, e.g., plasmids, phage or shuttle vectors, or eukaryotic vectors.

**Protein Expression.** To obtain recombinant expression (e.g., high level) expression of a cloned gene, such as those cDNAs encoding AMPK pathway components, one typically subclones the relevant coding nucleic acids into an expression vector that contains a strong promoter to direct transcription, a transcription/translation terminator, and a ribosome binding site for translational initiation. Suitable bacterial promoters are well known in the art and described, e.g., in Sambrook *et al.,* and Ausubel *et al, supra.* Bacterial expression systems for expression are available in, e.g., *E. coli, Bacillus sp.,* and *Salmonella* (Palva et al., Gene 22:229-235 (1983); Mosbach et al., Nature 302:543-545 (1983). Kits for such expression systems are commercially available. Eukaryotic expression systems for mammalian cells, yeast (e.g., *S. cerevisiae, S. pombe, Pichia,* and *Hanseula),* and insect cells are well known in the art and are also commercially available.

Selection of the promoter used to direct expression of a heterologous nucleic acid depends on the particular application. The promoter is preferably positioned about the same distance from the heterologous transcription start site as it is from the transcription start site in its natural setting. As is known in the art, however, some variation in this distance can be accommodated without loss of promoter function.

In addition to the promoter, the expression vector typically contains a transcription unit or expression cassette that contains all the additional elements required for expression in host cells. A typical expression cassette thus contains a promoter operably linked to the coding nucleic acid sequence and signals required for efficient polyadenylation of the transcript, ribosome binding sites, and translation termination. Additional elements of the cassette may include enhancers and, if genomic DNA is used as the structural gene, introns with functional splice donor and acceptor sites.

In addition to a promoter sequence, the expression cassette should also contain a transcription termination region downstream of the structural gene to provide for efficient termination. The termination region may be obtained from the same gene as the promoter sequence or may be obtained from different genes.

The particular expression vector used to transport the genetic information into the cell is not particularly critical. Any of the conventional vectors used for expression in eukaryotic or prokaryotic cells may be used. Standard bacterial expression vectors include plasmids such as pBR322 based plasmids, pSKF, pET23D, and fusion expression systems such as MBP, GST, and LacZ. Epitope tags can also be added to recombinant proteins to provide convenient methods of isolation, e.g., c-myc-, or a hexa-histidine tag.

Expression vectors containing regulatory elements from eukaryotic viruses are typically used in eukaryotic expression vectors, e.g., SV40 vectors, papilloma virus vectors, and vectors derived from Epstein-Barr virus. Other exemplary eukaryotic vectors include pMSG, pAV009/A$^+$, pMTO10/A$^+$, pMAMneo-5, baculovirus pDSVE, and any other vector allowing expression of proteins under the direction of the CMV promoter, SV40 early promoter, SV40 later promoter, metallothionein promoter, murine mammary tumor virus promoter, Rous sarcoma virus promoter, polyhedrin promoter, or other promoters shown effective for expression in eukaryotic cells.

Expression of proteins from eukaryotic vectors can be also be regulated using inducible promoters. With inducible promoters, expression levels are tied to the concentration of inducing agents, such as tetracycline or ecdysone, by the incorporation of response elements for these agents into the promoter. Generally, high level expression is obtained from inducible promoters only in the presence of the inducing agent; basal expression levels are minimal. Inducible expression vectors are often chosen if expression of the protein of interest is detrimental to eukaryotic cells.

Some expression systems have markers that provide gene amplification such as thymidine kinase and dihydrofolate reductase. Alternatively, high yield expression systems not involving gene amplification are also suitable, such as using a baculovirus vector in insect cells, with a nucleic acid sequence encoding an AMPK pathway component, e.g., an AMPK subunit, under the direction of the polyhedrin promoter or other strong baculovirus promoters. For example, baculovirus vectors can be prepared for each of the three AMPK subunits, alpha, beta, and gamma. Viruses prepared from such vectors can be used to co-infect an insect cell to produce an AMPK complex.

The elements that are typically included in expression vectors also include a replicon that functions in *E. coli,* a gene encoding antibiotic resistance to permit selection of bacteria that harbor recombinant plasmids, and unique restriction sites in nonessential regions of the plasmid to allow insertion of eukaryotic sequences. The prokaryotic sequences can be chosen such that they do not interfere with the replication of the DNA in eukaryotic cells.

Standard transfection methods are used to produce bacterial, mammalian, yeast or insect cell lines that express large quantities of AMPK pathway components, which are then purified using standard techniques *(see, e.g.,* Colley et al., J. Biol. Chem. 264:17619-17622 (1989); Guide to Protein Purification, in Methods in Enzymology, vol. 182 (Deutscher, ed., 1990)). Transformation of eukaryotic and prokaryotic cells are performed according to standard techniques (*see, e.g.,* Morrison, J. Bact. 132:349-351 (1977); Clark-Curtiss & Curtiss, Methods in Enzymology 101:347-362 (Wu et al., eds, 1983).

Any of the well-known procedures for introducing foreign nucleotide sequences into host cells may be used. These include the use of calcium phosphate transfection, protoplast fusion, electroporation, liposomes, microinjection, plasma vectors, viral vectors and any of the other well known methods for introducing cloned genomic DNA, cDNA, synthetic DNA or other foreign genetic material into a host cell *(see, e.g.,* Sambrook *et al., supra*). It is only necessary that the particular genetic engineering procedure used be capable of successfully introducing at least one gene into the host cell.

After the expression vector is introduced into the cells, the transfected cells are cultured under conditions favoring expression or activating expression. The protein can then be isolated from a cell extract, cell membrane component or vesicle, or media.

Expression vectors with appropriate regulatory sequences can also be used to express a heterologous gene in a nematode. In one example, the expression vector is injected in the gonad of the nematode, and the vector is incorporated, e.g., as an extra-chromosomal array in progeny of the nematode. The vector can further include a second gene (e.g., a marker gene) that indicates the presence of the vector, e.g., the *rol-6* marker. For example, the heterologous gene can be a mammalian gene, e.g., a mammalian cDNA, or a fragment thereof. See, generally, Riddle et al., eds., C. elegans II. Plainview (NY): Cold Spring Harbor Laboratory Press; 1997.

**Protein Purification.** Either naturally occurring or recombinant AMPK pathway components can be purified for use in functional assays. Naturally occurring AMPK pathway components can be purified, e.g., from tissue samples, e.g., human, murine, or bovine tissue. Recombinant AMPK pathway components can be purified from any suitable expression system, e.g., those described above.

AMPK pathway components may be purified to substantial purity by standard techniques, including selective precipitation with such substances as ammonium sulfate; column chromatography, affinity purification, immunopurification methods, and others *(see, e.g.,* Scopes, Protein Purification: Principles and Practice (1982); U.S. 4,673,641; Ausubel *et al., supra;* and Sambrook *et al., supra*). For example, recombinant AMPK pathway components can include an affinity tag that can be used for purification, e.g., in combination with other steps. For example, Crute et al. (1998) J. Biol. Chem. 273:35347-35354 describe use of a glutathione-S-transferase N-terminal tag to purify recombinant AMPK proteins.

Recombinant proteins are expressed by transformed bacteria in large amounts, typically after promoter induction; but expression can be constitutive. Promoter induction with IPTG is one example of an inducible promoter system. Bacteria are grown according to standard procedures in the art. Fresh or frozen bacteria cells are used for isolation of protein. Proteins expressed in bacteria may form insoluble aggregates ("inclusion bodies"). Several protocols are suitable for purifying proteins from inclusion bodies. *See, e.g.,* Sambrook *et al., supra;* Ausubel *et al., supra*). If the proteins are soluble or exported to the periplasm, they can be obtained from cell lysates or periplasmic preparations.

Differential Precipitation. Salting-in or out can be used to selectively precipitate an AMPK pathway component or a contaminating protein. An exemplary salt is ammonium sulfate. Ammonium sulfate precipitates proteins on the basis of their solubility. The more hydrophobic a protein is, the more likely it is to precipitate at lower ammonium sulfate concentrations. A typical protocol includes adding saturated ammonium sulfate to a protein solution so that the resultant ammonium sulfate concentration is between 20-30%. This concentration precipitates many of the more hydrophobic proteins. The precipitate is analyzed to determine if the protein of interest is precipitated or in the supernatant. Ammonium sulfate is added to the supernatant to a concentration known to precipitate the protein of interest. The precipitate is then solubilized in buffer and the excess salt removed if necessary, either through dialysis or diafiltration.

Column chromatography. AMPK pathway components can be separated from other proteins on the basis of its size, net surface charge, hydrophobicity, and affinity for ligands. In addition, antibodies raised against proteins can be conjugated to column matrices and the proteins immunopurified. All of these methods are well known in the art. It will be apparent to one of skill that chromatographic techniques can be performed at any scale and using equipment from many different manufacturers (e.g., Pharmacia Biotech). See, generally, Scopes, Protein Purification: Principles and Practice (1982).

Affinity purification can be used to purify AMPK. For example, a substrate peptide for AMPK can be used as an affinity reagent to purify AMPK from an extract.

Antibodies to AMPK Pathway Components

Methods of producing polyclonal and monoclonal antibodies that react specifically with the AMPK pathway components are known to those of skill in the art (*see, e.g.,* Coligan, Current Protocols in Immunology (1991); Harlow & Lane, *supra;* Goding, Monoclonal Antibodies: Principles and Practice (2d ed. 1986); and Kohler & Milstein, Nature 256:495-497 (1975). Such techniques include antibody preparation by selection of antibodies from libraries of recombinant antibodies in phage or similar vectors, as well as preparation of polyclonal and monoclonal antibodies by immunizing rabbits or mice *(see, e.g.,* Huse et al., Science 246:1275-1281 (1989); Ward et al., Nature 341:544-546 (1989)).

A number of immunogens comprising portions of AMPK pathway components may be used to produce antibodies specifically reactive with an AMPK pathway component. For example, recombinant protein or an antigenic fragment thereof, can be isolated as described herein. Recombinant protein can be expressed in eukaryotic or prokaryotic cells as described above, and purified as generally described above. Recombinant protein is the preferred immunogen for the production of monoclonal or polyclonal antibodies. Alternatively, a synthetic peptide derived from the sequences disclosed herein and conjugated to a carrier protein can be used an immunogen. Naturally occurring protein may also be used either in pure or impure form. The product is then injected into an animal capable of producing antibodies. Either monoclonal or polyclonal antibodies may be generated, for subsequent use in immunoassays to measure the protein.

Methods of production of polyclonal antibodies are known to those of skill in the art. An inbred strain of mice (e.g., BALB/C mice) or rabbits is immunized with the protein using a standard adjuvant, such as Freund's adjuvant, and a standard immunization protocol. The animal's immune response to the immunogen preparation is monitored by taking test bleeds and determining the titer of reactivity to the beta subunits. When appropriately high titers of antibody to the immunogen are obtained, blood is collected from the animal and antisera are prepared. Further fractionation of the antisera to enrich for antibodies reactive to the protein can be done if desired (*see,* Harlow & Lane, *supra*).

Monoclonal antibodies may be obtained by various techniques familiar to those skilled in the art. Briefly, spleen cells from an animal immunized with a desired antigen are immortalized, commonly by fusion with a myeloma cell *(see,* Kohler & Milstein, Eur. J. Immunol. 6:511-519 (1976)). Alternative methods of immortalization include transformation with Epstein Barr Virus, oncogenes, or retroviruses, or other methods well known in the art. Colonies arising from single immortalized cells are screened for production of antibodies of the desired specificity and affinity for the antigen, and yield of the monoclonal antibodies produced by such cells may be enhanced by various techniques, including injection into the peritoneal cavity of a vertebrate host. Alternatively, one may isolate DNA sequences which encode a monoclonal antibody or a binding fragment thereof by screening a DNA library from human B cells according to the general protocol outlined by Huse, et al., Science 246:1275-1281 (1989).

Monoclonal antibodies and polyclonal sera are collected and titered against the immunogen protein in an immunoassay, for example, a solid phase immunoassay with the immunogen immobilized on a solid support. Typically, polyclonal antisera with a titer of $10^4$ or greater are selected and tested for their cross reactivity against nonspecific proteins, using a competitive binding immunoassay. Specific polyclonal antisera and monoclonal antibodies will usually bind with a $K_d$ of at least about 0.1 mM, more usually at least about 1 $\mu$M, preferably at least about 0.1 $\mu$M or better, and most preferably, 0.01 $\mu$M or better. Antibodies specific only for a particular ortholog, such as a human ortholog, can also be made, by subtracting out other cross-reacting orthologs from a species such as a non-human mammal.

Once the specific antibodies against AMPK pathway components are available, the protein can be detected by a variety of immunoassay methods. In addition, the antibody can be used therapeutically, e.g., to modulate AMPK pathway activity. For a review of immunological and immunoassay procedures, see Basic and Clinical Immunology (Stites & Terr eds., 7th ed. 1991). Moreover, the immunoassays of the present invention can be performed in any of several configurations, which are reviewed extensively in Enzyme Immunoassay (Maggio, ed., 1980); and Harlow & Lane, *supra.*

It is also possible to use protein arrays to detect an AMPK pathway component, e.g., to concurrently detect a plurality of AMPK pathway components. Exemplary methods for producing protein arrays are provided in De Wildt et al. (2000) Nat. Biotechnol. 18:989-994; Lueking et al. (1999) Anal. Biochem. 270:103-111; Ge (2000) Nucleic Acids Res. 28, e3, I-VII; MacBeath and Schreiber (2000) Science 289:1760-1763; WO 0/98534, WO01/83827, WO02/12893, WO 00/63701, WO 01/40803 and WO 99/51773. In some implementations, polypeptides (including peptides) are spotted onto discrete addresses of the array, e.g., at high speed, e.g., using commercially available robotic apparati, e.g., from Genetic MicroSystems or BioRobotics. The array substrate can be, for example, nitrocellulose, plastic, glass, e.g., surface-modified glass. The array can also include a porous matrix, e.g., acrylamide, agarose, or another polymer.

Genetic Information

Genetic information about an AMPK pathway component, particularly a human AMPK pathway component, can be obtained, e.g., by evaluating genetic material (e.g., DNA orRNA) from a subject (e.g., as described below). Genetic information refers to any indication about nucleic acid sequence content at one or more nucleotides. Genetic information can include, for example, an indication about the presence or absence of a particular polymorphism, e.g., one or more nucleotide variations. Exemplary polymorphisms include a single nucleotide polymorphism (SNP), a restriction site or restriction fragment length, an insertion, an inversion, a deletion, a repeat (e.g., trinucleotide repeat, a retroviral repeat), and so forth.

For example, Stapleton et al. FEBS Lett. 1997 Jun 16;409(3):452-6 describes the chromosomal location of genes encoding some human AMPK subunit as follows AMPK-alphal (5p11-p14), AMPK-betal (12q24.1-24.3) and AMPK-gammal (12q12-q14). For example, the AMPK-alphal gene can include the region defined by 40,767K-40,779K bp of human chromosome 5. Exemplary SNPs in these regions are available from public SNP database.

Exemplary SNPs for AMPK alpha 1 subunit gene on human chromosome 5 in the region of 40759622 to 40798617:

| start | stop | dbSNP rs# | local loci |
|---|---|---|---|
| 40759174 | 40759174 | rs29741 | PRKAA1:locus; |
| 40759267 | 40759267 | rs29740 | PRKAA1:locus; |
| 40760955 | 40760955 | rs1053118 | PRKAA1:locus; |
| 40760961 | 40760961 | rs1053117 | PRKAA1:locus; |
| 40760971 | 40760971 | rs1053116 | PRKAA1:locus; |
| 40763016 | 40763016 | rs1044254 | PRKAA1; |
| 40763018 | 40763018 | rs1044253 | PRKAA1; |
| 40763897 | 40763897 | rs29737 | PRKAA1:intron; |
| 40764270 | 40764270 | rs4957350 | PRKAA1:intron; |
| 40765785 | 40765785 | rs4263541 | PRKAA1:intron; |
| 40765862 | 40765862 | rs249428 | PRKAA1:intron; |
| 40767538 | 40767538 | rs1002424 | PRKAA1:intron; |
| 40767599 | 40767599 | rs1002423 | PRKAA1:intron; |
| 40767879 | 40767879 | rs1801924 | PRKAA1; |
| 40768102 | 40768103 | rs3840323 | PRKAA1:intron; |
| 40770144 | 40770144 | rs3071208 | PRKAA1:intron; |
| 40770356 | 40770361 | rs1610943 | PRKAA1:intron; |
| 40770367 | 40770367 | rs1645491 | PRKAA1:intron; |
| 40770541 | 40770541 | rs193864 | PRKAA1:intron; |
| 40771357 | 40771357 | rs257009 | PRKAA1:intron; |
| 40772212 | 40772212 | rs837103 | PRKAA1:intron; |
| 40772978 | 40772978 | rs837102 | PRKAA1:intron; |
| 40773596 | 40773599 | rs3071898 | PRKAA1:intron; |
| 40773598 | 40773599 | rs3071898 | PRKAA1:intron; |
| 40773600 | 40773600 | rs2876823 | PRKAA1:intron; |
| 40773694 | 40773694 | rs463179 | PRKAA1:intron; |
| 40773840 | 40773840 | rs837101 | PRKAA1:intron; |
| 40774095 | 40774095 | rs185381 | PRKAA1:intron; |
| 40774718 | 40774718 | rs1692243 | PRKAA1:intron; |

(continued)

| start | stop | dbSNP rs# | local loci |
|---|---|---|---|
| 40774933 | 40774933 | rs1057505 | PRKAA1:intron; |
| 40775438 | 40775438 | rs3792822 | PRKAA1:intron; |
| 40777391 | 40777391 | rs837100 | PRKAA1:intron; |
| 40777572 | 40777572 | rs865664 | PRKAA1:intron; |
| 40778782 | 40778782 | rs154275 | PRKAA1:intron; |
| 40779371 | 40779372 | rs3834834 | PRKAA1:intron; |
| 40779832 | 40779832 | rs154276 | PRKAA1:intron; |
| 40781780 | 40781780 | rs154277 | PRKAA1:intron; |
| 40782380 | 40782380 | rs249429 | PRKAA1:intron; |
| 40783055 | 40783055 | rs3805494 | PRKAA1:intron; |
| 40783290 | 40783290 | rs154278 | PRKAA1:intron; |
| 40783404 | 40783404 | rs3805492 | PRKAA1:intron; |
| 40783422 | 40783422 | rs154279 | PRKAA1:intron; |
| 40783558 | 40783558 | rs154280 | PRKAA1:intron; |
| 40783981 | 40783981 | rs154281 | PRKAA1:intron; |
| 40784514 | 40784514 | rs837267 | PRKAA1:intron; |
| 40786324 | 40786324 | rs171606 | PRKAA1:intron; |
| 40787611 | 40787611 | rs4957351 | PRKAA1:intron; |
| 40787633 | 40787633 | rs152372 | PRKAA1:intron; |
| 40787664 | 40787664 | rs4957352 | PRKAA1:intron; |
| 40787722 | 40787722 | rs152373 | PRKAA1:intron; |

| start | stop | dbSNP rs# | local loci |
|---|---|---|---|
| 40787830 | 40787830 | rs154282 | PRKAA1:intron; |
| 40788074 | 40788074 | rs154283 | PRKAA1:intron; |
| 40788715 | 40788715 | rs154284 | PRKAA1:intron; |
| 40790268 | 40790268 | rs3833649 | PRKAA1:intron; |
| 40790770 | 40790770 | rs4957353 | PRKAA1:intron; |
| 40792294 | 40792294 | rs3805490 | PRKAA1:intron; |
| 40792347 | 40792347 | rs152374 | PRKAA1:intron; |
| 40793299 | 40793299 | rs249430 | PRKAA1:intron; |
| 40793485 | 40793485 | rs3805489 | PRKAA1:intron; |
| 40796009 | 40796009 | rs154268 | PRKAA1:intron; |
| 40796174 | 40796174 | rs3805487 | PRKAA1:intron; |
| 40796186 | 40796186 | rs3805486 | PRKAA1:intron; |
| 40796887 | 40796887 | rs466108 | PRKAA1:intron; |
| 40797803 | 40797803 | rs154269 | PRKAA1:intron; |
| 40797952 | 40797952 | rs168731 | PRKAA1:intron; |
| 40798051 | 40798051 | rs1644988 | PRKAA1:intron; |

The gene for the AMPK Beta subunit isoform 1 is on chromosome 12 at positions 119888548 119902275 Exemplary SNPs include:

| start | stop | dbSNP rs# | local loci |
|---|---|---|---|
| 119888766 | 119888766 | rs3182909 | LOC347959:locus;PRKAB1; |
| 119888923 | 119888923 | rs1043350 | LOC347959:locus;PRKAB1; |
| 119888926 | 119888926 | rs1043352 | LOC347959:locus;PRKAB1; |
| 119888947 | 119888947 | rs1062688 | LOC347959:locus;PRKAB1; |
| 119888960 | 119888960 | rs1043354 | LOC347959:locus;PRKAB1; |

(continued)

| start | stop | dbSNP rs# | local loci |
|-------|------|-----------|------------|
| 119891867 | 119891867 | rs278143 | LOC347959:locus;PRKAB1:intron; |
| 119893277 | 119893277 | rs2285593 | LOC347959:locus;PRKAB1:intron; |
| 119893316 | 119893317 | rs3999587 | LOC347959:locus;PRKAB1:intron; |
| 119894026 | 119894026 | rs278144 | LOC347959:locus;PRKAB1:intron; |
| 119894952 | 119894952 | rs278145 | LOC347959:locus;PRKAB1:intron; |
| 119895848 | 119895848 | rs278146 | LOC347959:locus;PRKAB1:intron; |
| 119896296 | 119896296 | rs278147 | LOC347959:locus;PRKAB1:intron; |
| 119896475 | 119896475 | rs278148 | LOC347959:locus;PRKAB1:intron; |
| 119897360 | 119897360 | rs278149 | PRKAB1:intron; |
| 119897803 | 119897803 | rs278150 | PRKAB1:intron; |
| 119899347 | 119899347 | rs278151 | PRKAB1:intron; |
| 119900132 | 119900132 | rs278152 | PRKAB1:intron; |
| 119902036 | 119902036 | rs4213 | PRKAB1; |
| 119902944 | 119902945 | rs2308131 | |

It is possible to digitally record or communicate genetic information in a variety of ways. Typical representations include one or more bits, or a text string. For example, a biallelic marker can be described using two bits. In one embodiment, the first bit indicates whether the first allele (e.g., the minor allele) is present, and the second bit indicates whether the other allele (e.g., the major allele) is present. For markers that are multi-allelic, e.g., where greater than two alleles are possible, additional bits can be used as well as other forms of encoding (e.g., binary, hexadecimal text, e.g., ASCII or Unicode, and so forth). In some embodiments, the genetic information describes a haplotype, e.g., a plurality of polymorphisms on the same chromosome. However, in many embodiments, the genetic information is unphased.

Methods of Evaluating Genetic Material

There are numerous methods for evaluating genetic material to provide genetic information. These methods can be used to evaluate a gene that encodes an AMPK pathway component as well as other loci.

Nucleic acid samples can analyzed using biophysical techniques (e.g., hybridization, electrophoresis, and so forth), sequencing, enzyme-based techniques, and combinations-thereof. For example, hybridization of sample nucleic acids to nucleic acid microarrays can be used to evaluate sequences in an mRNA population and to evaluate genetic polymorphisms. Other hybridization based techniques include sequence specific primer binding (e.g., PCR or LCR); Southern analysis of DNA, e.g., genomic DNA; Northern analysis of RNA, e.g., mRNA; fluorescent probe based techniques (see, e.g., Beaudet et al. (2001) Genome Res. 11(4):600-8); and allele specific amplification. Enzymatic techniques include restriction enzyme digestion; sequencing; and single base extension (SBE). These and other techniques are well known to those skilled in the art.

Electrophoretic techniques include capillary electrophoresis and Single-Strand Conformation Polymorphism (SSCP) detection (see, e.g., Myers et al. (1985) Nature 313:495-8 and Ganguly (2002) Hum Mutat. 19(4):334-42). Other biophysical methods include denaturing high pressure liquid chromatography (DHPLC).

In one embodiment, allele specific amplification technology that depends on selective PCR amplification may be used to obtain genetic information. Oligonucleotides used as primers for specific amplification may carry the mutation of interest in the center of the molecule (so that amplification depends on differential hybridization) (Gibbs et al. (1989) Nucleic Acids Res. 17:2437-2448) or at the extreme 3' end of one primer where, under appropriate conditions, mismatch can prevent, or reduce polymerase extension (Prossner (1993) Tibtech 11:238). In addition, it is possible to introduce a restriction site in the region of the mutation to create cleavage-based detection (Gasparini et al. (1992) Mol. Cell Probes 6:1). In another embodiment, amplification can be performed using Taq ligase for amplification (Barany (1991) Proc. Natl. Acad. Sci USA 88:189). In such cases, ligation will occur only if there is a perfect match at the 3' end of the 5' sequence making it possible to detect the presence of a known mutation at a specific site by looking for the presence or absence of amplification.

Enzymatic methods for detecting sequences include amplification based-methods such as the polymerase chain reaction (PCR; Saiki, et al. (1985) Science 230, 1350-1354) and ligase chain reaction (LCR; Wu. et al. (1989) Genomics 4, 560-569; Barringer et al. (1990), Gene 1989, 117-122; F. Barany. 1991, Proc. Natl. Acad. Sci. USA 1988, 189-193; transcription-based methods utilize RNA synthesis by RNA polymerases to amplify nucleic acid (U.S. Pat. No. 6,066,457; U.S. Pat. No. 6,132,997; U.S. Pat. No. 5,716,785; Sarkar et al., Science (1989) 244:331-34; Stofler et al., Science (1988) 239:491); NASBA (U.S. Patent Nos. 5,130,238; 5,409,818; and 5,554,517); rolling circle amplification (RCA; U.S. Patent Nos. 5,854,033 and 6,143,495) and strand displacement amplification (SDA; U.S. Patent Nos. 5,455,166 and 5,624,825). Amplification methods can be used in combination with other techniques.

Other enzymatic techniques include sequencing using polymerases, e.g., DNA polymerases and variations thereof such as single base extension technology. See, e.g., U.S. 6,294,336; U.S. 6,013,431; and U.S. 5,952,174

Mass spectroscopy (e.g., MALDI-TOF mass spectroscopy) can be used to detect nucleic acid polymorphisms. In one embodiment, (e.g., the MassEXTEND™ assay, SEQUENOM, Inc.), selected nucleotide mixtures, missing at least one dNTP and including a single ddNTP is used to extend a primer that hybridizes near a polymorphism. The nucleotide mixture is selected so that the extension products between the different polymorphisms at the site create the greatest difference in molecular size. The extension reaction is placed on a plate for mass spectroscopy analysis.

Fluorescence based detection can also be used to detect nucleic acid polymorphisms. For example, different terminator ddNTPs can be labeled with different fluorescent dyes. A primer can be annealed near or immediately adjacent to a polymorphism, and the nucleotide at the polymorphic site can be detected by the type (e.g., "color") of the fluorescent dye that is incorporated.

Hybridization to microarrays can also be used to detect polymorphisms, including SNPs. For example, a set of different oligonucleotides, with the polymorphic nucleotide at varying positions with the oligonucleotides can be positioned on a nucleic acid array. The extent of hybridization as a function of position and hybridization to oligonucleotides specific for the other allele can be used to determine whether a particular polymorphism is present. See, e.g., U.S. 6,066,454.

In one implementation, hybridization probes can include one or more additional mismatches to destabilize duplex formation and sensitize the assay. The mismatch may be directly adjacent to the query position, or within 10, 7, 5, 4, 3, or 2 nucleotides of the query position. Hybridization probes can also be selected to have a particular $T_m$, e.g., between 45-60°C, 55-65°C, or 60-75°C. In a multiplex assay, $T_m$'s can be selected to be within 5, 3, or 2°C of each other, e.g., probes for SNPs can be selected with these criteria.

It is also possible to directly sequence the nucleic acid for a particular genetic locus, e.g., by amplification and sequencing, or amplification, cloning and sequence. High throughput automated (e.g., capillary or microchip based) sequencing apparati can be used. In still other embodiments, the sequence of a protein of interest is analyzed to infer its genetic sequence. Methods of analyzing a protein sequence include protein sequencing, mass spectroscopy, sequence/epitope specific immunoglobulins, and protease digestion.

Any combination of the above methods can also be used. The above methods can be used to evaluate any genetic locus, e.g., in a method for analyzing genetic information from particular groups of individuals or in a method for analyzing a polymorphism associated with an age related disorder.

Computer Implementations

Certain aspects of the invention can be implemented in digital electronic circuitry, or in computer hardware, firmware, software, or in combinations thereof. Methods of the recording, storing, and/or analyzing genetic information can be implemented using a computer program product tangibly embodied in a machine-readable storage device for execution by a programmable processor; and method actions can be performed by a programmable processor executing a program of instructions to perform functions of the invention by operating on input data and generating output. For example, the methods can be implemented advantageously in one or more computer programs that are executable on a programmable system including at least one programmable processor coupled to receive data and instructions from, and to transmit data and instructions to, a data storage system, at least one input device, and at least one output device. Each computer program can be implemented in a high-level procedural or obj ect oriented programming language, or in assembly or machine language if desired; and in any case, the language can be a compiled or interpreted language. Suitable processors include, by way of example, both general and special purpose microprocessors. A processor can receive instructions and data from a read-only memory and/or a random access memory. Generally, a computer will include one or more mass storage devices for storing data files; such devices include magnetic disks, such as internal hard disks and removable disks; magneto-optical disks; and optical disks. Storage devices suitable for tangibly embodying computer program instructions and data include all forms of non-volatile memory, including, by way of example, semiconductor memory devices, such as EPROM, EEPROM, and flash memory devices; magnetic disks such as, internal hard disks and removable disks; magneto-optical disks; and CD_ROM disks. Any of the foregoing can be supplemented by, or incorporated in, ASICs (application-specific integrated circuits).

In one implementation, information about one or more genes that encode an AMPK pathway component of a subject (e.g., information about one or both AMPK alpha subunit genes) is stored on a server. A user can send information about the subject (e.g., a patient, a relative of a patient, a sample of gametes (e.g., sperm or oocytes), fetal cells, or a candidate for a treatment) to the server, e.g., from a remote computer that communicates with the server using a network, e.g., the Internet. The server can compare the information about the subject, e.g., to reference information to produce an indication as to the individual propensity for an age-related disorder. For example, the reference information can be information derived from a reference individual, a particular sequence, or a population of sequences. The indication can be, for example, qualitative or quantitative. An exemplary qualitative indication includes a binary output or a descriptive output (e.g., text or other symbols indicating degree of propensity for an age-related disorder). An exemplary qualitative indication includes a statistical measure of the probability of developing an age-related disorder, a score, a percentage, or a parameter for a risk evaluation (e.g., a parameter that can be used in a financial evaluation).

It is also possible for the server to return the indication or information about related subjects (e.g., family members or subjects with similar AMPK pathway component loci), e.g., to a user. For example, the server can build a family tree based on a set of related subjects. Each individual can be, e.g., assigned a statistical score that evaluates probability of an age related disorder as a function of an AMPK pathway component gene, e.g., the gene encoding AMPK alpha subunit, and/or other factors. Accordingly, the server can include an electronic interface for receiving information from a user or from an apparatus that provides genetic information, e.g., a DNA analyzer such as a DNA sequencer.

In one method, information about an AMPK pathway component gene of a subject, e.g., the result of evaluating a polymorphism of a gene described herein, is provided (e.g., communicated, e.g., electronically communicated) to a third party, e.g., a hospital, clinic, a government entity, reimbursing party or insurance company (e.g., a life insurance company). For example, choice of medical procedure, payment for a medical procedure, payment by a reimbursing party, or cost for a service or insurance can be function of the information.

In one embodiment, a premium for insurance (e.g., life or medical) is evaluated as a function of information about one or more longevity associated polymorphisms, e.g., a polymorphism of an AMPK pathway component gene. For example, premiums can be increased (e.g., by a certain percentage) if a first polymorphism is present in the candidate insured, or decreased if a second polymorphism is present. Premiums can also be scaled depending on heterozygosity or homozygosity. For example, premiums can be assessed to distribute risk, e.g., commensurate with the allele distribution for the particular polymorphism. In another examples, premiums are assessed as a function of actuarial data that is obtained from individuals with one or more particular polymorphisms.

Genetic information about one or more polymorphisms of an AMPK pathway component gene can be used, e.g., in an underwriting process for life insurance. The information can be incorporated into a profile about a subject. Other information in the profile can include, for example, date of birth, gender, marital status, banking information, credit information, children and so forth. An insurance policy can be recommended as a function of the genetic information along with one or more other items of information in the profile. An insurance premium or risk assessment can also be evaluated as function of the genetic information. In one implementation, points are assigned for presence or absence of a particular allele. The total points for a particular polymorphisms and other risk parameters are summed. A premium is calculated as a function of the points, and optionally one or more other parameters.

Information about polymorphism of an AMPK pathway component gene can also be analyzed by a function that determines whether to authorize or transfer of funds to pay for a service or treatment provided to a subject. For example, an allele that is not associated with a particular disposition can trigger an outcome that indicates or causes a refusal to pay for a service or treatment provided to a subject. For example, an entity, e.g., a hospital, care giver, government entity, or an insurance company or other entity which pays for, or reimburses medical expenses, can use the outcome of a method described herein to determine whether a party, e.g., a party other than the subject patient, will pay for services or treatment provided to the patient. For example, a first entity, e.g., an insurance company, can use the outcome of a method described herein to determine whether to provide financial payment to, or on behalf of, a patient, e.g., whether to reimburse a third party, e.g., a vendor of goods or services, a hospital, physician, or other care-giver, for a service or treatment provided to a patient. For example, a first entity, e.g., an insurance company, can use the outcome of a method described herein to determine whether to continue, discontinue, enroll an individual in an insurance plan or program, e.g., a health insurance or life insurance plan or program.

Pharmacogenomics

Both prophylactic and therapeutic methods of treatment may be specifically tailored or modified, based on knowledge obtained from a pharmacogenomics analysis. In particular, a subject can be treated based on the presence or absence of a genetic polymorphism of an AMPK pathway component gene. Pharmacogenomics allows a clinician or physician to target prophylactic or therapeutic treatments to patients who will most benefit from the treatment and to avoid the treatment of patients who will experience toxic or other undesirable drug-related side effects. In particular, a diet or drug that affects longevity or an age-associated disease can be prescribed as a function of polymorphism of an AMPK pathway component gene of a subject. For example, if the individual's AMPK alpha subunit gene includes an allele that is predisposed to an age-associate disease relative to other alleles, the individual can be indicated for a prophylactic treatment for a drug that alleviates the disease. In another example, the individual is placed in a monitoring program; e.g., to closely monitor for physical manifestations of the disease.

Pharmaceutical Compositions

A compound that modulates the AMPK pathway can be incorporated into a pharmaceutical composition for administration to a subject, e.g., a human, a non-human animal, e.g., an animal patient (e.g., pet or agricultural animal) or an animal model (e.g., an animal model for aging or a metabolic disorder (e.g., a pancreatic or insulin related disorder). Such compositions typically include a small molecule (e.g., a small molecule that is an AMPK activator), nucleic acid molecule, protein, or antibody and a pharmaceutically acceptable carrier. As used herein the language "pharmaceutically acceptable carrier" includes solvents, dispersion media, coatings, antibacterial and antifungal agents, isotonic and absorption delaying agents, and the like, compatible with pharmaceutical administration. Other active compounds can also be incorporated into the compositions.

Exemplary compounds that can be used for activate AMPK or the AMPK pathway include:

1. Bi-guanides such as metformin. Metformin and other bi-guanides can be used to activate AMPK activity.

2. Thiazolidinediones, e.g., rosiglitazone and pioglitazone. Thiazolidinedones are a class of anti-diabetic drug that can , for example, inhibit mitochondrial oxidation in muscle cells. Rosiglitazone is an example of a thiazolidinedones, and is known to increase AMPK activity. These compound may activate AMPK by causing an increase in the AMP: ATP ratio. Other examples of thiazolidinedones include pioglitazone and troglitazone. See, e.g., Mudaliar and Henry (2001) Annu Rev. Med, 52:239-257.

3. An AMP analog such as AICAR= 5'-aminoimidazole-4-carboxyamide-riboside. AICAR can be used to activate AMPK kinase activity. Within cells, AICAR is converted to the AMP analog "ZMP" which activates AMPK.

4. Leptin and leptin-related molecules. Leptin is a peptide hormone that increases AMPK activity when applied to cells. See, Minokoshi (2002) Nature 415:339. Leptin, leptin variants, and synthetic forms of leptin can be used, e.g., peptides and other fragments described in US Published Application 2002-0037553.

5. Adiponectin and Adiponectin-related molecules. Globular and full-length adiponectin can stimulate AMPK activity in skeletal muscle. Yamauchi et al. (2002) Nature Medicine 8:1. Full length adiponectin stimulates AMPK activity in liver cells. Accordingly, adiponectin and adiponectin variants can be used to treat a cell or organism to increase AMPK pathway activity, e.g., to alter lifespan regulation.

A pharmaceutical composition is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, NJ) or phosphate buffered saline (PBS). In all cases, the composition must be sterile and should be fluid to the extent that easy syringability exists. It should be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, or sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. For the purpose of oral therapeutic administration, the active compound can be incorporated with excipients and used in the form of tablets, troches, or capsules, e.g., gelatin capsules. Oral compositions can also be prepared using a fluid carrier for use as a mouthwash. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art. The compounds can also be prepared in the form of suppositories or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to particular cells, e.g., a pituitary cell) can also be used as pharmaceutically acceptable carriers. These can be prepared according to methods known to those skilled in the art, for example, as described in U.S. 4,522,811.

It can be advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier.

Toxicity and therapeutic efficacy of such compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit high therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC50 (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

As defined herein, a therapeutically effective amount of protein or polypeptide (i.e., an effective dosage) ranges from about 0.001 to 30 mg/kg body weight, preferably about 0.01 to 25 mg/kg body weight, more preferably about 0.1 to 20 mg/kg body weight, and even more preferably about 1 to 10 mg/kg, 2 to 9 mg/kg, 3 to 8 mg/kg, 4 to 7 mg/kg, or 5 to 6 mg/kg body weight. The protein or polypeptide can be administered one time per week for between about 1 to 10 weeks, preferably between 2 to 8 weeks, more preferably between about 3 to 7 weeks, and even more preferably for about 4, 5, or 6 weeks. The skilled artisan will appreciate that certain factors may influence the dosage and timing required to effectively treat a subject, including but not limited to the severity of the disease or disorder, previous treatments, the general health and/or age of the subject, and other diseases present. Moreover, treatment of a subject with a therapeutically effective amount of a compound can include a single treatment or, preferably, can include a series of treatments.

For antibody compounds that modulate AMPK pathway components, one preferred dosage is 0.1 mg/kg of body weight (generally 10 mg/kg to 20 mg/kg). Generally, partially human antibodies and fully human antibodies have a longer half life within the human body than other antibodies. Accordingly, lower dosages and less frequent administration is often possible. Modifications such as lipidation can be used to stabilize antibodies and to enhance uptake and tissue penetration. A method for lipidation of antibodies is described by Cruikshank et al. ((1997) J. Acquired Immune Deficiency Syndromes and Human Retrovirology 14:193).

The present invention encompasses agents that modulate expression or activity of AMPK pathway components. An agent may, for example, be a small molecule. For example, such small molecules include, but are not limited to, peptides, peptidomimetics (e.g., peptoids), amino acids, amino acid analogs, polynucleotides, polynucleotide analogs, nucleotides, nucleotide analogs, organic or inorganic compounds (i.e.,. including heteroorganic and organometallic compounds) having a molecular weight less than about 10,000 grams per mole, organic or inorganic compounds having a molecular weight less than about 5,000 grams per mole, organic or inorganic compounds having a molecular weight less than about 1,000 grams per mole, organic or inorganic compounds having a molecular weight less than about 500 grams per mole, and salts, esters, and other pharmaceutically acceptable forms of such compounds.

Exemplary doses include milligram or microgram amounts of the small molecule per kilogram of subject or sample weight (e.g., about 1 microgram per kilogram to about 500 milligrams per kilogram, about 100 micrograms per kilogram to about 5 milligrams per kilogram, or about 1 microgram per kilogram to about 50 micrograms per kilogram. It is furthermore understood that appropriate doses of a small molecule depend upon the potency of the small molecule with respect to the expression or activity to be modulated. When one or more of these small molecules is to be administered to an animal (e.g., a human) in order to modulate expression or activity of a polypeptide or nucleic acid of the invention, a physician, veterinarian, or researcher may, for example, prescribe a relatively low dose at first, subsequently increasing the dose until an appropriate response is obtained. In addition, it is understood that the specific dose level for any particular animal subject will depend upon a variety of factors including the activity of the specific compound employed, the age, body weight, general health, gender, and diet of the subject, the time of administration, the route of administration, the rate of excretion, any drug combination, and the degree of expression or activity to be modulated.

The nucleic acid molecules that modulate the AMPK pathway can be inserted into vectors and used as gene therapy vectors. For example, the nucleic acid can encode an constitutively active AMPK protein subunit. Gene therapy vectors can be delivered to a subject by, for example, intravenous injection, local administration (see U.S. 5,328,470) or by stereotactic injection (see e.g., Chen et al. Proc. Natl. Acad. Sci. USA 91:3054-3057, 1994). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, e.g., retroviral vectors, the pharmaceutical preparation can include one or more cells which produce the gene delivery system.

The pharmaceutical compositions can be included in a container, pack, or dispenser together with instructions for administration.

Modulating Lifespan Regulation in Subjects

Agents that alter AMPK pathway activity can be used to modulate lifespan regulation in subjects, e.g., animal (e.g., mammalian, e.g., human subjects). The compositions can be administered to a subject, e.g., an adult subject, e.g., a healthy adult subject or a subject having an age-related disease. In the latter case, the method can include evaluating a subject, e.g., to characterize a symptom of an age-related disease or other disease marker, and thereby identifying a subject as having an age-related disease or being pre-disposed to such a disease. Exemplary age-related diseases include: cancer (e.g., breast cancer, colorectal cancer, CCL, CML, prostate cancer); skeletal muscle atrophy; adult-onset diabetes; diabetic nephropathy, neuropathy (e.g., sensory neuropathy, autonomic neuropathy, motor neuropathy, retinopathy); obesity; bone resorption; age-related macular degeneration, AIDS related dementia, ALS, Alzheimer's, Bell's Palsy, atherosclerosis, cardiac diseases (e.g., cardiac dysrhythmias, chronic congestive heart failure, ischemic stroke, coronary artery disease and cardiomyopathy), chronic renal failure, type 2 diabetes, ulceration, cataract, presbiopia, glomerulonephritis, Guillan-Barre syndrome, hemorrhagic stroke, rheumatoid arthritis, inflammatory bowel disease, multiple sclerosis, SLE, Crohn's disease, osteoarthritis, Parkinson's disease, pneumonia, and urinary incontinence.

In addition, many neurodegenerative disorders and disorders that are associated with protein aggregation or protein misfolding can also be age-related. Disorders involving a misfolded protein have been identified in mammals. These disorders include, for example, Parkinson's disease; prion diseases (including Creutzfeldt-Jakob disease (CJD), Fatal Familia insomnia (FFI), Gerstmann-Straussler-Scheinker disease (GSS), mad cow disease, Scrapie, and kuru); Familial Amyloid Polyneuropathy, Tauopathies (including Pick Disease, Lobar Atrophy, and Frontotemporal dementia); polyglutamine aggregation disorders, Fragile-X syndrome, myotonic dystrophy, Haw River Syndrome, hereditary ataxias, and Machado Joseph disease. Alzheimer's disease is an example of a disease in which amyloid is produced, e.g., as a result of protein aggregation. Amyloid is also produced in other disorders, e.g., due to transthyretin aggregation etc.

Moreover, aberrant aggregation is a common feature of many neurodegenerative diseases, not only disorders caused at least in part by polyglutamine aggregation. For example, aberrant aggregation is also a principal factor in Alzheimer's disease (amyloid plaques) and Parkinson's disease (Lewy bodies). The formation of protein aggregates may be involved at some stage in disease pathogenesis in a variety of disorders, neurological and otherwise (see, e.g., diseases caused by protein misfolding).

Exemplary neurodegenerative disorders that are caused at least in part by polyglutamine aggregation include: Spinalbulbar Muscular Atrophy (SBMA or Kennedy's Disease) Dentatorubropallidoluysian Atrophy (DRPLA), Spinocerebellar Ataxia 1 (SCA1), Spinocerebellar Ataxia 2 (SCA2), Machado-Joseph Disease (MJD; SCA3), Spinocerebellar Ataxia 6 (SCA6), Spinocerebellar Ataxia 7 (SCA7), and Spinocerebellar Ataxia 12 (SCA12). In a particular embodiment, the neurodegenerative disorder is Huntington's disease.

Symptoms and diagnosis of such diseases are well known to medical practitioners.

The compositions may also be administered to individuals being treated by other means for such diseases, for example, individuals being treated with a chemotherapeutic (e.g., and having neutropenia, atrophy, cachexia, nephropathy, neuropathy) or an elective surgery.

Subjects can be diagnosed and evaluated, e.g., before, during, and after treatment. Standard medical procedures can be used to monitor the health and fitness of the subject. In addition, a parameter of metabolic activity (e.g., insulin levels) can be monitored.

In some embodiments, the AMPK pathway modulating agent is directed to a particular cell (e.g., by using a targeting vehicle or by using a cell-type specific regulatory sequence for a nucleic acid). For example, the agent can be targeting to an adipose, liver, pancreatic, brain, or skeletal muscle cell. In some examples, the targeted tissue participates in metabolic regulation.

Evaluating polyglutamine aggregation

A variety of cell free assays, cell based assays, and organismal assays are available for evaluating polyglutamine aggregation, e.g., Huntingtin polyglutamine aggregation. Some examples are described, e.g., in U.S. 2003-0109476.

Assays (e.g., cell free, cell-based, or organismal) can include a reporter protein that includes a polyglutamine repeat region which has at least 35 polyglutamines. The reporter protein can be easily detectable, e.g., by fluorescence. For example, the protein is conjugated to a fluorophore, for example, fluorescein isothiocyanate (FITC), allophycocyanin (APC), R-phycoerythrin (PE), peridinin chlorophyll protein (PerCP), Texas Red, Cy3, Cy5, Cy7, or a fluorescence resonance energy tandem fluorophore such as PerCP-Cy5.5, PE-Cy5, PE-Cy5.5, PE-Cy7, PE-Texas Red, and APC-Cy7. In another example the protein is "intrinsically fluorescent" in that it has a chromophore is entirely encoded by its amino acid sequence and can fluoresce without requirement for cofactor or substrate. For example, the protein can include a green fluorescent protein (GFP)-like chromophore. As used herein, "GFP-like chromophore" means an intrinsically fluorescent protein moiety comprising an 11-stranded β-barrel with a central α-helix, the central α-helix having a conjugated π-resonance system that includes two aromatic ring systems and the bridge between them.

The GFP-like chromophore can be selected from GFP-like chromophores found in naturally occurring proteins, such as *A. victoria* GFP (GenBank accession number AAA27721), *Renilla reniformis* GFP, FP583 (GenBank accession no. AF168419) (DsRed), FP593 (AF272711), FP483 (AF168420), FP484 (AF168424), FP595 (AF246709), FP486 (AF168421), FP538 (AF168423), and FP506 (AF168422), and need include only so much of the native protein as is needed to retain the chromophore's intrinsic fluorescence. Methods for determining the minimal domain required for fluorescence are known in the art. Li et al., J. Biol. Chem. 272:28545-28549 (1997).

Alternatively, the GFP-like chromophore can be selected from GFP-like chromophores modified from those found in nature. Typically, such modifications are made to improve recombinant production in heterologous expression systems (with or without change in protein sequence), to alter the excitation and/or emission spectra of the native protein, to facilitate purification, to facilitate or as a consequence of cloning, or are a fortuitous consequence of research investigation. The methods for engineering such modified GFP-like chromophores and testing them for fluorescence activity, both alone and as part of protein fusions, are well-known in the art. Early results of these efforts are reviewed in Heim et al., Curr. Biol. 6:178-182 (1996), a more recent review, with tabulation of useful mutations, is found in Palm et al., "Spectral Variants of Green Fluorescent Protein," in Green Fluorescent Proteins, Conn (ed.), Methods Enzymol. vol. 302, pp. 378-394 (1999). A variety of such modified chromophores are now commercially available and can readily be used in the fusion proteins of the present invention.

For example, EGFP ("enhanced GFP"), Cormack et al., Gene 173:33-38 (1996); U.S. Pat. Nos. 6,090,919 and 5,804,387, is a red-shifted, human codon-optimized variant of GFP that has been engineered for brighter fluorescence, higher expression in mammalian cells, and for an excitation spectrum optimized for use in flow cytometers. EGFP can usefully contribute a GFP-like chromophore to the fusion proteins that further include a polyglutamine region. A variety of EGFP vectors, both plasmid and viral, are available commercially (Clontech Labs, Palo Alto, Calif., USA). Still other engineered GFP proteins are known. See, e.g., , Heim et al., Curr. Biol. 6:178-182 (1996); Cormack et al., Gene 173: 33-38 (1996), BFP2, EYFP ("enhanced yellow fluorescent protein"), EBFP, Ormo et al., Science 273:1392-1395 (1996), Heikal et al., Proc. Natl. Acad. Sci. USA 97:11996-12001 (2000). ECFP ("enhanced cyan fluorescent protein") (Clontech Labs, Palo Alto, Calif., USA). The GFP-like chromophore can also be drawn from other modified GFPs, including those described in U.S. Pat. Nos. 6,124,128; 6,096,865; 6,090,919; 6,066,476; 6,054,321; 6,027,881; 5,968,750; 5,874,304; 5,804,387; 5,777,079; 5,741,668; and 5,625,048.

In one embodiment, a reporter protein that includes a polyglutamine repeat region which has at least 35 polyglutamines. is used in a cell-based assay.

In one example, PC12 neuronal cell lines that have a construct engineered to express a protein encoded by HD gene exon 1 containing alternating, repeating codons (e.g., repeats of "CAA CAG CAG CAA CAG CAA", SEQ ID NO:_____) fused to an enhanced GFP (green fluorescent protein) gene can be used. See, e.g., Boado et al. J. Pharmacol. and Experimental Therapeutics 295(1): 239-243 (2000) and Kazantsev et al. Proc. Natl. Acad. Sci. USA 96: 11404-09 (1999). Expression of this gene leads to the appearance of green fluorescence co-localized to the site of protein aggregates. The HD gene exon 1-GFP fusion gene is under the control of an inducible promoter regulated by muristerone. A particular construct has approximately 46 glutamine repeats (encoded by either CAA or CAG). Other constructs have, for example, 103 glutamine repeats. PC12 cells are grown in DMEM, 5% Horse serum (heat inactivated), 2.5% FBS and 1% Pen-Strep, and maintained in low amounts on Zeocin and G418. The cells are plated in 24-well plates coated with poly-L-lysine coverslips, at a density of $5 \cdot 10^5$ cells/ml in media without any selection. Muristerone is added after the overnight incubation to induce the expression of HD gene exon 1-GFP. The cells can be contacted with a test compound, e.g., before or after plating and before or after induction. The data can be acquired on a Zeiss inverted 100M Axioskop equipped with a Zeiss 510 LSM confocal microscope and a Coherent Krypton Argon laser and a Helium Neon laser. Samples can be loaded into Lab-Tek II chambered coverglass system for improved imaging. The number of Huntingtin-GFP aggregations within the field of view of the objective is counted in independent experiments (e.g., at least three or seven independent experiments).

Other exemplary means for evaluating samples include a high throughput apparatus, such as the Amersham Biosciences IN Cell Analysis System and Cellomics™ ArrayScan HCS System which permit the subcellular location and concentration of fluorescently tagged moieties to be detected and quantified, both statically and kinetically. See also, U.S. Pat. No. 5,989,835.

Other exemplary mammalian cell lines include: a CHO cell line and a 293 cell line. For example, CHO cells with integrated copies of HD gene exon 1 with approximately 103Q repeats fused to GFP as a fusion construct encoding HD gene exon 1 Q103-GFP produce a visible GFP aggregation at the nuclear membrane, detectable by microscopy, whereas CHO cells with integrated copies of fusion constructs encoding HD gene exon 1 Q24-GFP in CHO cells do not produce a visible GFP aggregation at the nuclear membrane. In another example, 293 cells with integrated copies of the HD gene exon 1 containing 84 CAG repeats are used.

A number of animal model system for Huntington's disease are available. See, e.g., Brouillet, Functional Neurology 15(4): 239-251 (2000); Ona et al. Nature 399: 263-267 (1999), Bates et al. Hum Mol Genet. 6(10):1633-7 (1997); Hansson et al. J. of Neurochemistry 78: 694-703; and Rubinsztein, D. C., Trends in Genetics, Vol. 18, No. 4, pp. 202-209 (a review on various animal and non-human models of HD).

In one embodiment, the animal is a transgenic mouse that can express (in at least one cell) a human Huntingtin protein, a portion thereof, or fusion protein comprising human Huntingtin protein, or a portion thereof, with, for example, at least 36 glutamines (e.g., encoded by CAG repeats (alternatively, any number of the CAG repeats may be CAA) in the CAG repeat segment of exon 1 encoding the polyglutamine tract).

An example of such a transgenic mouse strain is the R6/2 line (Mangiarini et al. Cell 87: 493-506 (1996)). The R6/2 mice are transgenic Huntington's disease mice, which over-express exon one of the human HD gene (under the control of the endogenous promoter). The exon 1 of the R6/2 human HD gene has an expanded CAG/polyglutamine repeat lengths (150 CAG repeats on average). These mice develop a progressive, ultimately fatal neurological disease with many features of human Huntington's disease. Abnormal aggregates, constituted in part by the N-terminal part of Huntingtin (encoded by HD exon 1), are observed in R6/2 mice, both in the cytoplasm and nuclei of cells (Davies et al. Cell 90: 537-548 (1997)). For example, the human Huntingtin protein in the transgenic animal is encoded by a gene that includes at least 55 CAG repeats and more preferably about 150 CAG repeats.

These transgenic animals can develop a Huntington's disease-like phenotype. These transgenic mice are characterized by reduced weight gain, reduced lifespan and motor impairment characterized by abnormal gait, resting tremor, hindlimb clasping and hyperactivity from 8 to 10 weeks after birth (for example the R6/2 strain; see Mangiarini et al. Cell 87: 493-506 (1996)). The phenotype worsens progressively toward hypokinesia. The brains of these transgenic mice also demonstrate neurochemical and histological abnormalities, such as changes in neurotransmitter receptors (glutamate, dopaminergic), decreased concentration ofN-acetylaspartate (a marker of neuronal integrity) and reduced striatum and brain size. Accordingly, evaluating can include assessing parameters related to neurotransmitter levels, neurotransmitter receptor levels, brain size and striatum size. In addition, abnormal aggregates containing the transgenic part of or full-length human Huntingtin protein are present in the brain tissue of these animals (e.g., the R6/2 transgenic mouse strain). See, e.g., Mangiarini et al. Cell 87: 493-506 (1996), Davies et al. Cell 90: 537-548 (1997), Brouillet, Functional Neurology 15(4): 239-251 (2000) and Cha et al. Proc. Natl. Acad. Sci. USA 95: 6480-6485 (1998).

To test the effect of the test compound or known compound described in the application in an animal model, different concentrations of test compound are administered to the transgenic animal, for example by injecting the test compound into circulation of the animal. In one embodiment, a Huntington's disease-like symptom is evaluated in the animal. For example, the progression of the Huntington's disease-like symptoms, e.g. as described above for the mouse model, is then monitored to determine whether treatment with the test compound results in reduction or delay of symptoms. In another embodiment, disaggregation of the Huntingtin protein aggregates in these animals is monitored. The animal can then be sacrificed and brain slices are obtained. The brain slices are then analyzed for the presence of aggregates containing the transgenic human Huntingtin protein, a portion thereof, or a fusion protein comprising human Huntingtin protein, or a portion thereof. This analysis can includes, for example, staining the slices of brain tissue with anti-Huntingtin antibody and adding a secondary antibody conjugated with FITC which recognizes the anti-Huntingtin's antibody (for example, the anti-Huntingtin antibody is mouse anti-human antibody and the secondary antibody is specific for human antibody) and visualizing the protein aggregates by fluorescent microscopy. Alternatively, the anti-Huntingtin antibody can be directly conjugated with FITC. The levels of Huntingtin's protein aggregates are then visualized by fluorescent microscopy.

A *Drosophila melanogaster* model system for Huntington's disease is also available. See, e.g., Steffan et al., Nature, 413: 739-743 (2001) and Marsh et al., Human Molecular Genetics 9: 13-25 (2000). For example, a transgenic Drosophila can be engineered to express human Huntingtin protein, a portion thereof (such as exon 1), or fusion protein comprising human Huntingtin protein, or a portion thereof, with, for example, a polyglutamine region that includes at least 36 glutamines (e.g., encoded by CAG repeats (preferably 51 repeats or more) (alternatively, any number of the CAG repeats may be CAA)) The polyglutamine region can be encoded by the CAG repeat segment of exon 1 encoding the poly Q tract. These transgenic flies can also engineered to express human Huntingtin protein, a portion thereof (such as exon 1), or fusion protein comprising human Huntingtin protein, or a portion thereof, in neurons, e.g., in the Drosophila eye.

The test compound (e.g., different concentrations of the test compound) or a compound described herein can be administered to the transgenic Drosophila, for example, by applying the pharmaceutical compositions that include the compound into to the animal or feeding the compound as part of food. Administration of the compound can occur at various stages of the Drosophila life cycle. The animal can be monitored to determine whether treatment with the compound results in reduction or delay of Huntington's disease-like symptoms, disaggregation of the Huntingtin protein aggregates, or reduced lethality and/or degeneration of photoreceptor neurons are monitored.

Neurodegeneration due to expression of human Huntingtin protein, a portion thereof (such as exon 1), or fusion protein comprising human Huntingtin protein, or a portion thereof, is readily observed in the fly compound eye, which is composed of a regular trapezoidal arrangement of seven visible rhabdomeres (subcellular light-gathering structures) produced by the photoreceptor neurons of each Drosophila ommatidium. Expression of human Huntingtin protein, a portion thereof (such as exon 1), or fusion protein comprising human Huntingtin protein, or a portion thereof, leads to a progressive loss of rhabdomeres. Thus, an animal to which a test compound is administered can be evaluated for neuronal degeneration.

Morley et al. (2002) Proc. Nat. Acad. USA Vol. 99:10417 describes a *C. elegans* system for evaluating Huntington's disease related protein aggregation.

Evaluting Huntington's Disease

A variety of methods are available to evaluate and/or monitor Huntington's disease. A variety of clinical symptoms and indicia for the disease are known. Huntington's disease causes a movement disorder, psychiatric difficulties and cognitive changes. The degree, age of onset, and manifestation of these symptoms can vary. The movement disorder can include quick, random, dance-like movements called chorea.

One method for evaluating Huntington's disease uses the Unified Huntington's disease Rating Scale (UNDRS). It is also possible to use individual tests alone or in combination to evaluate if at least one symptom of Huntington's disease is ameliorated. The UNDRS is described in Movement Disorders (vol. 11:136-142,1996) and Marder et al. Neurology (54:452-458, 2000). The UNDRS quantifies the severity of Huntington's Disease. It is divided into multiple subsections: motor, cognitive, behavioral, functional. In one embodiment, a single subsection is used to evaluate a subject. These scores can be calculated by summing the various questions of each section. Some sections (such as chorea and dystonia) can include grading each extremity, face, bucco-oral-ligual, and trunk separately.

Exemplary motor evaluations include: ocular pursuit, saccade initiation, saccade velocity, dysarthria, tongue protrusion, finger tap ability, pronate/supinate, a fist-hand-palm sequence, rigidity of arms, bradykinesia, maximal dystonia (trunk, upper and lower extremities), maximal chorea (e.g., trunk, face, upper and lower extremities), gait, tandem walking, and retropulsion. An exemplary treatment can cause a change in the Total Motor Score 4 (TMS-4), a subscale of the UNDRS, e.g., over a one-year period.

Alzheimer's Disease

Alzheimer's Disease (AD) is a complex neurodegenerative disease that results in the irreversible loss of neurons. It provides merely one example of a neurodegenerative disease that has symptoms caused at least in part by protein aggregation. Clinical hallmarks of Alzheimer's Disease include progressive impairment in memory, judgment, orientation to physical surroundings, and language. Neuropathological hallmarks of AD include region-specific neuronal loss, amyloid plaques, and neurofibrillary tangles. Amyloid plaques are extracellular plaques containing the β amyloid peptide (also known as Aβ, or Aβ42), which is a cleavage product of the β-amyloid precursor protein (also known as APP). Neurofibrillary tangles are insoluble intracellular aggregates composed of filaments of the abnormally hyperphosphorylated microtubule-associated protein, tau. Amyloid plaques and neurofibrillary tangles may contribute to secondary events that lead to neuronal loss by apoptosis (Clark and Karlawish, Ann. Intern. Med. 138(5):400-410 (2003). For example, β-Amyloid induces caspase-2-dependent apoptosis in cultured neurons (Troy et al. J. Neurosci. 20(4):1386-1392). The deposition of plaques *in vivo* may trigger apoptosis of proximal neurons in a similar manner.

Mutations in genes encoding APP, presenilin-1, and presenilin-2 have been implicated in early-onset AD (Lendon et al. JAMA 227:825 (1997)). Mutations in these proteins have been shown to enhance proteolytic processing of APP via an intracellular pathway that produces Aβ. Aberrant regulation of Aβ processing may be central to the formation of amyloid plaques and the consequent neuronal damage associated with plaques.

A variety of criteria, including genetic, biochemical, physiological, and cognitive criteria, can be used to evaluate AD in a subject. Symptoms and diagnosis of AD are known to medical practitioners. Some exemplary symptoms and markers of AD are presented below. Information about these indications and other indications known to be associated with AD can be used as an "AD-related parameter." An AD-related parameter can include qualitative or quantitative information. An example of quantitative information is a numerical value of one or more dimensions, e.g., a concentration of a protein or a tomographic map. Qualitative information can include an assessment, e.g., a physician's comments or a binary ("yes"/"no") and so forth. An AD-related parameter includes information that indicates that the subject is not diagnosed with AD or does not have a particular indication of AD, e.g., a cognitive test result that is not typical of AD or a genetic APOE polymorphism not associated with AD.

Progressive cognitive impairment is a hallmark of AD. This impairment can present as decline in memory, judgment, decision making, orientation to physical surroundings, and language (Nussbaum and Ellis, New Eng. J. Med. 348(14):1356-1364 (2003)). Exclusion of other forms of dementia can assist in making a diagnosis of AD.

Neuronal death leads to progressive cerebral atrophy in AD patients. Imaging techniques (e.g., magnetic resonance imaging, or computed tomography) can be used to detect AD-associated lesions in the brain and/or brain atrophy.

AD patients may exhibit biochemical abnormalities that result from the pathology of the disease. For example, levels of tau protein in the cerebrospinal fluid is elevated in AD patients (Andreasen, N. et al. Arch Neurol. 58:349-350 (2001)). Levels of amyloid beta 42 (Aβ42) peptide can be reduced in CSF of AD patients (Galasko, D., et al. Arch. Neurol. 55:937-945 (1998)). Levels of Aβ42 can be increased in the plasma of AD patients (Ertekein-Taner, N., et al. Science 290:2303-2304 (2000)). Techniques to detect biochemical abnormalities in a sample from a subject include cellular, immunological, and other biological methods known in the art. For general guidance, see, e.g., techniques described in Sambrook & Russell, Molecular Cloning: A Laboratory Manual, 3rd Edition, Cold Spring Harbor Laboratory, N.Y. (2001), Ausubel et al., Current Protocols in Molecular Biology (Greene Publishing Associates and Wiley Interscience, N.Y. (1989), (Harlow, E. and Lane, D. (1988) Antibodies: A Laboratory Manual, Cold Spring Harbor Laboratory Press, Cold Spring Harbor, NY), and updated editions thereof.

For example, antibodies, other immunoglobulins, and other specific binding ligands can be used to detect a biomolecule, e.g., a protein or other antigen associated with AD. For example, one or more specific antibodies can be used to probe a sample. Various formats are possible, e.g., ELISAs, fluorescence-based assays, Western blots, and protein arrays. Methods of producing polypeptide arrays are described in the art, e.g., in De Wildt et al. (2000). Nature Biotech. 18, 989-994; Lueking et al. (1999). Anal. Biochem. 270, 103-111; Ge, H. (2000). Nucleic Acids Res. 28, e3, I-VII; MacBeath, G., and Schreiber, S.L. (2000). Science 289, 1760-1763; and WO 99/51773A1.

Proteins can also be analyzed using mass spectroscopy, chromatography, electrophoresis, enzyme interaction or using probes that detect post-translational modification (e.g., a phosphorylation, ubiquitination, glycosylation, methylation, or acetylation).

Nucleic acid expression can be detected in cells from a subject, e.g., removed by surgery, extraction, post-mortem or other sampling (e.g., blood, CSF). Expression of one or more genes can be evaluated, e.g., by hybridization based techniques, e.g., Northern analysis, RT-PCR, SAGE, and nucleic acid arrays. Nucleic acid arrays are useful for profiling multiple mRNA species in a sample. A nucleic acid array can be generated by various methods, e.g., by photolithographic methods (see, e.g., U.S. Patent Nos. 5,143,854; 5,510,270; and 5,527,681), mechanical methods (e.g., directed-flow methods as described in U.S. Patent No. 5,384,261), pin-based methods (e.g., as described in U.S. Pat. No. 5,288,514), and bead-based techniques (e.g., as described in PCT US/93/04145).

Metabolites that are associated with AD can be detected by a variety of means, including enzyme-coupled assays, using labeled precursors, and nuclear magnetic resonance (NMR). For example, NMR can be used to determine the relative concentrations of phosphate-based compounds in a sample, e.g., creatine levels. Other metabolic parameters such as redox state, ion concentration (e.g., $Ca^{2+}$)(e.g., using ion-sensitive dyes), and membrane potential can also be detected (e.g., using patch-clamp technology).

Information about an AD-associated marker can be recorded and/or stored in a computer-readable format. Typically the information is linked to a reference about the subject and also is associated (directly or indirectly) with information about the identity of one or more nucleotides in a gene that encodes an AMPK pathway component in the subject.

In one embodiment, it is possible to use a mouse model of AD. For example, US 6,509,515 describes one such model animal which is naturally able to be used with learning and memory tests1. The animal expresses an amyloid precursor protein (APP) sequence at a level in brain tissues such that the animal develops a progressive neurologic disorder within a short period of time from birth, generally within a year from birth, preferably within 2 to 6 months, from birth. The LAPP protein sequence is introduced into the animal, or an ancestor of the animal, at an embryonic stage, preferably the one cell, or fertilized oocyte, stage, and generally not later than about the 8-cell stage. The zygote or embryo is then developed to term in a pseudo-pregnant foster female. The amyloid precursor protein genes are introduced into an animal embryo so as to be chromosomally incorporated in a state which results in super-endogenous expression of the amyloid precursor protein and the development of a progressive neurologic disease in the cortico-limbic areas of the brain, areas of the brain which are prominently affected in progressive neurologic disease states such as AD. The gliosis and clinical manifestations in affected transgenic animals are indicative of a true neurologic disease. The progressive aspects of the neurologic disease are characterized by diminished exploratory and/or locomotor behavior and diminished 2-deoxyglucose uptake/utilization and hypertrophic gliosis in the cortico-limbic regions of the brain. Further, the changes that are seen are similar to those that are seen in some aging animals. Other animal models are also described in US 5,387,742; 5,877,399; 6,358,752; and 6,187,992.

In one embodiment, the animal also includes a deficiency in at least one cell in a AMPK pathway component, e.g., a genetic mutation, an antisense construct, a construct that produces RNAi. The deficiency can also be produced epigenetically, e.g., by administering RNAi, e.g., siRNA. This animal model can be used to screen for compounds which restore the level of activity to normal, e.g., produce the AD progression seen if the animal had an otherwise normal AMPK pathway component.

In another embodiment, the animal has at least one cell which has enhanced level of AMPK pathway activity. The animal can have enhanced levels of activity by pharmaceutical intervention, by genetic alteration (e.g., overproducing a positively acting axis component) or epigenetically (e.g., inhibiting an inhibitor of the axis) and so forth. The animal can be used to identify compounds that further inhibit disease, e.g., act synergistically with enhanced AMPK pathway activity therapy.

Biomarkers

In one aspect, the invention provides for the identification of biomarkers which are associated with altered AMPK pathway activity, and optionally also can (a) distinguish chronological age from biological, (b) can be assayed with a non-invasive specimen (e.g., blood, urine, skin, saliva, etc.), (c) possess appropriate dynamic range across age spans of interest and (d) are conserved among distinct species. In one embodiment, candidate biomarkers are identified by comparing global gene expression of cells, tissues, organs and organisms among wild type and organisms deficient in AMPK pathway activity, e.g., at the same chronological ages. In one embodiment, model organisms such as yeast, flies, nematode worms, i.e.., organisms with a lifespan less than 90 days, are used. Candidate biomarkers homologous to markers in the model organisms can then be tested, e.g., in mice and humans, via transcriptional profiling of relevant cells, tissues and organs or *in silico* analyses of gene expression databases. Or the biomarkers can be identified using murine and other mammalian systems (including human) directly. The markers may alone or in combination reliably distinguish AMPK pathway activity in an organism, and may also distinguish chronological vs. biological age across the life span of an organism, e.g., a human or mouse, possess one or more of the other desirable properties listed above, and provide surrogates for judging efficacy of life span extending drug candidates.

The term "average lifespan" refers to the average of the age of death of a cohort of organisms. In some cases, the "average lifespan" is assessed using a cohort of genetically identical organisms under controlled environmental conditions. Deaths due to mishap are discarded. For example, with respect to a nematode population, hermaphrodites that die as a result of the "bag of worms" phenotype are typically discarded. A variety of criteria can be used to determine whether organisms are of the "same" chronological age for the comparative analysis. Typically, the degree of accuracy required is a function of the average lifespan of a wild-type organism. For example, for the nematode *C. elegans,* for which the laboratory wild-type strain N2 lives an average of about 16 days under some controlled conditions, organisms of the same age may have lived for the same number of days. For mice, organism of the same age may have lived for the same number of weeks or months; for primates or humans, the same number of years (or within 2, 3, or 5 years); for *Drosophila,* the same number of weeks; and so forth. Generally, organisms of the same chronological age may have lived for an amount of time within 15, 10, 5, 3, 2 or 1% of the average lifespan of a wild-type organism of that species. In a preferred embodiment, the organisms are adult organisms, e.g. the organisms have lived for at least an amount of time in which the average wild-type organism has matured to an age at which it is competent to reproduce.

To identify a biomarker, a property associated with a candidate biomolecule in one organism is compared to the property of the corresponding biomolecule in the other organism, e.g., the organism with deficient or enhanced AMPK activity.

In one embodiment, the biomolecule is a nucleic acid molecule, which can include a DNA molecule (e.g. genomic DNA or cDNA generated from RNA), or RNA molecules (e.g. mRNA, tRNA, untranscribed RNAs). The nucleic acid molecule can be single-stranded or double-stranded. The nucleic acid molecule can be isolated or purified prior to analysis. If a nucleic acid molecule is identified as a biomarker, a variety of tools can be used to analyze subsequent samples. These tools include a probe or primer that is complementary to the nucleic acid molecule, a plasmid that includes the nucleic acid molecule, a host cell that can produce a protein encoded by the nucleic acid molecule, and a computer record that associates the nucleic acid molecule with a property corresponding to it in a particular sample. An isolated or purified nucleic acid molecule includes a nucleic acid molecule that is substantially free of other biomolecules present in the natural source of the nucleic acid. For example, a probe is an isolated nucleic acid molecule (although it may be present with other selected probes).

In another embodiment, the biomolecule is a protein (e.g., a polypeptide). An antibody or other ligand that specifically binds to the protein can be used to detect the protein. In many cases, a transcript which functions as a biomarker encodes a protein that is also a biomarker, and vice versa. In still other embodiments, the biomolecule is a polysaccharide (e.g. glucose, glycosaminoglycan), a lipid (e.g. phospholipid, sphingolipid, cholesterol), or other molecule, e.g., a metabolite or other compound (e.g., superoxide).

To identify a biomarker, a property associated with a biomolecule in the first organism is compared to a property associated with the corresponding molecule in the second organism. In one embodiment, the property is abundance. Abundance of a biomolecule can be binary (e.g., present or absent), semi-quantitative (e.g., absent, low, medium, high), or quantitative. In another embodiment, the property is chemical composition. For example, with respect to protein biomolecules, this property can refer to post-translational modification state. Examples of post-translational modifications include glycosylation, phosphorylation, sulfation, ubiquitination, acetylation, lipidation, prenylation, and proteolytic cleavage. Modifications can be specific to a particular amino acid position in the protein. Chemical composition also includes substrate-product transformations. For example, a particular compound may be found in the first organism, but present in modified form (e.g., product) in the second organism. The property can also refer to enzymatic activity. For a biomolecule that is an enzyme, it may have certain catalytic parameters (e.g., Kcat, Km, substrate specificity, allostery) in the first organism and other parameters in the second organism. In another embodiment, the property can be physical association with another biomolecule. In yet another embodiment, the property can refer to subcellular location of the biomolecule (e.g. ER, Golgi, cytosolic, nuclear, lysosomal, endosomal, plasma membrane, and extracellular matrix). Methods to evaluate these properties are described below or are known.

Generally, the property of the particular biomolecule is evaluated in the first and the second organisms. The respective properties are compared to determine if they have a preselected relationship. For example, for quantitative properties, they may differ by a preselected amount. The preselected amount can be any arbitrary value, and may not be known prior to the comparison, provided that the value is discrete and reproducible, e.g., for many comparisons of identical subjects or samples. Statistical significance can also be used to assess whether a preselected relationship is significant. Exemplary statistical tests include the Students T-test and log-rank analysis. Some statistically significant relationships have a P value of less than 0.05, or 0.02.

If the properties differ between the first and second organisms by a qualitatively or quantitatively detectable extent, then values (e.g., qualitative or quantitative values) are identified that are associated alterations in AMPK pathway activity.

Exemplary methods for evaluating biomolecules for the function as a marker of the aging process are described below and elsewhere herein.

In one embodiment, the organism has a short average lifespan (e.g., less than 5, 3, or 2 years or less than 10, 6, or 1 month). The organism can be a model organism, e.g., a well characterized organism that can be breed and maintained under laboratory conditions. In addition, the model organism may also have a genome that is well characterized, e.g., genetically mapped and sequenced. Examples of such organisms include yeast (e.g., *S. cerevisiae),* flies (e.g., *Drosophila),* fish (e.g., zebrafish), nematodes (e.g., *C. elegans* and *C. briggsae),* and mammals (e.g., rodents (such as mice)).

As seen, biomarkers can be identified by of an organism of one genotype with an organism of a second genotype. As used herein, the term "genotype" refers to the genetic composition of an individual. The first and second genotypes can be two different naturally occurring genotypes. In another embodiment, the genotype of the first organism is wild-type and the genotype of the second organism is mutant in a gene encoding an AMPK pathway component. In still another embodiment, both genotypes are mutant, e.g., one is mutant in an AMPK pathway component, the other in an age-associated gene. "Wild-type," as used herein, refers to a reference genotype, including a genotype that predominates in a natural population or laboratory population of organisms as compared to natural or laboratory mutant forms. The lifespan phenotype of an average wild-type organism is necessarily a normal lifespan for the species.

An organism with a mutant genotype includes at least one genetic alteration, typically altering an endogenous gene of the organism. Such genetic alterations can be mapped. Examples of genomic alterations associated with mutant forms include point mutations, deletions, insertions, chromosomal rearrangements, transposon insertions, and retroviral insertions. In some particular embodiments, the genotype includes an alteration that results from an exogenous nucleic acid, e.g., a synthetic gene deletion construct, a transgene that inserted by recombination, an exogenous gene on an episome inserted by transformation, an exogenously introduced transposon or an exogenously introduced retroviral sequence. Genetic alterations can arise spontaneously; they can be present in a natural population at a low frequency (e.g., less than 5 or 2%); they can be generated in the laboratory (e.g., by exposure to mutagens or recombinant nucleic acids; see below).

A variety of methods can be used to identify biomolecular markers that are associated with altered AMPK pathway activity and/or lifespan regulation. Typically, a plurality ofbiomolecules are evaluated for the first and second organism that differ in AMPK pathway activity levels. The property of each biomolecule is identified in the respective organisms. Properties that are detectably different identify the particular biomolecule as a marker, or at least a candidate biomarker.

**Nucleic Acid Markers.** In many embodiments, transcripts are analyzed from the two organisms. One method for comparing transcripts uses nucleic acid microarrays that include a plurality of addresses, each address having a probe specific for a particular transcript. Such arrays can include at least 100, or 1000, or 5000 different probes, so that a substantial fraction, e.g., at least 10, 25, 50, or 75% of the genes in an organism are evaluated mRNA can be isolated from a sample of the organism or the whole organism. The mRNA can be reversed transcribed into labeled cDNA. The labeled cDNAs are hybridized to the nucleic acid microarrays. The arrays are detected to quantitate the amount of cDNA that hybridizes to each probe, thus providing information about the level of each transcript.

Methods for making and using nucleic acid microarrays are well known. For example, nucleic acid arrays can be fabricated by a variety of methods, e.g., photolithographic methods (see, e.g., U.S. 5,143,854; U.S. 5,510,270; and U.S. 5,527,681), mechanical methods (e.g., directed-flow methods as described in U.S. 5,384,261), pin based methods (e.g., as described in U.S. 5,288,514), and bead based techniques (e.g., as described in PCT US/93/04145). The capture probe can be a single-stranded nucleic acid, a double-stranded nucleic acid (e.g., which is denatured prior to or during hybridization), or a nucleic acid having a single-stranded region and a double-stranded region. Preferably, the capture probe is single-stranded. The capture probe can be selected by a variety of criteria, and preferably is designed by a computer program with optimization parameters. The capture probe can be selected to hybridize to a sequence rich (e.g., non-homopolymeric) region of the nucleic acid. The $T_m$ of the capture probe can be optimized by prudent selection of the complementarity region and length. Ideally, the $T_m$ of all capture probes on the array is similar, e.g., within 20, 10, 5, 3, or 2˚C of one another. A database scan of available sequence information for a species can be used to determine potential cross-hybridization and specificity problems.

The isolated mRNA from samples for comparison can be reversed transcribed and optionally amplified, e.g., by rtPCR, e.g., as described in (U.S. 4,683,202). The nucleic acid can be labeled during amplification, e.g., by the incorporation of a labeled nucleotide. Examples of preferred labels include fluorescent labels, e.g., red-fluorescent dye Cy5 (Amersham) or green-fluorescent dye Cy3 (Amersham), and chemiluminescent labels, e.g., as described in U.S. 4,277,437. Alternatively, the nucleic acid can be labeled with biotin, and detected after hybridization with labeled streptavidin, e.g., streptavidin-phycoerythrin (Molecular Probes).

The labeled nucleic acid can be contacted to the array. In addition, a control nucleic acid or a reference nucleic acid can be contacted to the same array. The control nucleic acid or reference nucleic acid can be labeled with a label other than the sample nucleic acid, e.g., one with a different emission maximum. Labeled nucleic acids can be contacted to an array under hybridization conditions. The array can be washed, and then imaged to detect fluorescence at each address of the array.

A general scheme for producing and evaluating profiles can include the following. The extent of hybridization at an address is represented by a numerical value and stored, e.g., in a vector, a one-dimensional matrix, or one-dimensional array. The vector x has a value for each address of the array. For example, a numerical value for the extent of hybridization at a first address is stored in variable $x_a$. The numerical value can be adjusted, e.g., for local background levels, sample amount, and other variations. Nucleic acid is also prepared from a reference sample and hybridized to an array (e.g., the same or a different array), e.g., with multiple addresses. The vector y is construct identically to vector x. The sample expression profile and the reference profile can be compared, e.g., using a mathematical equation that is a function of the two vectors. The comparison can be evaluated as a scalar value, e.g., a score representing similarity of the two profiles. Either or both vectors can be transformed by a matrix in order to add weighting values to different nucleic acids detected by the array.

The expression data can be stored in a database, e.g., a relational database such as a SQL database (e.g., Oracle or Sybase database environments). The database can have multiple tables. For example, raw expression data can be stored in one table, wherein each column corresponds to a nucleic acid being assayed, e.g., an address or an array, and each row corresponds to a sample. A separate table can store identifiers and sample information, e.g., the batch number of the array used, date, and other quality control information.

Other methods for quantitating nucleic acid species include: quantitative RT-PCR. In addition, two nucleic acid populations can be compared at the molecular level, e.g., using subtractive hybridization or differential display.

In addition, once a set of nucleic acid transcripts are identified as being associated with AMPK pathway activity, it is also possible to develop a set of probes or primers that can evaluate a sample for such markers. For example, a nucleic acid array can be synthesized that includes probes for each of the identified markers.

**Protein Analysis**. The abundance of a plurality of protein species can be determined in parallel, e.g., using an array format, e.g., using an array of antibodies, each specific for one of the protein species. Other ligands can also be used. Antibodies specific for a polypeptide can be generated by known methods.

Methods for producing polypeptide arrays are described, e.g., in De Wildt et al., (2000) Nature Biotech. 18: 989-994; Lueking et al., (1999) Anal. Biochem. 270:103-111; Ge, H. (2000) Nucleic Acids Res. 28:e3, I-VII; MacBeath and Schreiber, (2000) Science 289, 1760-1763; Haab et al., (2001) Genome Biology 2(2):research0004.1; and WO 99/51773A1. A low-density (96 well format) protein array has been developed in which proteins are spotted onto a nitrocellulose membrane Ge, H. (2000) Nucleic Acids Res. 28, e3, I-VTI). A high-density protein array (100,000 samples within 222 X 222 mm) used for antibody screening was formed by spotting proteins onto polyvinylidene difluoride (PVDF) (Lueking et al. (1999) Anal. Biochem. 270, 103-111). Polypeptides can be printed on a flat glass plate that contained wells formed by an enclosing hydrophobic Teflon mask (Mendoza, et al. (1999). Biotechniques 27, 778-788.). Also, polypeptide can be covalently linked to chemically derivatized flat glass slides in a high-density array (1600 spots per square centimeter) (MacBeath, G., and Schreiber, S.L. (2000) Science 289, 1760-1763). De Wildt *et al.*, describe a high-density array of 18,342 bacterial clones, each expressing a different single-chain antibody, in order to screening antibody-antigen interactions (De Wildt et al. (2000). Nature Biotech. 18, 989-994). These art-known methods and other can be used to generate an array of antibodies for detecting the abundance of polypeptides in a sample. The sample can be labeled, e.g., biotinylated, for subsequent detection with streptavidin coupled to a fluorescent label. The array can then be scanned to measure binding at each address and analyze similar to nucleic acid arrays.

**Mass Spectroscopy.** Mass spectroscopy can also be used, either independently or in conjunction with a protein array or 2D gel electrophoresis. For 2D gel analysis, purified protein samples from the first and second organism are separated on 2D gels (by isoelectric point and molecular weight). The gel images can be compared after staining or detection of the protein components. Then individual "spots" can be proteolyzed (e.g., with a substrate-specific protease, e.g., an endoprotease such as trypsin, chymotrypsin, or elastase) and then subjected to MALDI-TOF mass spectroscopy analysis. The combination of peptide fragments observed at each address can be compared with the fragments expected for an unmodified protein based on the sequence of nucleic acid deposited at the same address. The use of computer programs (e.g., PAWS) to predict trypsin fragments, for example, is routine in the art. Thus, each address of spot on a gel or each address on a protein array can be analyzed by MALDI. The data from this analysis can be used to determine the presence, abundance, and often the modification state of protein biomolecules in the original sample. Most modifications to proteins cause a predictable change in molecular weight.

**Other methods.** Other methods can also be used to profile the properties of a plurality of protein biomolecules. These include ELISAs and Western blots. Many of these methods can also be used in conjunction with chromatographic methods and in situ detection methods (e.g., to detect subcellular localization).

**Other Biomolecules**. Other biomolecules (e.g., other than proteins and nucleic acids) can be detected by a variety of methods include: ELISA, antibody binding, mass spectroscopy, enzymatic assays, chemical detection assays, and so forth.

The following examples are merely illustrative of particular aspects of the invention described herein.

Example: Identification of AMPK alpha, beta and gamma-subunit homologs in *C. elegans*

Using Blast we identified homologs in *C. elegans* of the three subunits of AMPK: AMPK alpha, AMPK beta and AMPK gamma. Significant homologies were identified as follows:

**Table 3: Exemplary C. elegans AMPK Components**

| Protein | *C. elegans* gene |
| --- | --- |
| AMPK alpha | *aka-1* (T01C8.1) and *aka-2* (PAR2.3) |

(continued)

| Protein | *C. elegans* gene |
|---------|-------------------|
| AMPK beta | F55F3.1 and Y47D3A. 15 |
| AMPK gamma | T20F7.6, Y111B2A.8 and Y41G9A.3 |

*aka-1* and *aka-2* have a high degree of homology throughout the length of their predicted proteins to the human AMPKα subunits (PRKAA1 and PRKAA2). We named these genes AMP activated protein kinase alpha subunit 1 and subunit 2 (*aka-2* for PAR2.3). The full length amino acid sequence encoded by each gene was determined in part from an analysis of full length cDNAs for each gene. The kinase domains of AKA-1 and AKA-2 share 71% and 80% amino acid identity, respectively, with the kinase domain of the human AMPKα1 subunit.

Example: Identification of AMPK alpha T01 C8.1 as an antagonist of the Insulin Receptor/daf-2 in *C. elegans*

The *daf-2*(e1368) mutant is temperature sensitive. At 20°C, *daf-2(e1368)* animals develop normally into adulthood, passing through the L3 larval stage. At the restrictive temperature of 25°C, *daf-2(el368)* animals arrest development as dauers, an alternative 3rd stage larval form that does not advance to adulthood until exit from the dauer form. The constitutive entry into dauer is termed a Daf-c phenotype. We tested the AMPK subunit homologs identified above for antagonism of daf-2. We inactivated each of subunit homolog by RNAi (RNA interference) and determined if the RNAi treatment affected the ability of *daf-2(e1368)* mutants to develop as dauers at 25°C.

RNAi was prepared and administered as follows: specific primers to PCR-amplify a fragment of each gene were designed with the program Primer3 using C. *elegans* genomic DNA as a template, a T7 polymerase promoter (TAATACGACTCACTATAGGG, SEQ ID NO:10) was added 5' to each primer. After PCR amplification of each gene-fragment, double-stranded RNA (dsRNA) was generated by in vitro transcription with the T7 RNA Polymerase followed by an annealing reaction. The mutation *rrf-3(pk1426)* has been shown to improve the efficiency of RNAi and does not affect dauer formation. We soaked L4 stage *daf-2(e1368); rrf-3(pk1426)* animals in a dsRNA solution for 24 hours. We prepared RNAi for each of T01C8.1, PAR2.3, F55F3.1, Y47D3A.15,T20F7.6, Y111B2A.8, Y41 G9A.3 or no dsRNA control (i.e. Blank). After the 24 hours, we determined if the progeny of these animals arrest development as dauers or not, when grown at 25°C. We found that RNAi of T01C8.1 caused *daf-2(e1368); rrf-3(pk1426)* animals to not arrest as dauers. Instead, at least 80% of these animals grew to adulthood.

Thus like RNAi treatment with RNAi specific for daf- 18/PTEN, RNAi specific for T01C8.1(AMPKalpha) suppresses the Daf-c phenotype of daf-2(e1368) animals. Thus, T01C8.1 antagonizes insulin signaling in C. *elegans*.

We confirmed these results by a different RNAi method: injection RNAi. To do this, we injected dsRNA into prefertile daf 2(e1368); rrf-3(pk1426) adults, and examined their progeny. We found that reduction of *aka-1* gene activity by injection RNAi suppressed the Daf-c phenotype of *daf-2(e1368)* at 25°C but not at 27°C. This indicates that *aka-1* normally promotes dauer formation by antagonizing the activity of the insulin/IGF-1 pathway. In contrast, *aka-2(RNAi)* did not have an effect, either alone or in combination with *aka-1(RNAi)*.

Table 4: Temperature Dep. RNAi Phenotype

| | % Dauer | |
|---|---|---|
| | 25˚C | 27˚C |
| mock RNAi | approx >80%] | 100 |
| aka-1 RNAi | approx <10%] | 100 |
| aka-2 RNAi | approx >80%] | 100 |
| aka-1 and aka-2 RNAi | approx = <10%] | 100 |
| RNAi's were injected into a daf-2(e1368); rrf-3(pk1426) adults. | | |

We obtained *aka-1(ok524;* T01C8.1) from the Caenorhabditis Genetics Center, this mutant allele of T01C8.1 deletes 409 nucleotides of sequence between exon 3 and intron 3, resulting in an insertion of a stop codon and a truncated protein that lacks a complete kinase, inhibitory, and AMPKβγ-binding domains; therefore, *ok524* is predicted to be a molecular null.

We constructed double mutants between T01C8.1(ok524) and multiple daf-2 mutant alleles, and age-1 mutant allele, and evaluated the ability of these animals to develop as dauers or grow to adulthood. We found that in all cases T01C8.1 suppressed the daf-c phenotype of the daf-2 or age-1 mutants (See Table 5).

We also constructed a double mutant between T01C8.1 (ok524) and daf-7(e1372). daf-7 is a homolog of the Transforming Growth Factor-beta. Daf-7 mutants are Daf-c, but do not affect lifespan. We found that T01C8.1 enhanced the Daf-c phenotype of daf-7 (e1372) mutants. This indicates that T01C8.1 plays a complex role in the regulation of dauer formation, antagonizing insulin signalling and promoting TGF-beta signalling. In addition, this results indicates that T01C8.1 plays a specific role in each signaling pathway.

We found that *aka-1 (ok524)* did not cause dauer formation at temperatures ranging from 15˚C to 27˚C. Then, we determined the Daf-c phenotype of double mutants between *aka-1(ok524)* and a range of weak to strong *daf-2* mutant alleles. In all cases, *aka-1(ok524)* increased the temperature at which half the animals formed dauers , indicating that *aka-1* normally antagonizes the activity of the insulin/IGF-1 pathway. In addition, we found that *aka-1(ok524)* partially suppressed the Daf-c phenotype of mutants in downstream components of the insulin/IGF-1 signal-transduction cascade, including the *age-1* PI3-kinase, and the PDK and AKT homologues *pdk-1* and *akt-1* (Morris et al., 1996; Paradis et al., 1999; Paradis and Ruvkun, 1998), and upstream components thought to regulate insulin secretion, including the CAPS homologue *unc-31,* the syntaxin homologue *unc-64,* and the autosomal recessive polycystic kidney disease gene homologue *osm-5,* which is required for sensory cilium-structure (Ailion et al., 1999; Qin et al., 2001). Strikingly, at 27˚C, *aka-1(ok524)* did not suppress the Daf-c phenotype of the weakest *daf-2* allele tested, indicating that *aka-1* is not essential for the activity of the insulin/IGF-1 pathway, instead *aka-1* functions as a modulator of this pathway.

We also examined the role of *aka-1* in the other two pathways that regulate dauer formation. We found that *aka-1(ok524)* did not affect the Daf-c phenotype of a mutant in the transmembrane guanylyl cyclase *daf-11* (Birnby et al., 2000). This result shows that *aka-1* does not play a role in this pathway and indicates that *aka-1* role in antagonizing the insulin/IGF-1 pathway is specific.

Surprisingly, we found that double mutants between *aka-1(ok524)* and mutants in components of the TGFβ pathway, the *daf-7* TGFβ and the *daf-1* Type I TGFβ receptor (Georgi et al., 1990; Ren et al., 1996), were more likely to form dauers than the single mutants alone. This indicates that *aka-1* normally inhibits dauer formation by promoting the activity of the TGFβ pathway. Together these results show that *aka-1* plays a complex and specific role in the regulation of dauer formation: it antagonizes insulin/IGF-1 signaling and promotes TGFβ signaling.

**Table 5: Percentage Dauer**

| Strain | Temperature | | | | |
|---|---|---|---|---|---|
| | 15˚C | 20˚C | 22.5˚C | 25˚C | 27˚C |
| wild type | 0.0 | 0.0 | 0.0 | 0.0 | 0.0 |
| T01C8.1(ok524) | 0.0 | 0.0 | 0.0 | 0.0 | 0.0 |
| daf-2(e1370) | | 0.0 | 100.0 | 100.0 | 100.0 |
| daf-2(e1370); T01C8.1(ok524) | | 0.0 | 42.4 | 100.0 | 100.0 |
| daf-2(m577) | | 0.0 | 11.1 | 100.0 | 100.0 |
| daf-2(m577); T01C8.1(ok524) | | 0.0 | 0.0 | 85.2 | 100.0 |
| daf-2(e1368) | | 0.0 | 12.0 | 98.4 | 100.0 |
| daf-2(e1368); T01C8.1(ok524) | | 0.0 | 0.0 | 4.7 | 100.0 |
| daf-2(mu150) | | 0.0 | 0.0 | 100.0 | 100.0 |
| daf-2(mu150); T01C8.1(ok524) | | 0.0 | 0.0 | 0.0 | 100.0 |
| age-1(hx546) | | 0.0 | 0.0 | 0.0 | 100.0 |
| age-1 (hx546); T01 C8.1 (ok524) | | 0.0 | 0.0 | 0.0 | 44.4 |
| daf-7(e1372) | 7.8 | 13.0 | 79.3 | 100.0 | 100.0 |
| daf-7(e1372); T01C8.1(ok524) | 79.1 | 94.4 | 100.0 | 100.0 | 100.0 |

Example: AMPK alpha Function in *C. elegans* lifespan

Inactivation of genes that suppress the daf-2 Daf-c phenotype (such as daf-16/FOX0 and daf-18/PTEN) also suppresses the long lifespan (Age phenotype) of daf-2 mutants. RNAi that inactivates T01C8.1 is administered to a daf-2 mutant worm or to a daf-2(e1368), rrf-3(pk1426) mutant worm. The lifespan or a lifespan parameter of the worm is evaluated. We constructed a daf-2(e1368); T01C8.1(ok524) double mutant and compared its lifespan to that of wild-type, daf-2(e1368), and T01C8.1(ok524) controls. We found that T01C8.1 activity in necessary for daf-2(e1368) mutants to be long lived. For example, at the age of 27 days, when greater than 95% of daf-2(e1368) mutants are alive, while less than 20% of daf-2(e1368); T01C8.1(ok524) double mutants are alive at the same age. Also, we found that T01C8.1 (ok524) mutants live shorter than wild type, indicating that T01C8.1 normal function is to promote longevity.

We determined the role that *aka-1* plays in the various pathways that regulate lifespan. Mutations in components of the insulin/IGF-1 pathways cause animals to life up to twice as long as wild type (Guarente and Kenyon, 2000; Kenyon et al., 1993). We found that *aka-1(ok524)* fully suppressed the lifespan extension of *daf-2(m577),* indicating that *aka-1* activity controls lifespan by antagonizing the activity of the insulin/IGF-1 pathway. We also examined the lifespans of double mutants between *aka-1(ok524)* and three stronger *daf-2* mutant alleles (Gems et al., 1998), and found that in all cases *aka-1(ok524)* reduced their lifespan extension by at least 50%. In addition, we found that *aka-1(ok524)* partially supressed the long lifespan of *osm-5* mutants, fully supresed *unc-31* mutants, and also suppressed *age-1* and *pdk-1* mutants, suggesting that *aka-1* functions downstream or in parallel to these genes.

One possibility was that *aka-1* activity was solely required to promote the activity of the FOXO homologue *daf-16,* a transcription factor that is a downstream component of the insulin/IGF-1 signaling pathway (Lin et al., 1997; Ogg et al., 1997). If this were the case, lack of *aka-1* activity should not reduce the lifespans of *daf-16* null animals. We tested this hypothesis by comparing the lifespans of *daf 16(mu86); aka-1(ok524)* animals to those of the single mutants, and found that the double mutants lived shorter than the single mutants, even though they both contained null alleles in those genes. Thus, at least in part, *aka-1* promotes longevity in a *daf-16*-independent manner.

We also determined whether *aka-1* activity was required for two other pathways that control lifespan in a *daf-*

*16*-dependent manner: the Sir2 and germline pathways. Animals carrying a transgene that increases the gene-dosage of the NAD-dependent protein deacetylase homologue *sir-2.1* have lifespans that are 50 % longer than those of controls (Tissenbaum and Guarente, 2001). We found that *aka-1 (ok524)* suppressed the majority of the lifespan extension caused by this transgene. Thus, the ability of *sir-2.1* to promote longevity is largely, but not fully, dependent on *aka-1* activity.

Animals lacking germline stem-cells are also long lived in a *daf-16*-dependent manner (Arantes-Oliveira et al., 2002; Hsin and Kenyon, 1999). In order to determine whether *aka-1* played a role in this pathway, we constructed double mutants between *aka-1 (ok524)* and either *mes-1* mutants, which have no germline, or *glp-1 (or178)* mutants, which have a reduced number of germline stem-cells when grown at their restrictive temperature only during adulthood. We found lack of *aka-1* activity very modestly shortened the lifespan of these mutants. This indicates that germline stem-cells do not requiere *aka-1* activity to control lifespan, and that *aka-1* plays a role in some, but not all, *daf-16*-dependent pathways.

Mutations that impair mitochondrial function also extend lifespan (Feng et al., 2001; Wong et al., 1995). To determine whether *aka-1* played a role in this pathway, we constructed double mutants between *aka-1 (ok524)* and either *isp-1,* which encodes an iron sulphur protein of mitochondrial complex III (Feng et al., 2001), or *clk-1,* which encodes a mitochondrial hydroxylase required for ubiquinone biosynthesis (Ewbank et al., 1997), and found that they had lifespans intermediate between those of *aka-1(ok524)* and the corresponding mitochondrial mutant. These results suggest that *isp-1* and *clk-1* mutants are long lived, in part, because of *aka-1* activity.

The last pathway we tested is defined by the *eat* mutants, which affect the function of the pharynx, the feeding organ, and are thought to mimic calorie-restriction by limiting food intake (Lakowski and Hekimi, 1998). We constructed double mutants between *aka-1 (ok524)* and either a strong or a weak *eat-2* mutant, and found that lack of *aka-1* activity did not affect the lifespan of these mutants. This indicates that the lifespan extension caused by *eat-2* mutants does not require *aka-1* activity. Together, the results in this section indicate that *aka-1* plays a broad and specific role in the regulation of lifespan, and that it participates in both insulin-dependent and insulin-independent pathways.

**Table 6**

| Strain/treatment | Mean $\pm$ s.e.m. (days) | P |
|---|---|---|
| Lifepans at 20˚C | | |
| wild type | 20.4 $\pm$ 0.3 | |
| aka-1(ok524) | 17.6 $\pm$ 0.2 | < 0.0001 |
| daf-2(m577) | 24.2 $\pm$ 0.5 | < 0.0001 |
| daf-2(m577); aka-1(ok524) | 17.2 $\pm$ 0.5 | 0.8603 |
| | | < 0.0001 |
| daf-2(mu150) | 24.2 $\pm$ 0.7 | < 0.0001 |
| daf-2(mu150); aka-1(ok524) | 19.5 $\pm$ 0.4 | < 0.0001 |
| | | < 0.0001 |
| daf-2(e1368) | 36.7 $\pm$ 0.8 | < 0.0001 |
| daf-2(e1368); aka-1(ok524) | 25.1 $\pm$ 0.5 | < 0.0001 |
| | | < 0.0001 |
| daf-2(e1370) | 43.2 $\pm$ 1.3 | < 0.0001 |
| daf-2(e1370); aka-1(ok524) | 29.4 $\pm$ 1.0 | < 0.0001 |
| | | < 0.0001 |
| age-1(hx546) | 31.6 $\pm$ 1.2 | < 0.0001 |
| age-1(hx546); aka-1(ok524) | 22.8 $\pm$ 0.6 | < 0.0001 |
| | | < 0.0001 |
| pdk-1(sa709) | 25.1 $\pm$ 0.8 | < 0.0001 |
| pdk-1(sa709); aka-1(ok524) | 21.0 $\pm$ 0.6 | < 0.0001 |
| | | < 0.0001 |
| unc-31(e928) | 30.9 $\pm$ 1.0 | < 0.0001 |
| unc-31(e928); aka-1(ok524) | 17.9 $\pm$ 0.5 | 0.2911 |
| | | < 0.0001 |
| osm-5(p813) | 49.0 $\pm$ 1.5 | < 0.0001 |
| osm-5(p813); aka-1 (ok524) | 31.7 $\pm$ 0.7 | < 0.0001 |
| | | < 0.0001 |

(continued)

| Strain/treatment | Mean ± s.e.m. (days) | P |
|---|---|---|
| daf-16(mu86) | 17.1 ± 0.3 | 0.1135 |
| daf-16(mu86); aka-1(ok524) | 14.6 ± 0.2 | < 0.0001 |
| | | < 0.0001 |
| geIn3[sir-2.1 (+)] | 28.0 ± 0.7 | < 0.0001 |
| geIn3[sir-2.1(+)]; aka-1(ok524) | 20.7 ± 0.4 | < 0.0001 |
| | | < 0.0001 |
| isp-1(qm150) | 30.3 ± 1.4 | < 0.0001 |
| isp-1(qm150); aka-1(ok524) | 22.6 ± 0.7 | < 0.0001 |
| | | < 0.0001 |
| clk-1(qm30) | 24.5 ± 0.6 | < 0.0001 |
| clk-1(qm30); aka-1(ok524) | 20.0 ± 0.2 | < 0.0001 |
| | | < 0.0001 |
| eat-2(ad1116) | 23.0 ± 0.7 | < 0.0001 |
| eat-2(ad1116); aka-1(ok524) | 25.9 ± 0.8 | < 0.0001 |
| | | 0.0065 |
| eat-2(ad465) | 22.2 ± 0.8 | < 0.0001 |
| eat-2(ad465); aka-1(ok524) | 20.7 ± 0.8 | 0.1685 |
| | | < 0.0001 |
| Lifespans at 15°C from egg to L4 molt, 20°C during adulthood | | |
| wild type | 18.5 ± 0.3 | |
| aka-1(ok524) | 16.0 ± 0.2 | < 0.0001 |
| glp-1(or178) | 23.6 ± 0.6 | < 0.0001 |
| glp-1(or178); aka-1(ok524) | 21.5 ± 0.5 | < 0.0001 |
| | | 0.0011 |

Example: Time of action of T01C8.1 AMPKalpha in lifespan

Components of the insulin/IGF-1 pathway, including *daf-2* and *daf-16,* function during adulthood to regulate lifespan (Arantes-Oliveira et al., 2002; Dillin et al., 2002a). In contrast, mitochondrial components specify the lifespan of the organism during development and not during adulthood (Dillin et al., 2002b). If T01C8.1/AMPKalpha affects aging, it is possible that it does so by affecting some aspect of development (for example changing some aspect of the animal that is set during development into adulthood, such as the number of cells), alternatively T01C8.1/AMPKalpha may affect lifespan even after development to adulthood has occurred. To investigate when *aka-1* functions to control lifespan, we reduced *aka-1* activity using feeding RNAi at specific times during the life of the animal. We cultured *daf-2(e1368)* animals on bacteria expressing *aka-1* double-stranded RNA either throughout life or only during adulthood, and found that both treatments suppressed their longevity, and that the magnitude of the effect declined slightly in the adult-only *aka-1(RNAi).* This indicates that *aka-1* is required in the adult to promote longevity.

**Table 7**

| Strain/treatment | Mean ± s.e.m. (days) | P |
|---|---|---|
| Lifespans of rrf-3(pk1426); daf-2(e1368) grown at 20°C on HT115 bacteria expressing dsRNA for: | | |
| Vector control | 33.4 ± 0.7 | |
| aka-1 | 26.7 ± 0.4 | < 0.0001 |
| aka-2 | 30.9 ± 0.9 | 0.0157 |
| aka-1 and aka-2 | 23.1 ± 0.6 | < 0.0001 |
| | | < 0.0001 |
| | | < 0.0001 |

Example: *aka-1* activity is necessary and sufficient to regulate the lifespans of wild-type animals

We measured the lifespan of *aka-1(ok524)* animals and found that they were short lived relative to wild-type controls, indicating that *aka-1* is required for normal lifespan. We examined whether *aka-1(ok524)* animals had any other obvious defects that might influence their lifespans and found that they fed and moved normally, and had normal brood sizes, indicating that *aka-1 (ok524)* animals are healthy. We then asked whether an increase in *aka-1* gene-dosage would increase the lifespans of otherwise wild-type animals. We generated multiple transgenic lines containing the *aka-1* genomic region and a transformation marker and found that these animals lived 15 % longer than wild-type controls. In contrast, transgenic lines that contained only the transformation marker had lifespans indistinguishable from those of wild-type animals. Together, these results indicate that *aka-1* activity is necessary and sufficient to regulate the lifespans of wild-type animals.

**Table 8**

| Strain/treatment | Mean ± s.e.m. (days) | P |
|---|---|---|
| Lifepans at 20˚C | | |
| wild type | 20.4 ± 0.3 | |
| aka-1(ok524) | 17.6 ± 0.2 | < 0.0001 |
| Control transgene. Line 1 | 19.9 ± 0.6 | 0.6922 |
| Control transgene. Line 1 | 20.0 ± 0.5 | 0.5436 |
| Control transgene. Line 1 | 19.6 ± 0.8 | 0.9083 |
| Control transgene. Line 1 | 20.1 ± 0.4 | 0.4795 |
| aka-1 genomic region transgene. Line 1 | 22.9 ± 0.5 | < 0.0001 |
| aka-1 genomic region transgene. Line 1 | 22.1 ± 0.7 | 0.0003 |
| aka-1 genomic region transgene. Line 1 | 22.2 ± 0.5 | < 0.0001 |
| aka-1 genomic region transgene. Line 1 | 22.3 ± 0.7 | < 0.0001 |

Example: Role of aka-1 in heat tolerance

Many of the mutations that extend lifespan also confer increased thermotolerance (Lithgow et al., 1995). In addition, an increased gene-dosage of the small heat shock protein HSP-16 leads to increased thermotolerance and lifespan extension (Walker and Lithgow, 2003). To determine whether *aka-1* is required for thermotolerance, we compared the lifespans at 35˚C of wild type and *aka-1(ok524)* animals, and found that *aka-1 (ok524)* animals lived shorter than wild-type controls. In addition, we noticed that *aka-1 (ok524)* animals became paralyzed before they die at high temperature, while wild-type animals did not. This paralysis could be due to an increase in the sensitivity to heat induced neuromuscular-defects, or, alternatively, may reflect the inability of myosin heads to walk on actin fibers, perhaps due to ATP depletion. Together, these results show that *aka-1* promotes thermotolerance and prevents heat induced paralysis.

Example: Activation of T01C8.1/AMPKalpha and dauer formation and/or extend lifespan

We found that inactivation of T01C8.1/AMPKalpha is necessary for dauer formation to occur in *daf-2(e1368)* animals. Transgenic animals that carry a gene encoding a truncated form of T01C8.1/AMPKalpha that is constitutively active (according to Crute et al., J. Biol. Chem (1998) 273:35347-35354) are constructed. They are evaluated for lifespan or dauer pathway control.

Example: Assay for functional conservation of AMPK-alpha subunits

Inactivation of T01C8.1/AMPKalpha suppresses the Daf-c phenotype of *daf-2 (e1368)*. Transgenic animals are constructed that express a nematode gene encoding T01C8.1/AMPKalpha in a genetic background that is deficient in T01C8.1/AmPKalpha activity (i.e., *daf-2(e1368), T01C8.1⁻*). The animals are evaluated for lifespan or dauer pathway. Complementation by the transgene is indicated by restoration of the daf-c phenotype.

Transgenic animals are constructed that express a mammalian gene encoding AMPKalpha (particularly human and murine alpha) in this genetic background that is deficient in T01C8.1/AMPKalpha activity (i.e., *daf-2(e1368), T01C8.1).* These animals are similarly evaluated.

Example: Assay for regulation of daf-16/FOXO nuclear localization

daf-16 nuclear localization is regulated by daf-2 (Lin et al, Nature Genetics). daf-16 nuclear localization is assayed by detecting daf-16-GFP using fluorescence microscopy in animals lacking T01C8.1 activity.

Examples: Assay expression and subcellular localization

We prepare a nucleic acid construct that encodes a T01C8.1/AMPKalpha amino acid sequence fused to GFP. The construct includes 5' regulatory sequences from the T01C8.1 gene. Similar constructs with other tags (FLAG, 6-HIS, myc) are also used. Transgenic nematodes or other animals or cells that include the construct are generated. GFP (or the tag) is localized in cells by fluorescence microscopy. Other versions of the construct are prepared for nematode β and γ AMPK subunits.

Example: Purification of recombinant AMPK / or liver AMPK

An antibody specific to the peptide PGLKPHPERMPPLI (SEQ ID NO:11) is used to immunoprecipitate α2 AMPK and α1 AMPK from human cells. The antibody is coupled to protein A-SEPHAROSE™ beads. A protein extract from the human cells is bound to the beads, then the beads are washed with PBS. The protein is eluted from the beads using the PGLKPHPERMPPLI (SEQ ID NO:12) peptide. See, e.g., Carling et al. (1994) J. Biol. Chem. 269:11442).

Example: AMPK Kinase Assay

Eluted protein is combined with [$^{32}$P]ATP and the 15-amino acid "SAMS" peptide: HMRSAMSGLHLVKRR (SEQ ID NO:7) and incubated at 20˚C or 37˚C. After a fixed time, the reaction is quenched, and the peptide precipitated by addition of 40% TCA. Counts in the precipitated material are measured in a scintillation counter.

Example: AMPK Kinase Assay

The above AMPK kinase assay is performed in a microtitre plate. Different test compounds are included in wells of the plate. Two wells include buffer instead of a test compound. Results of the assay are downloaded to a computer and stored in a table of an SQL database (e.g., Microsoft Access®). The database is searched to identify compounds that reduce kinase activity by at least 40%.

Example: AMPK activity altering compounds

Compounds such as Metformin and rosiglitazone have been shown to stimulate AMPK activity. These compounds are administered to a test organism, such as *C. elegans,* to determine if they alter the lifespan of the organism. The compounds are also administered to an organism that includes an endogenous T01C8.1 activity and to an organism with deficient T01C8.1 activity. The deficient organisms can be generated by genetic mutation or RNAi treatment. Accordingly, the effect of these compounds and the T01C8.1 dependence of the effect are determined. In addition, the effect of the compounds on dauer formation is also tested. Any test compound or a library of compounds can be tested by this method.

Examples: *aka-1* Promotes Longevily During Larval Development and Adulthood

Components of the insulin/IGF-1 pathway, including *daf-2* and *daf-16,* and signals from the germline function during adulthood to regulate lifespan (Arantes-Oliveira et al., 2002; Dillin et al., 2002a). In contrast, mitochondrial components specify the lifespan of the organism during larval development and not during adulthood (Dillin et al., 2002b). To investigate when *aka-1* functions to control lifespan, we reduced *aha-1* activity by RNAi at specific times during the life of the animal. We cultured *rrf-3(pk1426); daf-2(e1368)* animals on bacteria expressing *aka-1* double-stranded RNA starting at hatching, starting at the beginning of adulthood, or starting at either six or ten days after the beginning of adulthood. We found that all treatments significantly shorten the lifespan of *rrf-3(pk1426); daf-2(e1368)* mutants, except for the one starting ten days after the beginning of adulthood. In addition, we found that as the age when the treatment starts increases the magnitude of the lifespan shortening progressively decreases. Strikingly, *aka-1(RNAi)* is effective even when the treatment starts six days after the beginning of adulthood, when reproduction is complete. These results indicate that *aka-1* functions to promote longevity both during larval development and adulthood.

*rrf-3(pk1426); daf-2(e1368)* animals were grown on control bacteria (vector only) or transferred from control bacteria onto bacteria expressing *aka-1* dsRNA at the indicated age. The fraction of animals remaining alive was plotted against animal age.

**Table 9**

| Strain/treatment | Mean $\pm$ s.e.m. (days) | P |
|---|---|---|
| Lifespans of rrf-3(pk1426); daf-2(e1368) grown at 20˚C on HT115 bacteria expressing dsRNA for: | | |
| Vector control | 33.4 $\pm$ 0.7 | |
| aka-1 | 26.7 $\pm$ 0.4 | < 0.0001 |
| Vector control from egg to L4 molt. | 29.8 $\pm$ 0.4 | < 0.0001 |
| aka-1 starting at day 0 of adulthood | | < 0.0001 |
| Vector control from egg to day 6 of adulthood. | 31.3 $\pm$ 0.5 | < 0.0001 |
| aka-1 starting at day 6 of adulthood | | |
| Vector control from egg day 10 of adulthood. | 32.5 $\pm$ 0.7 | 0.0348 |
| aka-1 starting at day 10 of adulthood | | |

### Site of *aka-1* Action for the Regulation of Dauer Formation and Lifespan

Two components of the insulin/IGF-1 pathway, *daf-2* and *age-1*, function cell nonautonomously in multiple sets of signaling cells, including neuronal and non-neuronal cell types to regulate dauer formation and lifespan (Apfeld and Kenyon, 1998; Wolkow et al., 2000). To determine the tissues where *aka-1* functions to control dauer formation and lifespan, we used tissue-specific promoters to express an *aka-1* cDNA in the neurons, muscle, or intestine of *daf-2 (e1368); aka-1(ok524)* animals. We then examined the development and lifespan of these transgenic animals.

First, we found that transgenes containing a complete *aka-1* genomic fragment or transgenes expressing the *aka-1* cDNA in all cells cause *daf-2(e1368); aka-1(ok524)* animals to form dauers between 90 % and 100 % of the time at 25˚C. This indicates that these transgenes restore *aka-1* activity effectively. Second, we found that restoring *aka-1* activity by expressing an *aka-1* cDNA solely in the neurons or in the body wall muscles of *daf-2(e1368); aka-1(ok524)* animals causes them to form dauers 15 % of the time. These dauers exhibit the characteristic remodeling of the pharynx and epidermis, even though these tissues lack *aka-1* activity. Therefore, *aka-1* activity in either neurons or body wall muscles can be sufficient to promote dauer development even in tissues that lack *aka-1* activity. Thus, *aka-1* functions cell nonautonomusly to promote dauer formation. In contrast, expression of the *aka-1* cDNA in the intestine of *daf-2 (e1368); aka-1(ok524)* animals does not lead to dauer formation. Together, these results indicate that *aka-1* functions cell nonautonomously in a subset of tissues to antagonize insulin/IGF-1 signaling in dauer formation.

We then measured the lifespans at 20˚C of the various *aka-1* transgenic animals. Control transgenes containing a complete *aka-1* genomic fragment or expressing the *aka-1* cDNA in all cells extend the lifespans of *daf-2(e1368); aka-1 (ok524)* animals by 35 % and 21 %, representing 77 % and 45 % rescue, respectively. In contrast, expressing *aka-1* cDNA only in the neurons, body wall muscle, or intestine of *daf-2(e1368); aka-1(ok524)* does not extend lifespan. These results are consistent with *aura-1* functioning cell autonomously to promote longevity: if *aka-1* controls the longevity of each tissue independently of one another, then restoring *aka-1* activity in one tissue will not extend the lifespans of other tissues and the animal will not be long lived. Alternatively, *aka-1* may function cell nonautonomously, but it may be necessary to restore *aka-1* activity in multiple or different tissues to extend the lifespan of *daf-2(e1368); aka-1(ok524)* animals.

**Table 10**

| Strain/treatment | Mean $\pm$ s.e.m. (days) | P |
|---|---|---|
| Lifespans at 20˚C wild type | 20.4 $\pm$ 0.3 | |
| aka-1(ok524) | 17.6 $\pm$ 0.2 | < 0.0001[c] |
| Control transgene. Line 1 | 19.9 $\pm$ 0.6 | 0.6922[c] |
| Control transgene. Line 1 | 20.0 $\pm$ 0.5 | 0.5436[c] |
| Control transgene. Line 1 | 19.6 $\pm$ 0.8 | 0.9083[c] |
| Control transgene. Line 1 | 20.1 $\pm$ 0.4 | 0.4795[c] |
| aka-1 genomic region transgene. Line 1 | 22.9 $\pm$ 0.5 | < 0.0001[c] |
| aka-1 genomic region transgene. Line 1 | 22.1 $\pm$ 0.7 | 0.0003[c] |
| aka-1 genomic region transgene. Line 1 | 22.2 $\pm$ 0.5 | < 0.0001[c] |

(continued)

| Strain/treatment | Mean $\pm$ s.e.m. (days) | P |
|---|---|---|
| aka-1 genomic region transgene. Line 1 | 22.3 $\pm$ 0.7 | < 0.0001[c] |

Here is a table with all the lifespan data and the legend for the p values:

**Table 11: Statistical Analysis of Adult Lifespans.**

| Strain/treatment | Mean $\pm$ s.e.m. (days) | P |
|---|---|---|
| Lifepans at 20˚C | | |
| wild type | 20.4 $\pm$ 0.3 | |
| aka-1(ok524) | 17.6 $\pm$ 0.2 | < 0.0001[c] |
| Control transgene. Line 1 | 19.9 $\pm$ 0.6 | 0.6922[c] |
| Control transgene. Line 1 | 20.0 $\pm$ 0.5 | 0.5436[c] |
| Control transgene. Line 1 | 19.6 $\pm$ 0.8 | 0.9083[c] |
| Control transgene. Line 1 | 20.1 $\pm$ 0.4 | 0.4795[c] |
| aka-1 genomic region transgene. Line 1 | 22.9 $\pm$ 0.5 | < 0.0001[c] |
| aka-1 genomic region transgene. Line 1 | 22.1 $\pm$ 0.7 | 0.0003[c] |
| aka-1 genomic region transgene. Line 1 | 22.2 $\pm$ 0.5 | < 0.0001[c] |
| aka-1 genomic region transgene. Line 1 | 22.3 $\pm$ 0.7 | < 0.0001[c] |
| daf-2(m577) | 24.2 $\pm$ 0.5 | < 0.0001[d] |
| daf-2(m577); aka-1(ok524) | 17.2 $\pm$ 0.5 | 0.8603[d] |
| | | < 0.0001[e] |
| daf-2(mu150) | 24.2 $\pm$ 0.7 | < 0.0001[d] |
| daf-2(mu150); aka-1(ok524) | 19.5 $\pm$ 0.4 | < 0.0001[d] |
| | | < 0.0001[e] |
| daf-2(e1368) | 36.7 $\pm$ 0.8 | < 0.0001[d] |
| daf-2(e1368); aka-1(ok524) | 25.1 $\pm$ 0.5 | < 0.0001[d] |
| | | < 0.0001[e] |
| daf-2(e1370) | 43.2 $\pm$ 1.3 | < 0.0001[d] |
| daf-2(e1370); aka-1(ok524) | 29.4 $\pm$ 1.0 | < 0 .0001[d] |
| | | < 0.0001[e] |
| age-1(hx546) | 31.6 $\pm$ 1.2 | < 0.0001[d] |
| age-1(hx546); aka-1(ok524) | 22.8 $\pm$ 0.6 | < 0.0001[d] |
| | | < 0.0001[e] |
| pdk-1(sa709) | 25.1 $\pm$ 0.8 | < 0.0001[d] |
| pdk-1(sa709); aka-1(ok524) | 21.0 $\pm$ 0.6 | < 0.0001[d] |
| | | < 0.0001[e] |
| unc-31(e928) | 30.9 $\pm$ 1.0 | < 0.0001[d] |
| unc-31(e928); aka-1(ok524) | 17.9 $\pm$ 0.5 | 0.2911[d] |
| | | < 0.0001[e] |
| osm-5(p813) | 49.0 $\pm$ 1.5 | < 0.0001[d] |
| osm-5(p813); aka-1(ok524) | 31.7 $\pm$ 0.7 | < 0.0001[d] |
| | | < 0.0001[e] |
| daf-16(mu86) | 17.1 $\pm$ 0.3 | 0.1135[d] |
| daf-16(mu86); aka-1(ok524) | 14.6 $\pm$ 0.2 | < 0.0001[d] |
| | | < 0.0001[e] |
| geIn3[sir-2.1(+)] | 28.0 $\pm$ 0.7 | < 0.0001[d] |
| geIn3[sir-2.1(+)]; aka-1(ok524) | 20.7 $\pm$ 0.4 | < 0.0001[d] |
| | | < 0.0001[e] |
| isp-1(qm150) | 30.3$\pm$1.4 | < 0,0001[d] |
| isp-1(qm150); aka-1(ok524) | 22.6 $\pm$ 0.7 | < 0.0001[d] |

(continued)

| Strain/treatment | Mean ± s.e.m. (days) | P |
|---|---|---|
| | | < 0.0001[e] |
| clk-1(qm30) | 24.5 ± 0.6 | < 0.0001[d] |
| clk-1(qm30); aka-1(ok524) | 20.0 ± 0.2 | < 0.0001[d] |
| | | < 0.0001[e] |
| eat-2(ad1116) | 23.0±0.7 | < 0.0001[d] |
| eat-2(ad1116); aka-1(ok524) | 25.9± 0.8 | < 0.0001[d] |
| | | 0.0065[e] |
| eat-2(ad465) | 22.2±0.8 | < 0.0001[d] |
| eat-2(ad465); aka-1(ok524) | 20.7± 0.8 | 0.1685[d] |
| | | < 0.0001[c] |
| Lifespans at 15°C from egg to L4 molt, 20°C during adulthood | | |
| wild type | 18.5 ± 0.3 | |
| aka-1(ok524) | 16.0+0.2 | < 0.0001[c] |
| glp-1(or178) | 23.6 ± 0.6 | < 0.0001[d] |
| glp-1(or178); aka-1(ok524) | 21.5 ± 0.5 | < 0.0001[d] |
| | | 0.0011[e] |
| Lifespans of rrf-3(pk1426); daf-2(e1368) grown at 20°C on HT115 bacteria expressing dsRNA for: | | |
| Vector control | 33.4 ± 0.7 | |
| aka-1 | 26.7 ± 0.4 | < 0.0001[f] |
| aka-2 | 30.9±0.9 | 0.0157[f] |
| aka-1 and aka-2 | 23.1 ± 0.6 | < 0.0001[f] |
| | | < 0.0001[g] |
| | | < 0.0001[h] |
| Vector control from egg to L4 molt. aka-1 starting at day 0 of adulthood | 29.8 ± 0.4 | < 0.0001[f] |
| | | < 0.0001[g] |
| Vector control from egg to day 6 of adulthood. aka-1 starting at day 6 of adulthood | 31.3 ± 0.5 | < 0.0001[f] |
| Vector control from egg day 10 of adulthood. aka-1 starting at day 10 of adulthood | 32.5±0.7 | 0.0348[f] |
| Lifespans at 20°C of daf-2(e1368); aka-1 (ok524) transgenic animals | | |
| No transgene | 25.1 ± 0.5 | |
| Transformation marker only. Line 1 | 26.0 ± 0.6 | 0.0586[i] |
| Transformation marker only. Line 2 | 24.6 ± 1.0 | 0.6975[i] |
| Transformation marker only. Line 3 | 25.7±0.6 | 0.1118[i] |
| aka-1 genomic region. Line 1 | 35.5 ± 0.9 | < 0.0001[i] |
| aka-1 genomic region. Line 2 | 34.3 ± 0.6 | < 0.0001[i] |
| aka-1 genomic region. Line 3 | 33.6 ± 0.7 | < 0.0001[i] |
| aka-1 genomic region. Line 4 | 32.6 ± 0.9 | < 0.0001[i] |
| aka-1 cDNA expressed in all cells (dpy-30 promoter). Line 1 | 29.8 ± 0.6 | < 0.0001[i] |
| aka-1 cDNA expressed in all cells (dpy-30 promoter). Line 2 | 32.8 ± 0.8 | < 0.0001[i] |
| aka-1 cDNA expressed in all cells (dpy-30 promoter). Line 3 | 29.5 ± 0.7 | < 0.0001[i] |
| aka-1 cDNA expressed in neurons (unc-119 promoter). Line 1 | 26.9 ± 0.5 | 0.0189[i] |
| aka-1 cDNA expressed in neurons | 24.6 ± 0.7 | 0.8376[i] |

(continued)

| Strain/treatment | Mean ± s.e.m. (days) | P |
|---|---|---|
| (unc-119 promoter). Line 2 aka-1 cDNA expressed in neurons (unc-119 promoter). Line 3 | 24.8 ± 0.5 | 0.8479[i] |
| aka-1 cDNA expressed in body muscle (myo-3 promoter). Line 1 | 23.5 ± 0.7 | 0.2951[i] |
| aka-1 cDNA expressed in body muscle (myo-3 promoter). Line 2 | 24.5 ± 0.8 | 0.9328[i] |
| aka-1 cDNA expressed in gut (elt-2 promoter). Line 1 | 24.4 ± 0.5 | 0.6933[i] |
| aka-1 cDNA expressed in gut (elt-2 promoter). Line 2 | 25.9 ± 0.5 | 0.0659[i] |
| aka-1 cDNA expressed in gut (elt-2 promoter). Line 3 | 24.5 ± 0.7 | 0.9396[i] |

[b] The total number of observations equals the number of animals that died plus the number censored. Animals that crawled off the plate, exploded, or bagged were censored at the time of the event. This step incorporated those animals until the censor date, and was necessary to avoid the loss of information; for example, if a 50-day-old animal crawls off the plate, it is important to include that information in the data set, as the animal was long lived. Control and experimental animals were cultured in parallel and transferred to new plates at the same time. We used JMP 5 (SAS) software for statistical analysis and to determine means and percentiles. The logrank (Mantel-Cox) test was used to test the hypothesis that the survival functions among groups were equal (Lawless, 1982). P values were calculated for individual experiments consisting of control and experimental animals examined at the same time.

[c] Compared with wild type.

[d] Compared with *aka-1(ok524)*.

[e] Compared with the single mutant corresponding to the mutation other than *aka-1(ok524)* present in the double mutant

[f] Compared with vector control in the same genetic background.

[g] Compared with *aka-1* RNAi in the same genetic background.

[h] Compared with *aka-2* RNAi in the same genetic background.

[i] Compared with *daf-2(e1368); aka-1(ok524)* with no transgene.

**Example: Measurement of AMP/ATP ratio with age:**

To determine if the AMP/ATP ratio changed with age, we measured the levels of AMP, ADP, and ATP directly. We lysed 30 adult worms of the appropriate age, grown at the indicated temperature, lysed them, and separated the extract by Reversed Phase HPLC, measuring the Absorbance 260nm. We used a C. elegans strain that lacks sperm at 25C [CF512: fem-1(-); fer-15(-)]. We found that the AMP/ATP ratio increases with age.

**Table 12**

| | AMP/ATP | | |
|---|---|---|---|
| age (days) | Average | StdDev | n |
| 1 | 0.701 | 0.110 | 4 |
| 2 | 0.517 | 0.024 | 3 |
| 3 | 0.545 | 0.047 | 2 |
| 5 | 0.556 | 0.056 | 2 |
| 7 | 0.871 | 0.098 | 2 |
| 9 | 1.306 | 0.038 | 2 |
| 11 | 1.716 | 0.239 | 4 |
| 13 | 2.302 | 0.161 | 4 |
| 15 | 3.390 | 0.652 | 3 |

| (continued) | | | |
|---|---|---|---|
| | AMP/ATP | | |
| age (days) | Average | StdDev | n |
| 17 | 5.148 | 1.132 | 4 |

### Example: AMP/ATP ratio predicts remaining age potential

To determine if the AMP/ATP ratio predicts the remaining lifespan of the animal, we separated CF512 13 day old adult worms grown at 25C into 2 groups. The first group consisted of animals that moved spontaneously (Group 1), while the second group consisted of animals that moved only in response to prodding (Group 2). Each group was further subdivided at random into two subgroups. For one of these subgroups the AMP/ATP ratio was determined, for the other subgroup the remaining lifespan was measured. We then compared the AMP/ATP ratio and lifespan of group 1 and group 2 animals to those of control animals consisting of live CF512 13 day old adult worms that were not categorized based on the degree of movement.

We found that Group 1 animals have a lower AMP/ATP ratio than control animals, whereas Group 2 animals have a higher AMP/ATP ratio.

**Table 13**

| age (days) | AMP/ATP | | |
|---|---|---|---|
| | Average | StdDev | n |
| Group 1: "moves spontaneously" | 2.00499 | 0.0262 | 2 |
| Group 2: "moves in response to prodding" | 3.46658 | 0.2853 | 3 |
| Control | 2.30158 | 0.1608 | 4 |

We also found that Group 1 animals live longer than controls, while Group 2 animals lived shorter than controls.

Together, these findings indicate that the AMP/ATP ratio serves as a biomarker, since it predicts the remaining lifespan of the animals.

### Example: Effect of Starvation on the AMP/ATP ratio

We found that starvation caused the AMP/ATP ratio to increase. This increase is proportional to the length of time under starvation In addition, in starved animals, the AMP/ATP ratio returns to normal after an hour of feeding. Also, in aka-1(oak524) the AMP/ATP ratio is higher under normal feeding conditions, and it further increases upon starvation.

**Table 14**

| | age (days) | AMP/ATP | |
|---|---|---|---|
| | | Average | StdDev |
| wild type | Fed | 0.9555 | 0.0559 |
| | Starved 6 hours | 1.2906 | 0.0152 |
| | Starved 9hours | 1.4513 | 0.0708 |
| | Starved 9h, then fed 1 hour | 0.9504 | 0.0962 |
| aka-1(ok524) | Fed | 1.1514 | 0.0696 |
| | Starved 6 hours | 1.6765 | 0.0894 |

### Example: Effect of Sodium Azide on the AMP/ATP ratio

We found that exposure to 1 mM sodium azide caused the AMP/ATP ratio to increase. In addition, in the treated animals, the AMP/ATP ratio returns to normal an hour after growth conditions without sodium azide.

**Table 15**

| age (days) | Treatment | AMP/ATP | | |
| --- | --- | --- | --- | --- |
| | | Average | StdDev | n |
| N2 | none | 0.96 | 0.06 | 5 |
| | 1 mM Azide 1 hour | 2.08 | | 1 |
| | 1 mM Azide 4 hours | 2.53 | 0.43 | 2 |
| | 1 mM Azide 4 hours, then no azide 1 hour | 1.08 | 0.21 | 2 |
| ok524 | none | 1.15 | 0.07 | 3 |
| | 1 mM Azide 1 hour | 3.19 | | 1 |
| | 1 mM Azide 4 hours | 4.34 | 0.45 | 2 |
| | 1 mM Azide 4 hours, then no azide 1 hour | 1.24 | 0.14 | 2 |
| | 1mM Azide 4 hours, then no azide 2 hour | 1.29 | 0.11 | 2 |

We also found that aka-1(ok524) animals are more sensitive than wild type animals to killing by exposure to 1 mM sodium azide for 18 hours.

**Example: Effect of High Temperature on the AMP/ATP ratio**

We found that exposure to 35˚C caused the AMP/ATP ratio to increase. In addition, this treatment causes the AMP/ATP ratio to increase faster in aka-1(ok524) animals than in wild type.

**Example: Effect of Mutants that Affect Lifespan and Age on the AMP/ATP ratio at 20 ˚C**

We found that the AMP/ATP ratio increases with age (except from day 1 to day 2, where it decreases). In aka-1(ok524) animals the AMP/ATP ratio is higher, therefore aka-1 promotes a low AMP/ATP ratio. daf-2(e1368) and daf-2(e1370) have lower AMP/ATP ratios than wild type. Therefore daf-2 normally promotes a higher AMP/ATP ratio. In addition, daf-2(e1370) and daf-2(e1370); aka-1(ok524) animals have both equally elevated AMP/ATP ratios compared to wild type, therefore the effect of loss of daf-2 function on the AMP/ATP ratio is dependent on aka-1 activity. isp1- and clk-1 mutants, which affect oxidative phosphorylation, have a higher AMP/ATP ratio than wild type, consistent with a role in ATP production. The effect of isp-1 on the AMP/ATP ratio not solely due to aka-1 inactivation, since isp-1; aka-1 double mutants have a much higher AMP/ATP ratio than either single mutant. Therefore aka-1 functions in parallel to isp-1 to promote. a low AMP/ATP ratio. eat-2 mutants, which have feeding defects, have a higher AMP/ATP ratio than wild type. The effect eat-2 is not solely due to aka-1 inactivation, since eat-2; aka-1 double mutants have a much higher AMP/ATP ratio than either single mutant. Therefore aka-1 functions in parallel to eat-2 to promote a low AMP/ATP ratio. daf-16, which is required for the longevity of daf-2 mutants, does not affect the AMP/ATP ratio.

**Example: Higher accumulation of lipofuscin in aka-1 mutants**

Lipofuscin is a fluorescent pigment that accumulates in the gut with age and therefore serves as a biomarker. Me measured lipofuscin-like autofluorescence in the anterior portion of the gut and found that the rate of increase in this variable in was higher in aka-1 mutants than in wild type animals. Since aka-1 animals live shorter than wild type and accumulate lipofuscin at a faster rate, we conclude that they age faster than wild type.

**Table 16**
**Gut Autofluorescence**

| Age | wild type | aka-1(ok524) |
| --- | --- | --- |
| 1 | 314.9 $\pm$ 24.2 | 276.6 $\pm$46.5 |
| 4 | 609.8 $\pm$ 88.8 | 718.7 $\pm$ 100.2 |
| 7 | 795.9 $\pm$ 83.0 | 1088.4 $\pm$ 190.6 |

**Claims**

1. A method of evaluating a compound, the method comprising:

   - contacting a polypeptide *in vitro* with a test compound, wherein the polypeptide comprises a sequence at least 85 % identical to (i) an alpha AMPK subunit, (ii) a beta AMPK subunit, (iii) a gamma AMPK subunit, or (iv) a functional domain thereof;
   - evaluating an interaction between the test compound and the polypeptide;
   - contacting a cell or nori-human organism that produces the polypeptide with the test compound; and
   - evaluating a rate of aging of the cell or non-human organism

   wherein an increase in AMPK activity is indicative of the ability of the test compound to enhance the lifespan of the cell or non-human organism.

2. The method of claim 1, wherein evaluating the rate of aging comprises one or more of:

   (a) assessing the life span of the cell or the organism;
   (b) assessing the presence or abundance of a gene transcript or gene product in the cell or organism that has a biological age-dependent expression pattern;
   (c) evaluating resistance of the cell or organism to stress;
   (d) evaluating one or more metabolic parameters of the cell or organism;
   (e) evaluating the proliferative capacity of the cell or a set of cells present in the organism; and
   (f) evaluating physical appearance or behaviour of the cell or organism,
   particularly wherein the evaluating comprises evaluating AMP, ADP, or ATP, more particularly wherein the evaluating comprises evaluating an AMP-ATP ratio.

3. The method of claim 1 wherein the polypeptide comprises a mammalian amino acid sequence of at least 100 amino acids, particularly wherein the polypeptide comprises a human amino acid sequence of at least 100 amino acids.

4. The method of claim 1 wherein evaluating the interaction between the test compound and the polypeptide comprises evaluating binding of the test compound to the polypeptide; or
   wherein evaluating the interaction between the test compound and the polypeptide comprises evaluating a biological activity of the polypeptide, particularly wherein evaluating the interaction between the test compound and the polypeptide comprises evaluating a kinase activity of the polypeptide, more particularly wherein the *in vitro* contacting comprises forming a reaction mixture that includes the test compound, the polypeptide, a substrate, and ATP and evaluating at least one component of the mixture or parameter of the mixture.

5. The method of claim 1,

   (a) wherein at least 60 % of the expected normal life span of the organism has elapsed prior to the organism being contacted with the test compound;
   (b) wherein evaluating the rate of aging comprises measuring the life span of one or more organisms contacted with the test compound;
   (c) wherein a statistical value descriptive of life span for a group of genetically matched organisms contacted with the test compound is measured and that statistical value is compared to a corresponding statistical value descriptive of life span for a control group of genetically matched organisms that have not been contacted with the test compound, particularly wherein the statistical value comprises an average or a median;
   (d) wherein the rate of aging of the cell is determined; or
   (e) further comprising repeating the method with one or more additional compounds to thereby evaluate a library of test compounds.

6. The method of claim 1, wherein the cell is isolated from an organism that has been contacted with the test compound; particularly wherein the cell is contacted with the test compound in vitro; or particularly wherein the cell is a transgenic cell, more particularly wherein the transgenic cell comprises a transgene encoding a protein that comprises a mammalian polypeptide at least 80 % homologous to human AMPK polypeptide, or more particularly wherein the transgenic cell comprises a transgene encoding a protein that comprises a human AMPK polypeptide.

7. A method of evaluating a protein, the method comprising

(a) identifying a candidate protein, wherein the candidate protein comprises a sequence of at least 100 amino acids that is at least 50 % identical to an AMPK subunit or is at least 30 % identical to an AMPK kinase domain;
(b) identifying one or more polymorphisms in the gene that encodes the candidate protein; and
(c) evaluating relationships between the presence of one or more of the polymorphisms and the longevity of the non-human organism that contains the polymorphism to identify a correlation between the one or more polymorphisms and longevity of the organism, thereby evaluating the protein.

**Patentansprüche**

1. Verfahren zum Evaluieren einer Verbindung, wobei das Verfahren umfasst:

   - In-Kontakt-Bringen eines Polypeptids *in vitro* mit einer Testverbindung, wobei das Polypeptid eine Sequenz umfasst, die mindestens 85 % identisch mit (i) einer alpha-AMPK-Untereinheit, (ii) einer beta-AMPK-Untereinheit, (iii) einer gamma-AMPK-Untereinheit oder (iv) einer funktionellen Domäne davon ist;
   - Evaluieren einer Wechselwirkung zwischen der Testverbindung und dem Polypeptid;
   - In-Kontakt-Bringen einer Zelle oder eines nichtmenschlichen Organismus', die/der das Polypeptid herstellt, mit der Testverbindung; und
   - Evaluieren einer Geschwindigkeit des Altems der Zelle oder des nichtmenschlichen Organismus',

   wobei ein Anstieg an AMPK-Aktivität ein Hinweis auf die Fähigkeit der Testverbindung ist, die Lebenserwartung der Zelle oder des nicht menschlichen Organismus' zu erhöhen.

2. Verfahren nach Anspruch 1, wobei das Evaluieren der Geschwindigkeit des Altems eines oder mehrere umfasst von:

   (a) Abschätzen der Lebenserwartung der Zelle oder des Organismus';
   (b) Abschätzen der Anwesenheit oder der Menge eines Gentranskripts oder Genprodukts in der Zelle oder dem Organismus, welches ein biologisches altersabhängiges Expressionsmuster hat;
   (c) Evaluieren des Widerstands der Zelle oder des Organismus' gegenüber Stress;
   (d) Evaluieren eines oder menrerer Stoffwechselparameter der Zelle oder des Organismus';
   (e) Evaluieren der Fähigkeit der Zelle oder eines Satzes an Zellen, die im Organismus vorhanden sind, zu proliferieren; und
   (f) Evaluieren der physikalischen Erscheinung oder des Verhaltens der Zelle oder des Organismus',
   insbesondere wobei das Evaluieren ein Evaluieren von AMP, ADP oder ATP umfasst, weiter insbesondere wobei das Evaluieren ein Evaluieren eines AMP-ATP - Verhältnisses umfasst.

3. Verfahren nach Anspruch 1, wobei das Polypeptid eine Säuger-Aminosäuresequenz von mindestens 100 Aminosäuren umfasst, insbesondere wobei das Polypeptid eine menschliche Aminosäuresequenz von mindestens 100 Aminosäuren umfasst.

4. Verfahren nach Anspruch 1, wobei das Evaluieren der Wechselwirkung zwischen der Testverbindung und dem Polypeptid ein Evaluieren des Bindens der Testverbindung an das Polypeptid umfasst; oder
   wobei das Evaluieren der Wechselwirkung zwischen der Testverbindung und dem Polypeptid ein Evaluieren einer biologischen Aktivität des Polypeptids umfasst, insbesondere wobei das Evaluieren der Wechselwirkung zwischen der Testverbindung und dem Polypeptid das Evaluieren einer Kinaseaktivität des Polypeptids umfasst, weiter insbesondere wobei das *In-vitro*-in-Kontakt-Bringen das Bilden eines Reaktionsgemisches umfasst, das die Testverbindung, das Polypeptid, ein Substrat und ATP einschließt, und Evaluieren von mindestens einem Bestandteil der Mischung oder eines Parameters der Mischung.

5. Verfahren nach Anspruch 1,

   (a) wobei mindestens 60 % der erwarteten normalen Lebenserwartung des Organismus' verstrichen ist, bevor der Organismus mit der Testverbindung in Kontakt gebracht wird;
   (b) wobei das Evaluieren der Geschwindigkeit des Alterns das Messen der Lebenserwartung von einem oder mehreren Organismen umfasst, die mit der Testverbindung in Kontakt gebracht werden;
   (c) wobei ein statistischer Wert gemessen wird, der beschreibend ist für die Lebenserwartung für eine Gruppe von genetisch abgeglichenen Organismen, die mit der Testverbindung in Kontakt gebracht werden, und wobei dieser statistische Wert mit einem entsprechenden statistischen Wert verglichen wird, der beschreibend für die Lebenserwartung für eine Kontrollgruppe von genetisch abgeglichenen Organismen ist, die nicht mit der Testverbindung in Kontakt gebracht wurden, insbesondere wobei der statistische Wert einen Durchschnittswert oder einen Median umfasst;

(d) wobei die Geschwindigkeit des Alterns der Zelle bestimmt wird; oder

(e) weiterhin umfassend das Wiederholen des Verfahrens mit einem oder mehreren zusätzlichen Verbindungen, um **dadurch** eine Bibliothek an Testverbindungen zu evaluieren.

6. Verfahren nach Anspruch 1, wobei die Zelle aus einem Organismus isoliert wird, der mit der Testverbindung in Kontakt gebracht wurde; insbesondere wobei die Zelle mit der Testverbindung *in vitro* in Kontakt gebracht wird; oder insbesondere wobei die Zelle eine transgene Zelle ist, weiter insbesondere wobei die transgene Zelle ein Transgen umfasst, welches ein Protein kodiert, das ein Säugerpolypeptid mindestens 80 % homolog zum menschlichen AMPK-Polypeptid umfasst, oder weiter insbesondere wobei die transgene Zelle ein Transgen umfasst, welches ein Protein kodiert, das ein menschliches AMPK-Polypeptid umfasst.

7. Verfahren zum Evaluieren eines Proteins, wobei das Verfahren umfasst

(a) Identifizieren eines Kandidatenproteins, wobei das Kandidatenprotein eine Sequenz von mindestens 100 Aminosäuren umfasst, die mindestens 50 % identisch mit einer AMPK-Untereinheit ist, oder mindestens 30 % identisch mit einer AMPK-Kinasedomäne ist;

(b) Identifizieren von einem oder mehreren Polymorphismen in dem Gen, das das Kandidatenprotein kodiert; und

(c) Evaluieren von Beziehungen zwischen der Anwesenheit von einem oder mehreren der Polymorphismen und der Langlebigkeit des nicht menschlichen Organismus', der den Polymorphismus enthält, um eine Korrelation zwischen dem einen oder mehreren Polymorphismen und der Langlebigkeit des Organismus' zu identifizieren, wodurch das Protein bewertet wird.

## Revendications

1. Procédé pour évaluer un composé, le procédé comprenant :

- la mise en contact d'un polypeptide *in vitro* avec un composé d'essai, où le polypeptide comprend une séquence identique à au moins 85 % à (i) une sous-unité d'alpha-AMPK, (ii) une sous-unité de bêta-AMPK, (iii) une sous-unité de gamma-AMPK, ou (iv) un domaine fonctionnel de celle-ci ;

- l'évaluation d'une interaction entre le composé d'essai et le polypeptide ;

- la mise en contact d'une cellule ou d'un organisme non humain qui produit le polypeptide avec le composé d'essai ; et

- l'évaluation d'une vitesse de vieillissement de la cellule ou de l'organisme non humain

dans lequel une augmentation d'activité d'AMPK est indicative de la capacité du composé d'essai à accroître la durée de vie de la cellule ou de l'organisme non humain.

2. Procédé selon la revendication 1, dans lequel l'évaluation de la vitesse de vieillissement comprend l'une ou plusieurs parmi :

(a) une estimation de la durée de vie de la cellule ou de l'organisme ;

(b) une estimation de la présence ou l'abondance d'un transcript de gène ou d'un produit de gène dans la cellule ou l'organisme qui présente un profil d'expression dépendant de l'âge biologique ;

(c) une évaluation de la résistance de la cellule ou de l'organisme à un stress ;

(d) une évaluation d'un ou plusieurs paramètres métaboliques de la cellule ou de l'organisme ;

(e) une évaluation de la capacité de prolifération de la cellule ou d'un ensemble de cellules présent(e) dans l'organisme ; et

(f) une évaluation de l'aspect ou du comportement physique de la cellule ou de l'organisme,

en particulier dans lequel l'évaluation comprend une évaluation d'AMP, d'ADP, ou d'ATP, de manière plus particulière dans lequel l'évaluation comprend une évaluation d'un rapport AMP-ATP.

3. Procédé selon la revendication 1, dans lequel le polypeptide comprend une séquence d'acides aminés mammifère d'au moins 100 acides aminés, en particulier dans lequel le polypeptide comprend une séquence d'acides aminés humaine d'au moins 100 acides aminés.

**4.** Procédé selon la revendication 1, dans lequel l'évaluation de l'interaction entre le composé d'essai et le polypeptide comprend une évaluation de la liaison du composé d'essai au polypeptide ; ou dans lequel l'évaluation de l'interaction entre le composé d'essai et le polypeptide comprend une évaluation d'une activité biologique du polypeptide, en particulier dans lequel l'évaluation de l'interaction entre le composé d'essai et le polypeptide comprend une évaluation d'une activité kinase du polypeptide, de manière plus particulière dans lequel la mise en contact *in vitro* comprend une formation d'un mélange réactionnel qui inclut le composé d'essai, le polypeptide, un substrat, et l'ATP et une évaluation d'au moins un composant du mélange ou un paramètre du mélange.

**5.** Procédé selon la revendication 1,

(a) dans lequel au moins 60 % de la durée de vie normale attendue de l'organisme s'est écoulée avant de mettre l'organisme en contact avec le composé d'essai ;
(b) dans lequel l'évaluation de la vitesse de vieillissement comprend une mesure de la durée de vie d'un ou plusieurs organismes mis en contact avec le composé d'essai ;
(c) dans lequel une valeur statistique descriptive de la durée de vie concernant un groupe d'organismes génétiquement appariés mis en contact avec le composé d'essai est mesurée et cette valeur statistique est comparée à une valeur statistique correspondante descriptive de la durée de vie concernant un groupe témoin d'organismes génétiquement réassortis qui n'ont pas été mis en contact avec le composé d'essai, en particulier dans lequel la valeur statistique comprend une moyenne ou une médiane ;
(d) dans lequel la vitesse de vieillissement de la cellule est déterminée ; ou
(e) comprenant de plus une répétition du procédé avec un ou plusieurs composés supplémentaires, évaluant ainsi une bibliothèque de composés d'essai.

**6.** Procédé selon la revendication 1, dans lequel la cellule est isolée d'un organisme qui a été mis en contact avec le composé d'essai ; en particulier dans lequel la cellule est mise en contact avec le composé d'essai *in vitro ;* ou en particulier dans lequel la cellule est une cellule transgénique, de manière plus particulière dans lequel la cellule transgénique comprend un transgène codant pour une protéine qui comprend un polypeptide mammifère au moins 80 % homologue au polypeptide d'AMPK humain, ou de manière plus particulière dans lequel la cellule transgénique comprend un transgène codant pour une protéine qui comprend un polypeptide d'AMPK humain.

**7.** Procédé pour évaluer une protéine, le procédé comprenant

(a) l'identification d'une protéine candidate, où la protéine candidate comprend une séquence d'au moins 100 acides aminés qui est identique à au moins 50 % à une sous-unité d'AMPK ou qui est identique à au moins 30 % à un domaine d'AMPK kinase ;
(b) l'identification d'un ou plusieurs polymorphismes dans le gène qui code pour la protéine candidate ; et
(c) l'évaluation des relations entre la présence d'un ou plusieurs des polymorphismes et la longévité de l'organisme non humain qui contient le polymorphisme pour identifier une corrélation entre le ou les polymorphismes et la longévité de l'organisme, évaluant ainsi la protéine.

hAMPKα1  
hAMPKα2  
AKA-2  
AKA-1  

hAMPKα1    MATAEKQKHDGRVKIGHYILGDTLGVGTFGKVKVGKHLTGHKVAVKILNR  
hAMPKα2    MAEKQKHDGRVKIGHYVLGDTLGVGTFGKVKVGKHQLTGHKVAVKILNR  
AKA-2      MPPSGRFDRTIALAGTGHLKIGNPVIKETIGKGAFGAVKRGTHIQIGYDVAIKILNR  
AKA-1      MFSHQDRDRDRKEDGGGDGTEMKSKSRSQPSGLNRVKNLSRKLSAKSRKERKDRDSTDNSSKMSSPGGETSTKQQGELKAQIKIGHYILKETLGVGTFGKVRVGIHETTQYKVAVKILNR  

hAMPKα1    QKIRSLDVVGKIRREIQNLKLFRHPHIIKLYQVISTPSDIFMVMEYVSGGELFDYICKNGRLDEKESRRLFQQILSGVDYCHRHMVVHRDLKPENVLLDAHMNAKIADFGLSNMMSDGEF  
hAMPKα2    QKIRSLDVVGKIKREIQNLKLFRHPHIIKLYQVISTPTDFFMVMEYVSGGELFDYICKHGRVBEMBARRLFQQILSAVDYCHRHMVVHRDLKPENVLLDAHMNAKIADFGLSNMMSDGEF  
AKA-2      GRMKGLGTVNKIRNEIDNLQKLTPHITRLFRVISTPSDIBLVMELVSGGELPSYITRKCAIPIRBSRRYFQQILSGVDYCHNHMIVHRDLKPENVLLDANKNIKIADFGLSNMMTDGDL  
AKA-1      QKIKSLDVVGKIRREIQNLSLFRHPHITRLYQVISTPSDIFMIMEHVSGGELFDYIVKHCRUKTAPARRFFQQIISGVDYCHRHMVVHRDLKPENVLLDBQNNVKIADFGLSNIMTDGDF  
└ aka-1(ok524) = stop codon created by a 409 bp deletion  

hAMPKα1    LRTSCGSPNYAAPEVISGRLYAGPEVDIWSSGVILYALLCGTLPFDDDHVPTLFKKICDGIFYTPQYLNPSVISLLKHMLQVDPMKRASIKDIREHEWFKQDLPKYLFPEDPSYSSTMID  
hAMPKα2    LRTSCGSPNYTAPEVISGRLYAGPEVDIWSCGVILYALLCGTLPFDDEHVPTLFKKIRGGVFYIPEYLNRSVATLLMHMLQVDPLKRATIKDIREHEWFKQGLPSYLFPEDPSYDANVID  
AKA-2      LSTACGSPNYAAPELISNKLYVGPEVDLHSCGVILYAMLCGTLPFDDQNVPTLFAKIKSGRYTVPYSMBKQAADLISTMLQVDPVKRADVKRIEVNHSWFRIDLPYYLFPECEN-ESSIVD  
AKA-1      LRTSCGSPNYAAPEVISGKLYAGPEVDVWSCGVILYALLCGTLPFDDEHVPSLFRKIKSGVFPTPDFLBRPIVNLLHHMLCVDPMKRATIKDVIAHEWFQKDLPNYLFPPINESEASIVD  

hAMPKα1    DEALKEVCEKFECSEEVLSCLYNRNHQDPIAVAYHLIDNRRTIMNEAKDFYLATSPPD-SFLDDHHLTR----PHPEVPPFLVAETPRARHTLDELNPQKSKHQGVRKAKHIGIRSQ  
hAMPKα2    DEAVKEVCEKFECTESEVMNSLYSGDPQDQLAVAYHLIDNRRIMNQASEFYLASSPPSGSFMDDSAMHIPPGLKPHPERMPPLIADSPKARCPLDALNTTKPKSLAVKKAKWRQGIRSQ  
AKA-2      IDVWQSVAEKFDVKEEDVTGALLABDHHHFLCIAYRLEVNHKRNADESSQKAMEDFWEIGKTMKMGSTSLPVGATTKT---------NVGRKILEGLK------KEQKKLTWNLGIRAC  
AKA-1      IEAVREVTERYHVABEEVTSALLGDDPHHHLSIAYNLIVDNKRIADETAKLSIEBFYQVTPNKGPGPVHR------HPBHIAASVS--SKITPTLD--NTEASGANRNKRAKEHLGIRSQ  

hAMPKα1    SRPNDIMAEVCRAIKQLDYEWKVVNPYYLRVRRK-NPVTSTYSKMSLQLYQVD----SRTYLLDFRSID-DB--ITEAKSGTATPQRSGSVSNYRS-CQRSDS----------------  
hAMPKα2    SKPYDIMAEVYRAMKQLDFEWKVVNAMHLRVRRK-NPVTGNYVKMSLQLYLVD----NRSYLLDFKSID-DE--VVEQRSGSSTPQRSCBAAGLHR-PRSSFD----------------  
AKA-2      LDPVETMKHVPLSLKSVDMEWKVLSMYHIIVRSKPTEINPDPVKVSLQLFALDKKENNKGYLLDFKGLTEDEEAVPPSRCRBRAASVSVTLAKSKSDLNGNSSKVPMSPLSPMSPISPSV  
AKA-1      SRPEDIKFEVFRAMKQLDMEWKVLNPYHVIVRRKPDAPAADPPKMSLQLYQVD----QRSYLLDFKELA-DF--ESGSASAGSSRHASMSMPQKPAGIRGTRT----------------  

hAMPKα1    ---------DABAQGKSSEVSLTS-SVTSLDSSPVDLTBRPG-------SHIIEFBBMCANLIKILAQ-  
hAMPKα2    ---------STTAESHSLSGSLTG-SLT--GSTLSSVSBRLG-------SHIMDFBBMCASLITTLAR-  
AKA-2      NIPKVRVDDADASLKSSLNSSIYMADIBNSMESLDEVSTQSSEPEAPIRSQMEFBATCHIIMQALLAB  
AKA-1      ---------SSMPQAMSMEASIEKMEVHDPSDMSCDVTBPPPSPGGAKL-SQDMQFHEICAALIGTLAR-  

— Kinase domain  
···· Inhibitory domain  
--· AMPKβγ-interaction domain  

FIG. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5010175 A
- WO 9119735 A
- WO 9320242 A
- WO 9200091 A
- US 5288514 A
- US 5539083 A
- US 9610287 W
- US 5593853 A
- US 5569588 A
- US 5549974 A
- US 5525735 A
- US 5519134 A
- US 5506337 A
- US 5223409 A, Ladner
- US 5631169 A, Lakowicz
- US 4868103 A, Stavrianopoulos
- US 4109496 A
- US 5283317 A
- WO 9410300 A
- WO 0112851 A
- US 6406853 B
- US 6343257 B
- US 20020086356 A
- US 20030084471 A
- US 6534261 B
- WO 0042219 A
- US 6511808 B
- US 20020081724 A
- US 4683195 A
- US 4683202 A
- US 4673641 A
- WO 098534 A
- WO 0183827 A
- WO 0212893 A
- WO 0063701 A
- WO 0140803 A
- WO 9951773 A
- US 6066457 A
- US 6132997 A
- US 5716785 A
- US 5130238 A
- US 5409818 A
- US 5554517 A
- US 5854033 A
- US 6143495 A
- US 5455166 A
- US 5624825 A
- US 6294336 B
- US 6013431 A
- US 5952174 A
- US 6066454 A
- US 20020037553 A
- US 4522811 A
- US 5328470 A
- US 20030109476 A
- US 6090919 A
- US 5804387 A
- US 6124128 A
- US 6096865 A
- US 6066476 A
- US 6054321 A
- US 6027881 A
- US 5968750 A
- US 5874304 A
- US 5777079 A
- US 5741668 A
- US 5625048 A
- US 5989835 A
- WO 9951773 A1
- US 5143854 A
- US 5510270 A
- US 5527681 A
- US 5384261 A
- US 9304145 W
- US 6509515 B
- US 5387742 A
- US 5877399 A
- US 6358752 A
- US 6187992 A
- US 9304145 B
- US 4277437 A

**Non-patent literature cited in the description**

- Current Protocols in Molecular Biology. John Wiley & Sons, 1989, 6.3.1-6.3.6
- **BATEMAN et al.** *Nucleic Acids Res.,* 2002, vol. 30 (1), 276-280
- **SONHAMMER et al.** *Proteins,* 1997, vol. 28 (3), 405-420
- **GRIBSKOV et al.** *Meth. Enzymol.,* 1990, vol. 183, 146-159

- **GRIBSKOV et al.** *Proc. Natl. Acad. Sci. USA,* 1987, vol. 84, 4355-4358
- **KROGH et al.** *J. Mol. Biol.,* 1994, vol. 235, 1501-1531
- **STULTZ et al.** *Protein Sci.,* 1993, vol. 2, 305-314
- **R. DURBIN et al.** Biological sequence analysis: probabilistic models of proteins and nucleic acids. Cambridge University Press, 1998
- **CORPET et al.** *Nucl. Acids Res.,* 1999, vol. 27, 263-267
- **ALTSCHUL et al.** *Nucleic Acids Res.,* 1997, vol. 25, 3389-3402
- **GOUZY et al.** *Computers and Chemistry,* 1999, vol. 23, 333-340
- **NEEDLEMAN ; WUNSCH.** *J. Mol. Biol.,* 1970, vol. 48, 444-453
- **MEYERS ; MILLER.** *CABIOS,* 1989, vol. 4, 11-17
- **ALTSCHUL et al.** *J. Mol. Biol.,* 1990, vol. 215, 403-10
- **CRUTE et al.** *J. Biol. Chem.,* 1998, vol. 273, 35347-35354
- **WINDER ; HARDIE.** *Am. J. Physiol.,* vol. 277, E1-E10
- *J. Appl. Physiol.,* 2001, vol. 91, 1017-1028
- **MICHELL et al.** *J. Biol. Chem.,* 1996, vol. 271, 28445
- **FURKA.** *Int. J. Pept. Prot. Res.,* 1991, vol. 37, 487-493
- **HOUGHTON et al.** *Nature,* 1991, vol. 354, 84-88
- **HOBBS et al.** *Proc. Nat. Acad. Sci. USA,* 1993, vol. 90, 6909-6913
- **HAGIHARA et al.** *J. Amer. Chem. Soc.,* 1992, vol. 114, 6568
- **HIRSCHMANN et al.** *J. Amer. Chem. Soc.,* 1992, vol. 114, 9217-9218
- **CHEN et al.** *J. Amer. Chem. Soc.,* 1994, vol. 116, 2661
- **CHO et al.** *Science,* 1993, vol. 261, 1303
- **CAMPBELL et al.** *J. Org. Chem.,* 1994, vol. 59, 658
- **VAUGHN et al.** *Nature Biotechnology,* 1996, vol. 14 (3), 309-314
- **LIANG et al.** *Science,* 1996, vol. 274, 1520-1522
- **DEWITT et al.** *Proc. Natl. Acad. Sci. U.S.A.,* 1993, vol. 90, 6909
- **ERB et al.** *Proc. Natl. Acad. Sci. USA,* 1994, vol. 91, 11422
- **ZUCKERMANN et al.** *J. Med. Chem.,* 1994, vol. 37, 2678
- **CARRELL et al.** *Angew. Chem. Int. Ed. Engl.,* 1994, vol. 33, 2059
- **CARELL et al.** *Angew. Chem. Int. Ed. Engl.,* 1994, vol. 33, 2061
- **GALLOP et al.** *J. Med. Chem.,* 1994, vol. 37, 1233
- **ZUCKERMANN, R.N. et al.** *J. Med. Chem.,* 1994, vol. 37, 2678-85
- **LAM.** *Anticancer Drug Des.,* 1997, vol. 12, 145
- **HOUGHTEN.** *Biotechniques,* 1992, vol. 13, 412-421
- **LAM.** *Nature,* 1991, vol. 354, 82-84
- **FODOR.** *Nature,* 1993, vol. 364, 555-556
- **CULL et al.** *Proc Natl Acad Sci USA,* 1992, vol. 89, 1865-1869
- **SCOTT ; SMITH.** *Science,* 1990, vol. 249, 386-390
- **DEVLIN.** *Science,* 1990, vol. 249, 404-406
- **CWIRLA et al.** *Proc. Natl. Acad. Sci.,* 1990, vol. 87, 6378-6382
- **FELICI.** *J. Mol. Biol.,* 1991, vol. 222, 301-310
- **HARDIE et al.** *Eur J Biochem,* 1997, vol. 246, 259
- **HARDIE et al.** *Annu Rev Biochem,* 1998, vol. 67, 851
- **VAVVAS et al.** *J Biol Chem,* 1997, vol. 272, 13256
- **WINDER et al.** *Am J Physiol Endocrinol Metab,* 1996, vol. 270, E299
- **DAVIES et al.** *Eur. J. Biochem,* 1989, vol. 186, 123
- **NASIR et al.** *Comb Chem HTS,* 1999, vol. 2, 177-190
- **JAMESON et al.** *Methods Enzymol,* 1995, vol. 246, 283
- **SEETHALA et al.** *Anal Biochem.,* 1998, vol. 255, 257
- **PARKER et al.** *Journal of Biomolecular Screening,* 2000, vol. 5, 77-88
- **SJOLANDER, S. ; URBANICZKY, C.** *Anal. Chem.,* 1991, vol. 63, 2338-2345
- **SZABO et al.** *Curr. Opin. Struct. Biol.,* 1995, vol. 5, 699-705
- **RIVAS, G. ; MINTON, A.P.** *Trends Biochem Sci,* 1993, vol. 18, 284-7
- Current Protocols in Molecular Biology. J. Wiley, 1999
- **HEEGAARD, N.H.** *J Mol Recognit,* 1998, vol. 11, 141-8
- **HAGE, D.S. ; TWEED, S.A.** *J Chromatogr B Biomed Sci Appl.,* 1997, vol. 699, 499-525
- **ZERVOS et al.** *Cell,* 1993, vol. 72, 223-232
- **MADURA et al.** *J. Biol. Chem.,* 1993, vol. 268, 12046-12054
- **BARTEL et al.** *Biotechniques,* 1993, vol. 14, 920-924
- **IWABUCHI et al.** *Oncogene,* 1993, vol. 8, 1693-1696
- **NAIKI et al.** *J Biol Chem.,* 19 April 2002, vol. 277 (16), 14011-9

- **UYEDA et al.** *Biochem Pharmacol.,* 15 June 2002, vol. 63 (12), 2075-80
- **GOLUB et al.** *Science,* 1999, vol. 286, 531
- **Y. C. MARTIN.** 3D Database Searching in Drug Design. *J. Med. Chem.,* 1992, vol. 35, 2145
- **A. C. GOOD ; J. S. MASON.** Three Dimensional Structure Database Searches. *Reviews in Comp. Chem.,* 1996, vol. 7, 67
- **Y. C. MARTIN.** Database searching in drug design. *J. Medicinal Chemistry,* 1992, vol. 35, 2145-54
- **FIRE, A.** *Trends Genet.,* 1999, vol. 15, 358-363
- **CLEMENS, J. C. et al.** *Proc. Natl. Sci. USA,* 2000, vol. 97, 6499-6503
- **BILLY et al.** *Proc. Natl. Sci. USA,* 2001, vol. 98, 14428-14433
- **ELBASHIR et al.** *Nature,* 2001, vol. 411 (683 6), 494-8
- **YANG, D. et al.** *Proc. Natl. Acad. Sci. USA,* 2002, vol. 99, 9942-9947
- **REBAR et al.** *Methods Enzymol,* 1996, vol. 267, 129
- **GREISMAN ; PABO.** *Science,* 1997, vol. 275, 657
- **ISALAN et al.** *Nat. Biotechnol,* 2001, vol. 19, 656
- **WU et al.** *Proc. Nat. Acad. Sci. USA,* 1995, vol. 92, 344
- **SAMBROOK ; RUSSELL.** Molecular Cloning: A Laboratory Manual. Cold Spring Harbor Laboratory, 2001
- **KRIEGLER.** Gene Transfer and Expression: A Laboratory Manual. 1990
- Current Protocols in Molecular Biology. 1994
- **GUBLER ; HOFFMAN.** *Gene,* 1983, vol. 25, 263-269
- **BENTON ; DAVIS.** *Science,* 1977, vol. 196, 180-182
- **GRUNSTEIN et al.** *Proc. Natl. Acad. Sci. USA.,* 1975, vol. 72, 3961-3965
- PCR Protocols: A Guide to Methods and Applications. 1990
- **PALVA et al.** *Gene,* 1983, vol. 22, 229-235
- **MOSBACH et al.** *Nature,* 1983, vol. 302, 543-545
- **COLLEY et al.** *J. Biol. Chem.,* 1989, vol. 264, 17619-17622
- Guide to Protein Purification, in Methods in Enzymology. 1990, vol. 182
- **MORRISON.** *J. Bact.,* 1977, vol. 132, 349-351
- **CLARK-CURTISS ; CURTISS et al.** Methods in Enzymology. 1983, vol. 101, 347-362
- C. elegans II. Plainview (NY. Cold Spring Harbor Laboratory Press, 1997
- **SCOPES.** *Protein Purification: Principles and Practice,* 1982
- **COLIGAN.** *Current Protocols in Immunology,* 1991
- **GODING.** Monoclonal Antibodies: Principles and Practice. 1986
- **KOHLER ; MILSTEIN.** *Nature,* 1975, vol. 256, 495-497
- **HUSE et al.** *Science,* 1989, vol. 246, 1275-1281
- **WARD et al.** *Nature,* 1989, vol. 341, 544-546
- **KOHLER ; MILSTEIN.** *Eur. J. Immunol.,* 1976, vol. 6, 511-519
- Basic and Clinical Immunology. 1991
- Enzyme Immunoassay. 1980
- **DE WILDT et al.** *Nat. Biotechnol.,* 2000, vol. 18, 989-994
- **LUEKING et al.** *Anal. Biochem.,* 1999, vol. 270, 103-111
- **GE.** *Nucleic Acids Res.,* 2000, vol. 28, e3, I-VII
- **MACBEATH ; SCHREIBER.** *Science,* 2000, vol. 289, 1760-1763
- **STAPLETON et al.** *FEBS Lett.,* 16 June 1997, vol. 409 (3), 452-6
- **BEAUDET et al.** *Genome Res.,* 2001, vol. 11 (4), 600-8
- **MYERS et al.** *Nature,* 1985, vol. 313, 495-8
- **GANGULY.** *Hum Mutat.,* 2002, vol. 19 (4), 334-42
- **GIBBS et al.** *Nucleic Acids Res.,* 1989, vol. 17, 2437-2448
- **PROSSNER.** *Tibtech,* 1993, vol. 11, 238
- **GASPARINI et al.** *Mol. Cell Probes,* vol. 6, 1
- **BARANY.** *Proc. Natl. Acad. Sci USA,* 1991, vol. 88, 189
- **SAIKI et al.** *Science,* 1985, vol. 230, 1350-1354
- **WU. et al.** *Genomics,* 1989, vol. 4, 560-569
- **BARRINGER et al.** *Gene,* 1990, 117-122
- **F. BARANY.** *Proc. Natl. Acad. Sci. USA,* 1988, 189-193
- **SARKAR et al.** *Science,* 1989, vol. 244, 331-34
- **STOFLER et al.** *Science,* 1988, vol. 239, 491
- **MUDALIAR ; HENRY.** *Annu Rev. Med,* 2001, vol. 52, 239-257
- **MINOKOSHI.** *Nature,* 2002, vol. 415, 339
- **YAMAUCHI et al.** *Nature Medicine,* 2002, vol. 8, 1
- **CRUIKSHANK et al.** *J. Acquired Immune Deficiency Syndromes and Human Retrovirology,* 1997, vol. 14, 193
- **CHEN et al.** *Proc. Natl. Acad. Sci. USA,* 1994, vol. 91, 3054-3057
- **LI et al.** *J. Biol. Chem.,* 1997, vol. 272, 28545-28549
- **HEIM et al.** *Curr. Biol.,* 1996, vol. 6, 178-182

- Spectral Variants of Green Fluorescent Protein. **PALM et al.** Methods Enzymol. 1999, vol. 302, 378-394
- **CORMACK et al.** *Gene,* 1996, vol. 173, 33-38
- **ORMO et al.** *Science,* 1996, vol. 273, 1392-1395
- **HEIKAL et al.** *Proc. Natl. Acad. Sci. USA,* 2000, vol. 97, 11996-12001
- **BOADO et al.** *J. Pharmacol. and Experimental Therapeutics,* 2000, vol. 295 (1), 239-243
- **KAZANTSEV et al.** *Proc. Natl. Acad. Sci. USA,* 1999, vol. 96, 11404-09
- **BROUILLET.** *Functional Neurology,* 2000, vol. 15 (4), 239-251
- **ONA et al.** *Nature,* 1999, vol. 399, 263-267
- **BATES et al.** *Hum Mol Genet.,* 1997, vol. 6 (10), 1633-7
- **HANSSON et al.** *J. of Neurochemistry,* vol. 78, 694-703
- **RUBINSZTEIN, D. C.** *Trends in Genetics,* vol. 18 (4), 202-209
- **MANGIARINI et al.** *Cell,* 1996, vol. 87, 493-506
- **DAVIES et al.** *Cell,* 1997, vol. 90, 537-548
- **CHA et al.** *Proc. Natl. Acad. Sci. USA,* 1998, vol. 95, 6480-6485
- **STEFFAN et al.** *Nature,* 2001, vol. 413, 739-743
- **MARSH et al.** *Human Molecular Genetics,* 2000, vol. 9, 13-25
- **MORLEY et al.** *Proc. Nat. Acad. USA,* 2002, vol. 99, 10417
- *Movement Disorders,* 1996, vol. 11, 136-142
- **MARDER et al.** *Neurology,* 2000, vol. 54, 452-458
- **CLARK ; KARLAWISH.** *Ann. Intern. Med.,* 2003, vol. 138 (5), 400-410
- **TROY et al.** *J. Neurosci.,* vol. 20 (4), 1386-1392
- **LENDON et al.** *JAMA,* 1997, vol. 227, 825
- **NUSSBAUM ; ELLIS.** *New Eng. J. Med.,* 2003, vol. 348 (14), 1356-1364
- **ANDREASEN, N. et al.** *Arch Neurol.,* 2001, vol. 58, 349-350
- **GALASKO, D. et al.** *Arch. Neurol.,* 1998, vol. 55, 937-945
- **ERTEKEIN-TANER, N. et al.** *Science,* 2000, vol. 290, 2303-2304
- **AUSUBEL et al.** Current Protocols in Molecular Biology. Greene Publishing Associates and Wiley Interscience, 1989
- **HARLOW, E. ; LANE, D.** Antibodies: A Laboratory Manual. Cold Spring Harbor Laboratory Press, 1988
- **DE WILDT et al.** *Nature Biotech.,* 2000, vol. 18, 989-994
- **GE, H.** *Nucleic Acids Res.,* 2000, vol. 28, e3, I-VII
- **MACBEATH, G. ; SCHREIBER, S.L.** *Science,* 2000, vol. 289, 1760-1763
- **HAAB et al.** *Genome Biology,* 2001, vol. 2 (2
- **GE, H.** *Nucleic Acids Res.,* 2000, vol. 28, e3, I-VTI
- **MENDOZA et al.** *Biotechniques,* 1999, vol. 27, 778-788
- **CRUTE et al.** *J. Biol. Chem,* 1998, vol. 273, 35347-35354
- **LIN et al.** *Nature Genetics*
- **CARLING et al.** *J. Biol. Chem.,* 1994, vol. 269, 11442